# FASCICULE DE BREVET EUROPEEN

(11) **EP 2 459 718 B1**
(45) Date de publication et mention de la délivrance du brevet: **28.11.2018**
(21) Numéro de dépôt: 10762957.8
(22) Date de dépôt: 30.07.2010
(51) Int. Cl.: C12N 15/115, C07K 14/745

(54) **PROCEDE POUR LA PURIFICATION DE PROTEINES DE LA COAGULATION A DOMAINE GLA**
VERFAHREN ZUR REINIGUNG VON GLA-DOMÄNE-KOAGULATIONSPROTEINEN
METHOD FOR PURIFYING GLA-DOMAIN COAGULATION PROTEINS

(30) Priorité: 31.07.2009 FR 0955406
(43) Date de publication de la demande: 06.06.2012
(73) Titulaire: Laboratoire Français du Fractionnement et des Biotechnologies, 91940 Les Ulis (FR)
(72) Inventeur: PERRET, Gérald, F-94600 Choisy Le Roi (FR); CHTOUROU, Sami, F-78990 Elancourt (FR); BIHOREAU, Nicolas, F-91400 Orsay (FR)
(74) Mandataire: Cabinet Becker et Associés
(86) Numéro de dépôt international: PCT/FR2010/051628
(87) Numéro de publication internationale: WO 2011/012830

(56) Documents cités:
- EP-A2- 0 354 354
- WO-A1-01/64748
- WO-A1-2010/094899
- WO-A1-2010/094900
- WO-A1-2010/094901
- WO-A2-2007/035532
- US-A1- 2003 175 703
- LAYZER JULIANA M ET AL: "Simultaneous generation of aptamers to multiple gamma-carboxyglutamic acid proteins from a focused aptamer library using DeSELEX and convergent selection", OLIGONUCLEOTIDES, vol. 17, no. 1, 1 avril 2007 (2007-04-01), pages 1-11, XP002544771, cité dans la demande
- LI YUAN ET AL: "Fabrication and characterization of RNA aptamer microarrays for the study of protein-aptamer interactions with SPR imaging", NUCLEIC ACIDS RESEARCH, vol. 34, no. 22, décembre 2006 (2006-12), pages 6416-6424, XP002571829, ISSN: 0305-1048 cité dans la demande
- NOMA TAKAHISA ET AL: "Screening of DNA aptamers against multiple proteins in tissue.", NUCLEIC ACIDS SYMPOSIUM SERIES, no. 49, 2005, pages 357-358, XP002571830, ISSN: 1746-8272

## Description

### DOMAINE DE L'INVENTION

La présente invention se rapporte au domaine de la purification des protéines, et en particulier au domaine de la purification des protéines de la coagulation à domaine GLA.

### ETAT DE LA TECHNIQUE

De manière générale, les protéines à domaine GLA forment une famille de protéines possédant une structure commune, le domaine GLA, qui consiste en une région localisée vers l'extrémité N-terminale de ces protéines et qui comprend une pluralité de résidus glutamine qui sont normalement carboxylés en résidus d'acide carboxy-glutamique ou « GLA ». Les protéines à domaine GLA comprennent en général une partie N-terminale appelée propeptide qui est reconnue par une carboxylase dépendante de la vitamine K. Après carboxylation des résidus glutamine du domaine GLA, le propeptide est clivé par protéolyse et la protéine à domaine GLA mature et active est libérée. Selon les protéines considérées, le domaine GLA est constitué d'environ 45 acides aminés comprenant de 9 à 12 résidus glutamine qui sont normalement carboxylés en Gla.

Les protéines à domaine GLA consistent en des protéines dites « vitamine K-dépendantes ». Les protéines à domaine GLA englobent des facteurs de la coagulation, des protéines du tissu osseux et des conopeptides. Les facteurs de la coagulation à domaine GLA englobent la prothrombine (Facteur II), le Facteur VII, le Facteur IX, le Facteur X, la Protéine C et la Protéine S. Les protéines à domaine GLA, y compris les protéines de la coagulation vitamine K-dépendantes à domaine GLA, sont présentées notamment dans l'article de Furie et al. (1999, Blood, Vol. 93 : 1798-1808).

Les protéines de la coagulation vitamine K-dépendantes à domaine GLA consistent en des protéines d'intérêt thérapeutique. Parmi celles-ci, le Facteur II, le Facteur VII, le Facteur IX
et le Facteur X, représentent des protéines d'un très grand intérêt thérapeutique qui sont administrés pour la prévention et le traitement de nombreux troubles de l'hémostase. Les protéines humaines de la coagulation à domaine GLA peuvent être purifiées à partir des fluides humains naturels, en général à partir de plasma sanguin humain. Par ailleurs, de nombreux travaux ont été entrepris afin de mettre au point des procédés de production et de purification
de protéines humaines recombinantes de la coagulation à domaine GLA. On peut citer par exemple le Facteur VII humain recombinant, qui est dores et déjà commercialisé en tant que médicament.

Pour l'obtention de protéines à domaine GLA purifiées à partir de fluides biologiques dans lesquels ces protéines sont produites naturellement ou bien sous forme de protéines recombinantes, des procédés de purification adaptés sont déjà connus dans l'état de la technique. Ces procédés comprennent en général une succession d'étapes de séparation sélective basées sur des étapes de précipitation des protéines, de passage sur des supports de chromatographie suivi d'étapes d'élution séquentielle, d'étapes de filtration en profondeur, d'ultrafiltration, ou encore d'étapes de concentration. Les procédés de purification de protéines de la coagulation à domaine GLA qui sont utilisés aujourd'hui pour produire des médicaments ne comprennent pas d'étape de chromatographie d'affinité. Une raison d'un tel choix technique réside dans les inconvénients engendrés par le détachement d'une partie des molécules ligand greffées sur le support d'affinité, qui sont retrouvées associées à la protéine thérapeutique purifiée dans le volume de l'éluat de chromatographie. A titre illustratif, on peut citer le produit Mononine®, qui est une composition pharmaceutique à base de Facteur IX humain purifié, qui est obtenu par un procédé utilisant un support d'immuno-affinité sur lequel sont immobilisés des anticorps monoclonaux anti-FIX de souris. Toutefois, la monographie du produit Mononine® spécifie la présence de traces d'anticorps monoclonaux murins anti-FIX humain dans le produit final, ce qui est de nature à entraîner des problèmes d'immunogénicité chez les patients traités car ils s'immunisent contre les « relargables » (anticorps et fragments d'anticorps murins). La monographie du produit Mononine® spécifie des contre-indications pour les patients allergiques aux protéines murines.

De manière générale, il existe un besoin dans l'état de la technique pour des procédés améliorés ou alternatifs de purification de protéines de la coagulation à domaine GLA vitamine
K-dépendantes. Cela englobe un besoin pour des procédés alternatifs ou améliorés pour l'obtention de compositions comprenant une seule protéine purifiée, comme par exemple le Facteur VII ou le Facteur IX, ainsi que les procédés alternatifs ou améliorés pour l'obtention de compositions comprenant une combinaison de protéines à domaine GLA, par exemple des compositions comprenant une combinaison de Facteur II, de Facteur VII, de Facteur IX et de Facteur X. Cela englobe un besoin pour des procédés alternatifs ou améliorés pour la purification de protéines à domaine GLA non recombinantes et de protéines à domaine GLA recombinantes.

### RESUME DE L'INVENTION

La présente invention concerne l'utilisation d'un aptamère d'ADN pour purifier simultanément les facteur VII, facteur IX, et facteur X dans du plasma sanguin, par:
a) mise en contact dudit plasma sanguin avec un support d'affinité sur lequel est immobilisé ledit aptamère désoxyribonucléique se liant spécifiquement aux facteur VII, facteur IX, et facteur X, afin de
   former des complexes entre (i) ledit aptamère désoxyribonucléique et (ii) les facteur VII, facteur IX, et facteur X,
b) libération desdits facteur VII, facteur IX, et facteur X à partir des complexes formés à l'étape a), et
c) récupération desdits facteur VII, facteur IX, et facteur X sous une forme purifiée,
caractérisée en ce que ledit aptamère désoxyribonucléique consiste en un aptamère de séquence choisie parmi les séquences SEQ ID NO : 3, 4, et 6 à 36.

### DESCRIPTION DES FIGURES

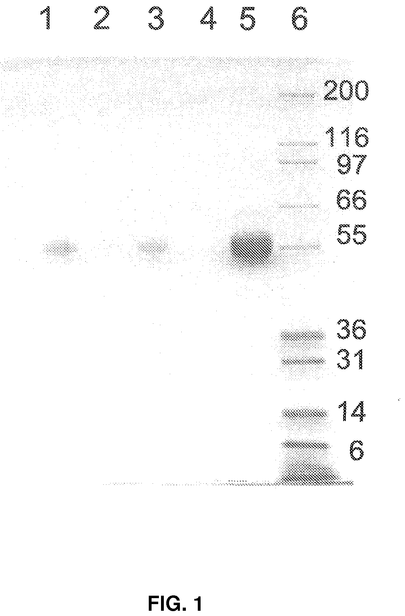

La **Figure 1** est le cliché d'un gel d'électrophorèse SDS-PAGE des protéines contenues dans les fractions successives obtenues lors de la purification de facteur IX plasmatique par chromatographie avec un support d'affinité sur lequel sont immobilisés des aptamères anti-GLA Mapt-1. Ligne 1 : matériel de départ ; ligne 2 : fraction non retenue (NR) ; ligne 3 : fraction d'élution (E1) ; ligne 4 : fraction de régénération (E2) ; ligne 5 : témoin de référence de Facteur IX purifié ; ligne 6 : protéines de référence de poids moléculaire connu.

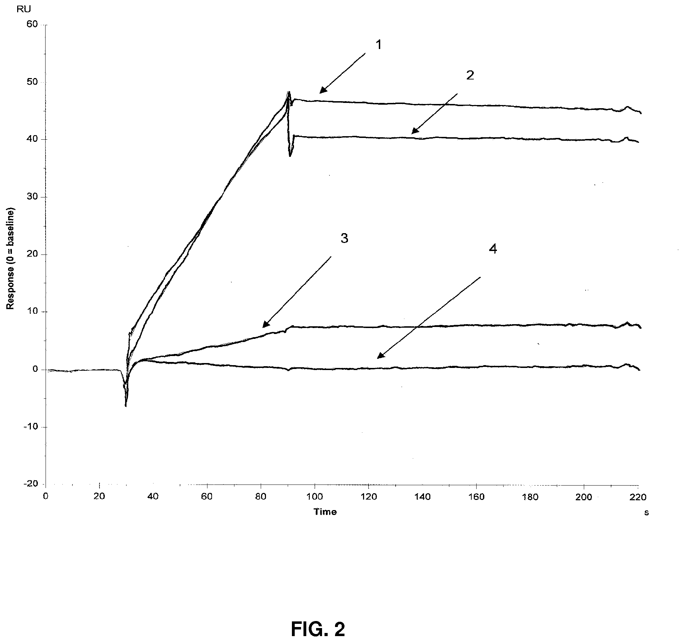

La **Figure 2** illustre les courbes de liaison (i) respectivement du Facteur VII, du Facteur IX ou du Facteur X, présent dans différentes compositions à (ii) un aptamère spécifique des protéines à domaine GLA immobilisé sur un support, dans un essai selon la technique de résonance plasmonique de surface. En abscisse : le temps, exprimé en secondes ; en ordonnées, le signal de résonance, exprimé en Unités de résonance arbitraires. Courbe n° 1 : préparation de Facteur IX humain recombinant (BeneFIX®) ; Courbe n° 2 : préparation de Facteur VII humain plasmatique ; Courbe n°3 : pré paration de Facteur X humain plasmatique ; Courbe n°4 : préparation de témoin négatif.

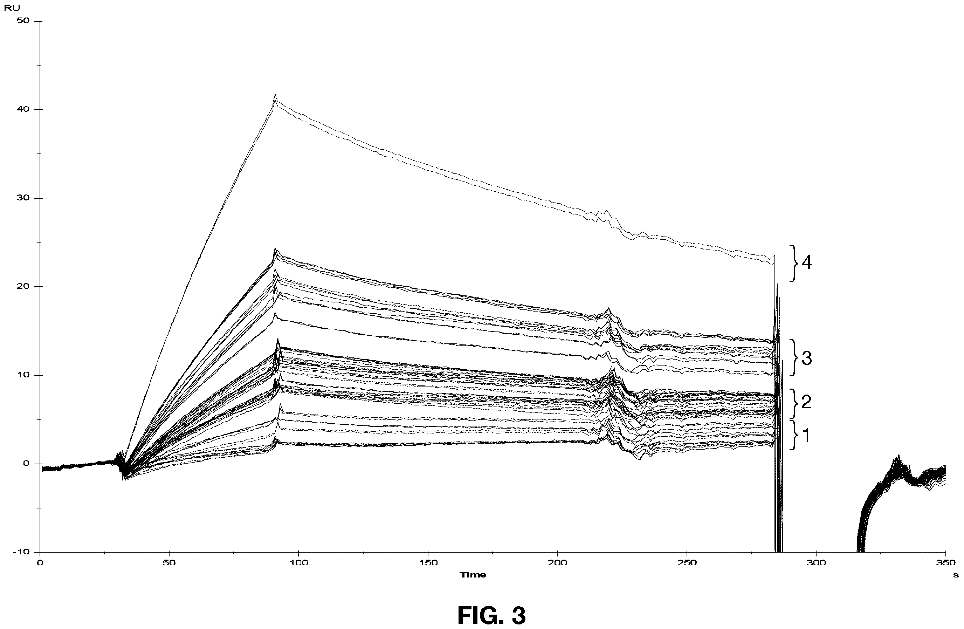

La **Figure 3** illustre les courbes de liaison d'une diversité d'acides nucléiques décrits ici au Facteur IX humain recombinant immobilisé sur un support, dans un essai selon la technique de résonnance plasmonique de surface. En abscisse : le temps, exprimé en secondes; en ordonnées, signal de résonance, exprimé en Unités de résonance arbitraires. Courbes « 1 » : acides nucléiques de faible affinité ; Courbes « 2 » : acides nucléiques d'affinité intermédiaire ; Courbes « 3 » : acides nucléiques d'affinité forte ; Courbes « 4 » : acide nucléique de très forte affinité.

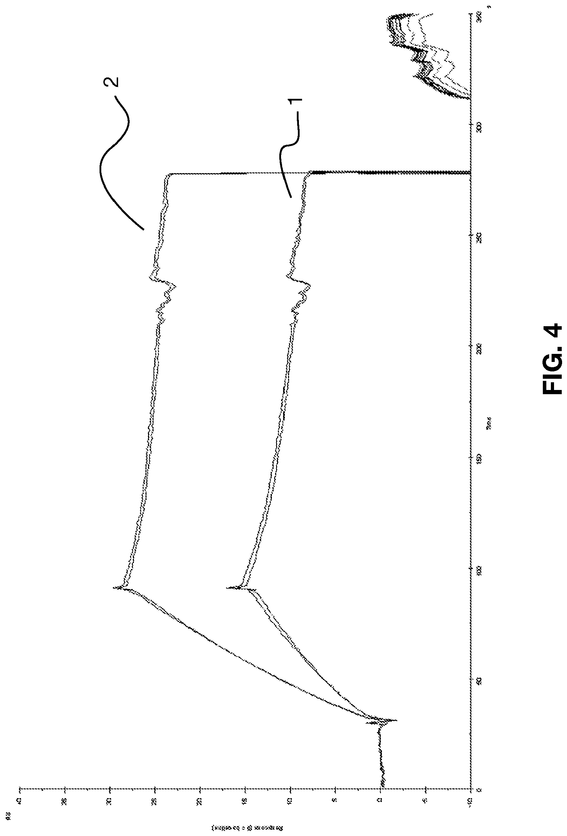

La **Figure 4** illustre les courbes de liaison des acides nucléiques aptamères « Mapt-1.2-CS » et Mapt-1.2.-CSO » au Facteur IX humain recombinant immobilisé sur un support, dans un essai selon la technique de résonnance plasmonique de surface. En abscisse : le temps, exprimé en secondes; en ordonnées, signal de résonance, exprimé en Unités de résonance arbitraires. La courbe n°1 est la courbe de liaiso n obtenue avec l'aptamère Mapt-1.2.-CS. La courbe n°2 est la courbe de liaison obtenue avec l'aptamère Mapt-1.2.-CSO.

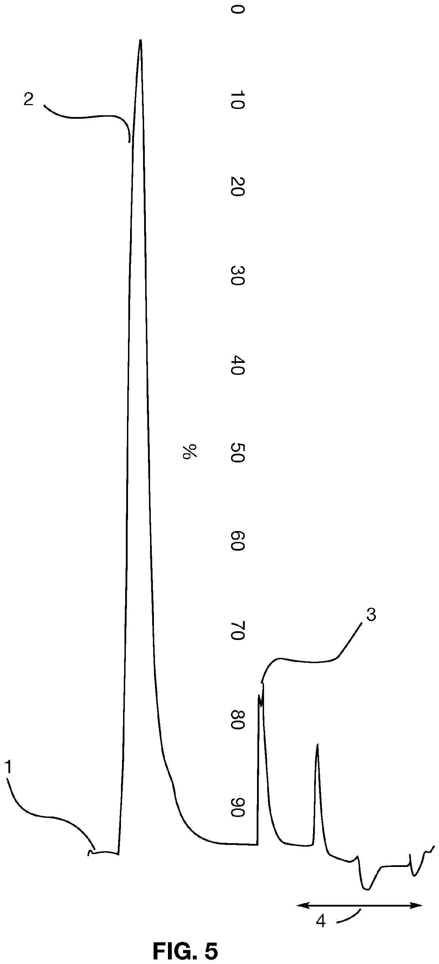

La **figure 5** illustre un profil de chromatographie obtenu lors de la mise en oeuvre du procédé de purification d'un Facteur IX humain plasmatique produit avec le support d'affinité sur lequel sont immobilisés des aptamères nucléiques anti-GLA. En abscisse : le temps ; en ordonnées : la valeur d'absorbance (D.O.) à 254 nanomètres. (1) : moment de l'injection du concentré de FIX humain plasmatique ; (2) : pic d'élimination de la fraction non retenue ; (3) : pic d'élution du FIX purifié ; (4) : pics générés pendant l'étape de régénération du support chromatographique.

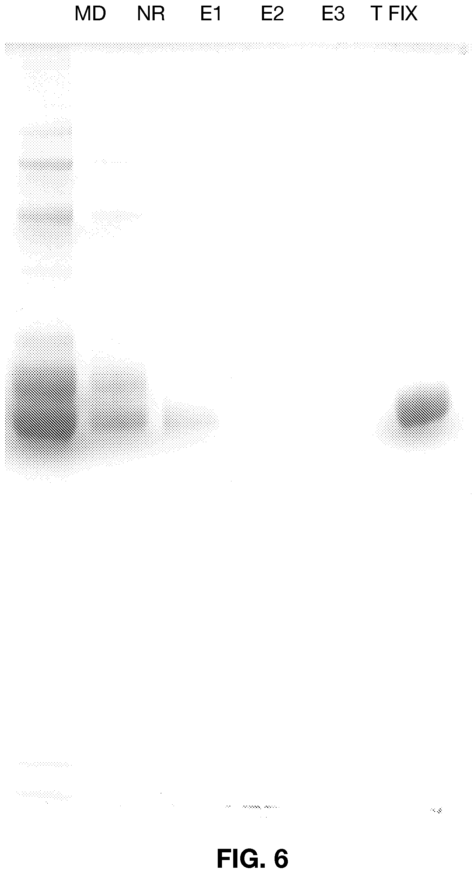

La **Figure 6** est le cliché d'un gel d'électrophorèse SDS-PAGE, avec gradient de 4 à 12% de Bis-Acrylamide sans réducteur, de protéines contenues dans une fraction purifiée de facteur IX humain plasmatique ayant subi une chromatographie sur un support d'affinité sur lequel sont greffés des aptamères nucléiques spécifiques de protéines à domaine GLA. Le gel SDS-PAGE a été traité par le Bleu de Coomassie. De la gauche vers la droite : ligne 1(« MD ») : Composition de départ, Concentré de Facteur IX humain plasmatique; ligne 2 (« NR ») : protéines contenues dans la fraction non retenue sur le support d'affinité ; ligne 3 (« E1 ») : protéines contenues dans la fraction d'élution E1 ; ligne 4 (« E2 ») : protéines contenues en sortie du support d'affinité, dans le tampon de régénération ; ligne 5 (« E3 ») : protéines contenues en sortie du support d'affinité, dans le tampon de régénération ; ligne 6 (« T FIX ») : fraction purifiée de facteur IX humain plasmatique

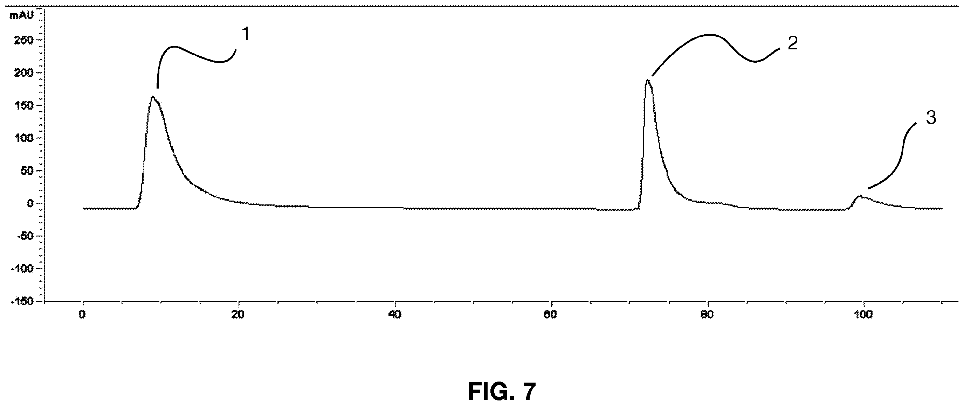

La **figure 7** illustre un profil de chromatographie obtenu lors de la mise en oeuvre du procédé de purification d'un Facteur IX humain plasmatique produit avec le support d'affinité dans lequel sont immobilisés des aptamères nucléiques anti-GLA. En abscisse ; le temps ; en ordonnées : la valeur d'absorbance (D.O.) à 254 nanomètres. (1) : fraction non retenue ; (2) : pic d'élution du FIX humain purifié ; (3) : pic de régénération du support chromatographique.

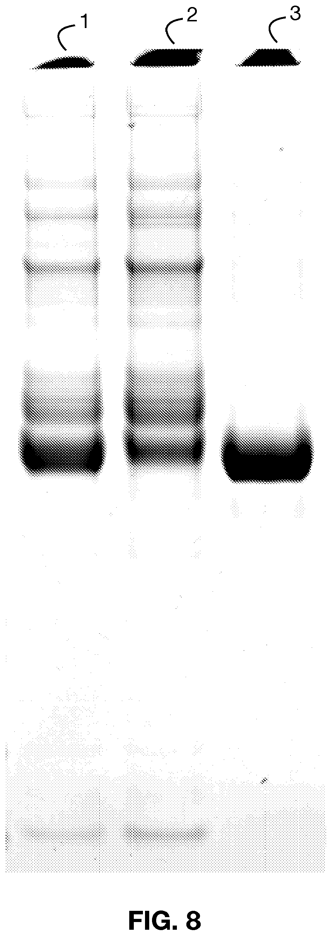

La **Figure 8** est le cliché d'un gel d'électrophorèse SDS-PAGE, avec gradient de 4 à 12% de Bis-Acrylamide sans réducteur, de protéines contenues dans une fraction purifiée de facteur IX humain plasmatique ayant subi une chromatographie sur un support d'affinité sur lequel sont greffés des aptamères nucléiques spécifiques de protéines à domaine GLA. Le gel SDS-PAGE a été traité par le Bleu de Coomassie. De la gauche vers la droite : ligne 1(« Départ ») : Composition de départ, Concentré de Facteur IX humain plasmatique ; ligne 2 (« Non Retenu ») : protéines contenues dans la fraction non retenue sur le support d'affinité ; ligne 3 (« Eluat ») : protéines contenues dans la fraction d'élution.

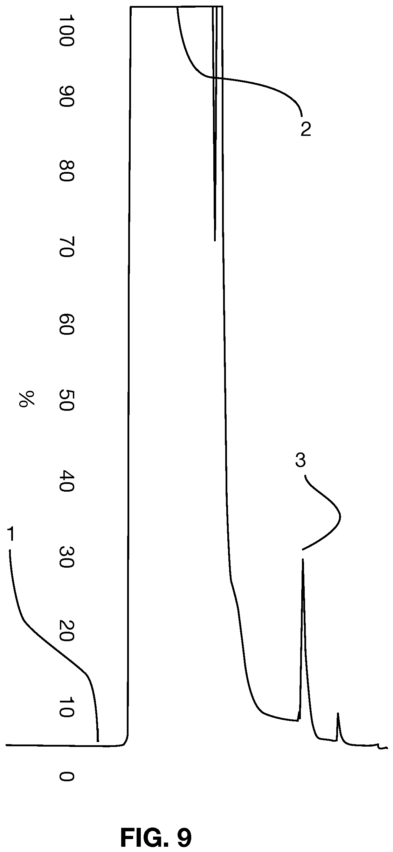

La **figure 9** illustre un profil de chromatographie obtenu lors de la mise en oeuvre du procédé de purification d'un Facteur IX humain transgénique produit dans le lait d'un truie transgénique avec le support d'affinité sur lequel sont immobilisés des aptamères nucléiques anti-GLA. En abscisse : le temps ; en ordonnées : la valeur d'absorbance (D.O.) à 254 nanomètres. (1): Moment d'injection du FIX transgénique, (2) : Fraction non retenue; (3): fraction d'élution.

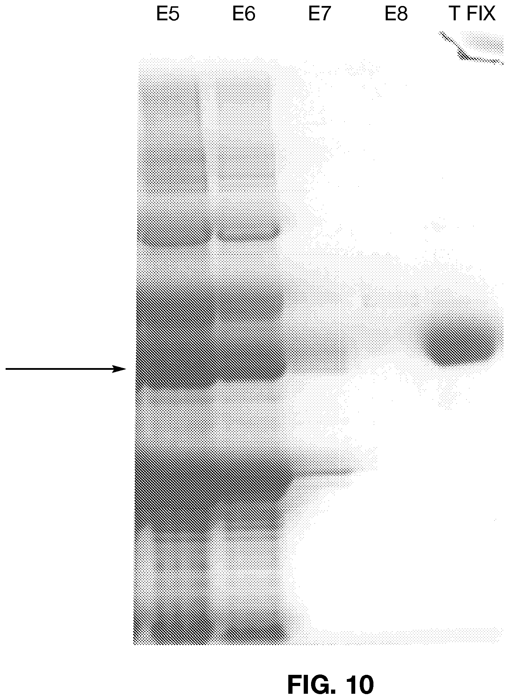

La **Figure 10** est le cliché d'un gel d'électrophorèse SDS-PAGEde protéines contenues dans une fraction pré-purifiée de facteur IX humain recombinant produit dans le lait d'une truie transgénique, ayant subi une chromatographie sur un support d'affinité sur lequel sont greffés des aptamères nucléiques spécifiques de protéines à domaine GLA. Le gel SDS-PAGE a été traité par le Bleu de Coomassie. De la gauche vers la droite : ligne 1(« E5 », « Départ ») : Composition de départ, Facteur IX humain transgénique pré-purifié sur MEP HyperCel ; ligne 2 (« E6 », « Non Retenu ») : protéines contenues dans la fraction non retenue sur le support d'affinité ; ligne 3 (« E7 », « Elution ») : protéines contenues dans la fraction d'élution ; ligne 4 (« E8 », « Régénération ») : protéines contenues dans la fraction de régénération ; ligne 5 (« T FIX », « Témoin FIX pure ») : Témoins FIX purifié . Flèche : position de migration du FIX.

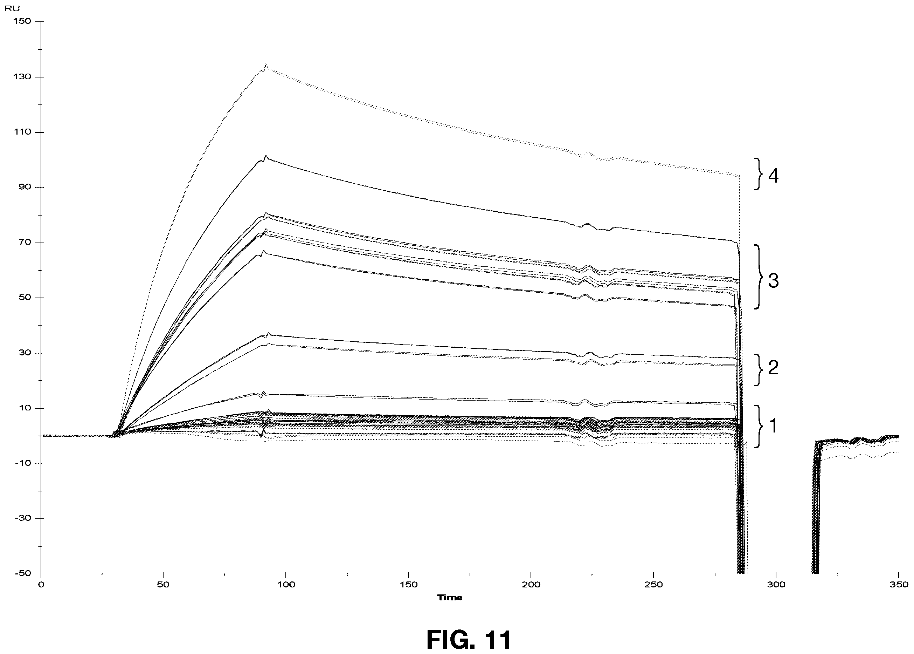

La **Figure 11** illustre les courbes de liaison d'une diversité d'acides nucléiques décrits ici au Facteur IX humain recombinant immobilisé sur un support, dans un essai selon la technique de résonnance plasmonique de surface. En abscisse : le temps, exprimé en secondes; en ordonnées, signal de résonance, exprimé en Unités de résonance arbitraires. Courbes « 1 » : acides nucléiques de faible affinité ; Courbes « 2 » : acides nucléiques d'affinité intermédiaire ; Courbes « 3 » : acides nucléiques d'affinité forte ; Courbes « 4 » : acide nucléique de très forte affinité.

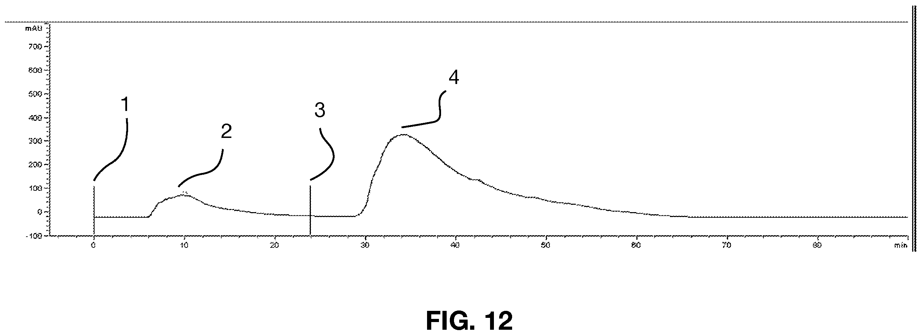

La **figure 12** illustre un profil de chromatographie obtenu lors de la mise en oeuvre du procédé de purification d'un Facteur VII humain transgénique produit avec le support d'affinité dans lequel sont immobilisés des aptamères nucléiques anti-GLA. En abscisse ; le temps ; en ordonnées : la valeur d'absorbance (D.O.) à 254 nanomètres. (1) : moment de l'injection ; (2) : fraction non retenue ; (3) : moment d'injection du tampon d'élution ; (4) : fraction d'élution.

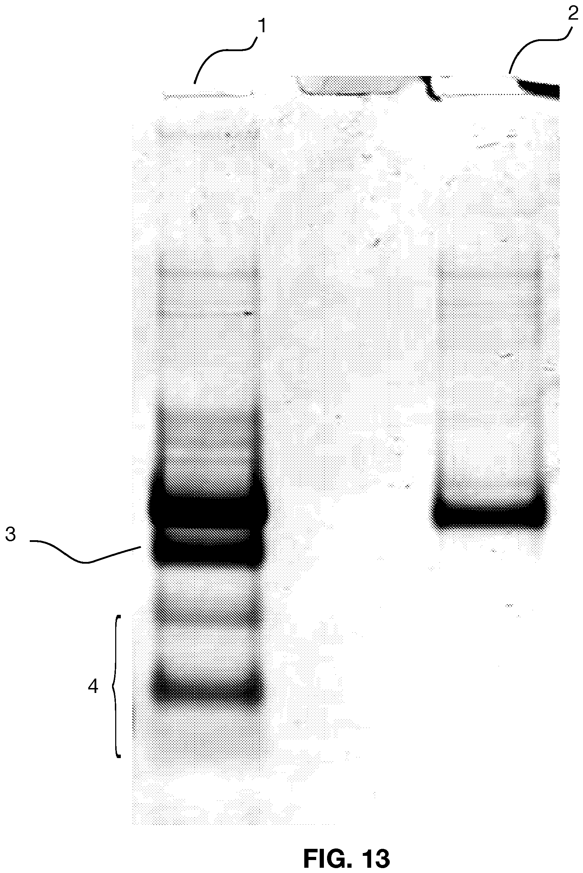

La **Figure 13** est le cliché d'un gel d'électrophorèse SDS-PAGE de protéines contenues dans une fraction pré-purifiée de facteur VII humain plasmatique, ayant subi une chromatographie sur un support d'affinité sur lequel sont greffés des aptamères nucléiques spécifiques de protéines à domaine GLA. Le gel SDS-PAGE a été traité par le Bleu de Coomassie. De la gauche vers la droite : (1) : ligne 1 (« Départ ») : Composition de départ, Facteur VII humain plasmatique; (2) : ligne 2 (« Eluat ») : protéines contenues dans la fraction d'élution ; (3) : FVII Des-Gla : formes de FVII mal glycosylées ; (4) : autres formes mal identifiées de FVII..

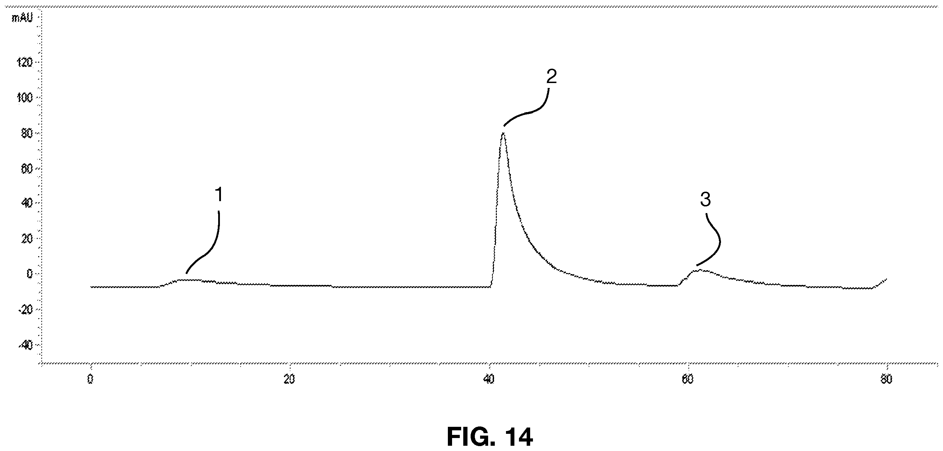

La **figure 14** illustre un profil de chromatographie obtenu lors de la mise en oeuvre du procédé de purification d'un Facteur VII humain transgénique produit avec le support d'affinité dans lequel sont immobilisés des aptamères nucléiques anti-GLA. En abscisse ; le temps ; en ordonnées : la valeur d'absorbance (D.O.) à 254 nanomètres. (1) : fraction non retenue ; (2) fraction d'élution ; (3) : fraction de régénération.

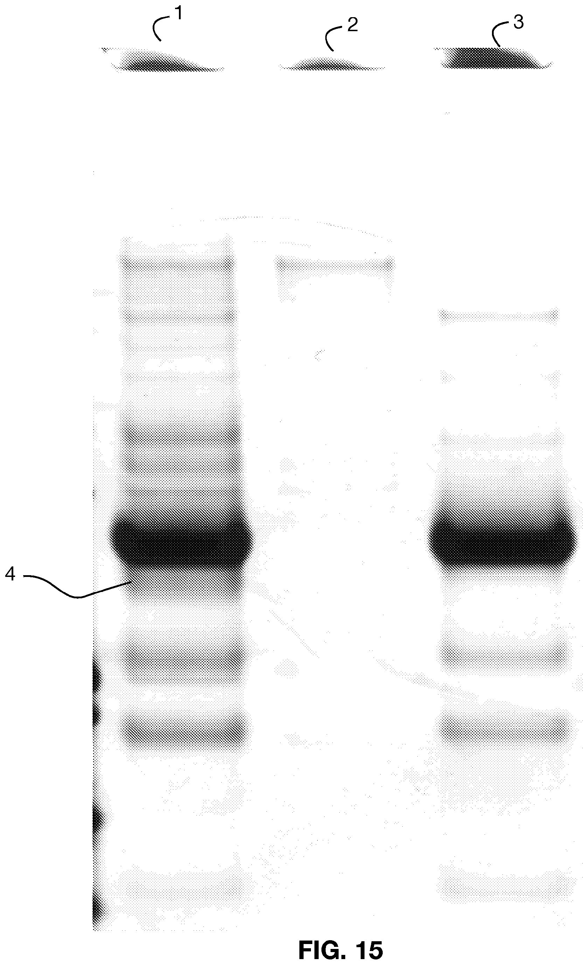

La **Figure 15** est le cliché d'un gel d'électrophorèse SDS-PAGE de protéines contenues dans une fraction pré-purifiée de facteur VII humain plasmatique, ayant subi une chromatographie sur un support d'affinité sur lequel sont greffés des aptamères nucléiques spécifiques de protéines à domaine GLA. Le gel SDS-PAGE a été traité par le Bleu de Coomassie. De la gauche vers la droite : (1) : ligne 1 « Départ ») : Composition de départ, (2) : Facteur VII humain plasmatique; ligne 2 (« NR ») : Fraction des proténes non retenues ; (3) : ligne 3 : (« Eluat ») : protéines contenues dans la fraction d'élution ; (4) : FVII Des-Gla, formes mal glycosylées de FVII..

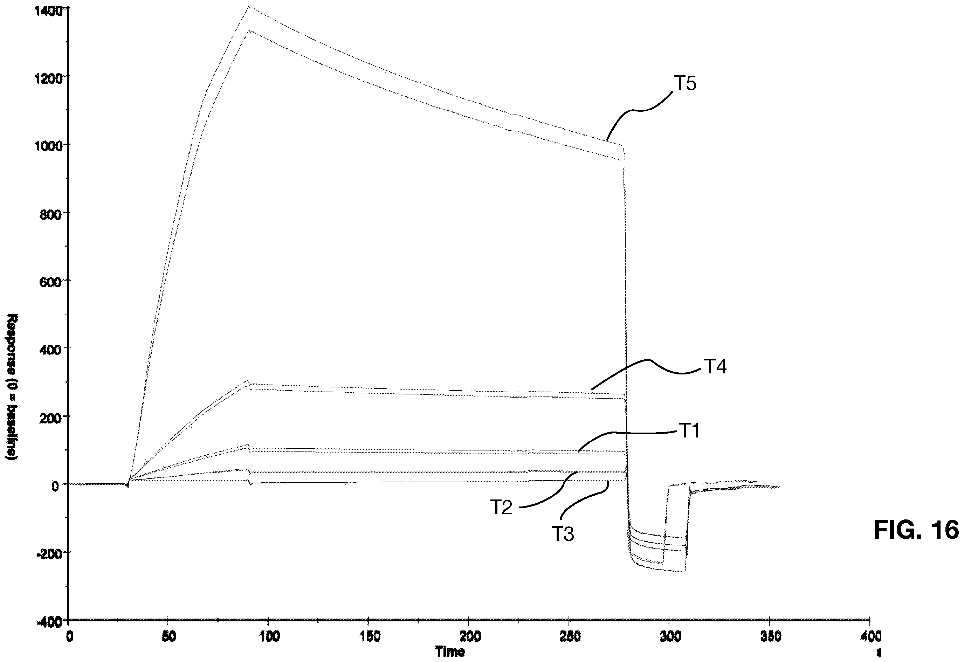

La **Figure 16** illustre les courbes de liaison de l'aptamère Mapt-2 immobilisé sur un support au Facteur VII humain plasmatique, dans un essai selon la technique de résonnance plasmonique de surface. Les courbes correspondent à la cinétique de fixation du FVII plasmatique lors de l'utilisation de différents tampons de course, respectivement, du bas vers le haut de la figure 16 : T3 : Tampon 3, T2 : Tampon 2, T1 : Tampon 1, T4 : Tampon 4 et T5 : Tampon 5. En abscisse : le temps, exprimé en secondes; en ordonnées : signal de résonance, exprimé en Unités de résonance arbitraires. Du bas vers le haut de la figure : courbes illustrant des interactions d'affinité croissante..

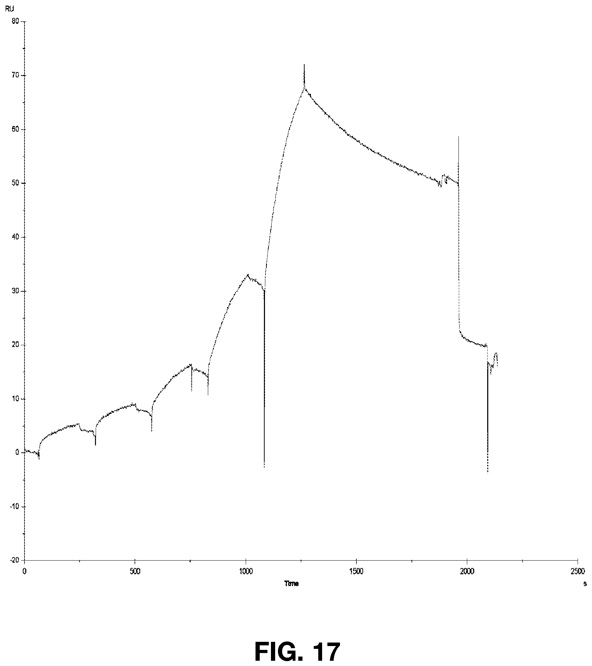

La **Figure 17** illustre les courbes de liaison de l'aptamère Mapt-2 immobilisé sur un support au Facteur VII humain plasmatique, dans un essai selon la technique de résonnance plasmonique de surface. La courbe permet de déterminer les paramètres de la cinétique de fixation du FVII plasmatique lors de l'utilisation du tampon 1 (Tris 50 mM, NaCl 50 mM, CaCl₂ 10 mM, MgCl₂ 4 mM, pH 7,5). En abscisse : le temps, exprimé en secondes; en ordonnées, signal de résonance, exprimé en Unités de résonance arbitraires.

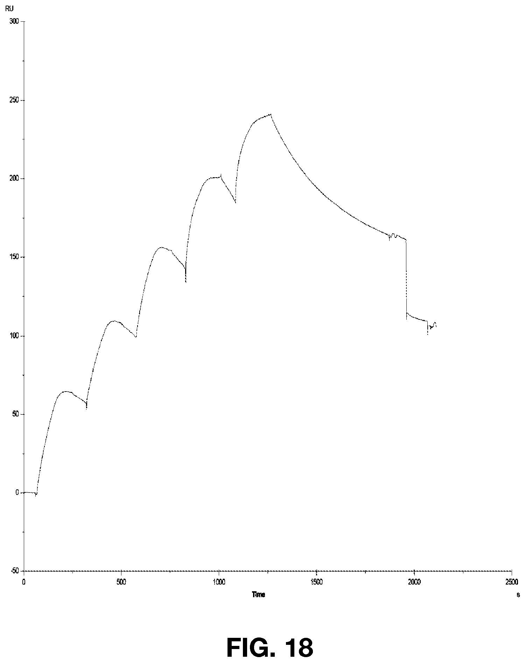

La **Figure 18** illustre les courbes de liaison de l'aptamère Mapt-2 immobilisé sur un support au Facteur VII humain plasmatique, dans un essai selon la technique de résonnance plasmonique de surface. La courbe correspond à la cinétique de fixation du FVII plasmatique lors de l'utilisation du tampon 5 (Tris 50 mM, CaCl₂ 10 mM, pH 7,5). En abscisse : le temps, exprimé en secondes; en ordonnées, signal de résonance, exprimé en Unités de résonance arbitraires.

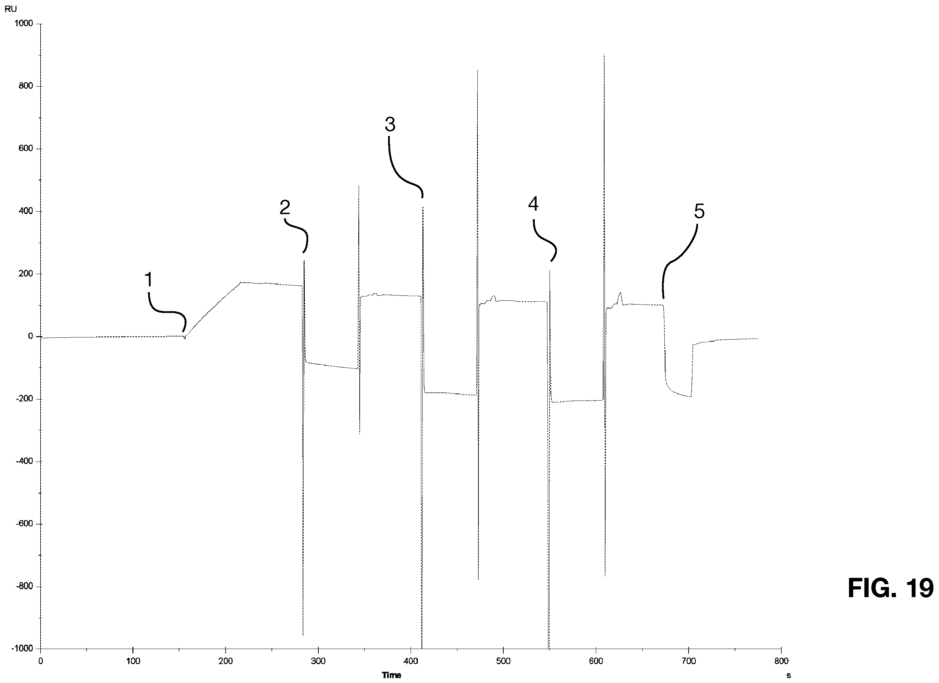

La **Figure 19** illustre les courbes de liaison de l'aptamère Mapt-2 immobilisé sur un support au Facteur VII humain plasmatique, dans un essai selon la technique de résonnance plasmonique de surface. Les courbes correspondent à la résistance de la liaison du FVII plasmatique à Mapt-2 lors de l'utilisation de différents tampons de lavage: (1): injection de FVII ; (2) : tampon Tris 50 mM, NaCl 1 M, CaCl₂ 10 mM, MgCl₂ 4 mM, pH 7,5 , (3) : tampon Tris 50 mM, NaCl 2 M, CaCl₂ 10 mM, MgCl₂ 4 mM, pH 7,5, (4) : tampon Tris 50 mM, NaCl 3 M, CaCl₂ 10 mM, MgCl₂ 4 mM, pH 7,5 et (5) : tampon Tris 50 mM, EDTA 10 mM. En abscisse : le temps, exprimé en secondes; en ordonnées : signal de résonance, exprimé en Unités de résonance arbitraires.

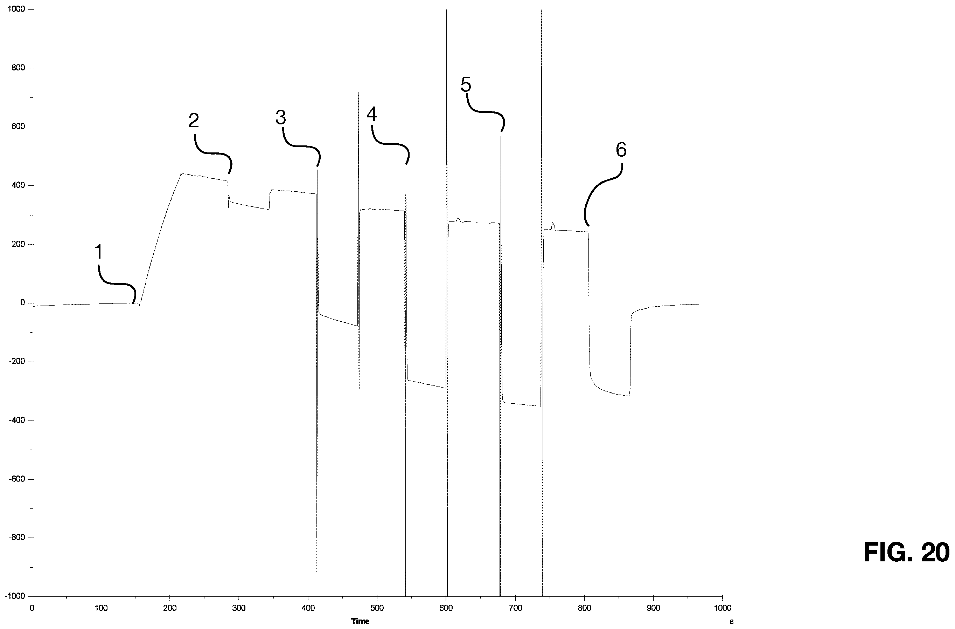

La **Figure 20** illustre les courbes de liaison de l'aptamère Mapt-2 immobilisé sur un support au Facteur VII humain plasmatique, dans un essai selon la technique de résonnance plasmonique de surface. Les courbes correspondent à la résistance de la liaison du FVII plasmatique à Mapt-2 lors de l'utilisation de différents tampons de lavage: (1): injection de FVII ; (2) : tampon Tris 50 mM, CaCl₂ 10 mM, MgCl₂ 4 mM, pH 7,5 , (3) : tampon Tris 50 mM, CaCl₂ 10 mM, MgCl₂ 4 mM, NaCl 1M, pH 7,5 , (4) : tampon Tris 50 mM, CaCl₂ 10 mM, MgCl₂ 4 mM, NaCl 2M, pH 7,5 ; (5) : tampon Tris 50 mM, CaCl₂ 10 mM, MgCl₂ 4 mM, NaCl 3M, pH 7,5 et (6) : tampon Tris 50 mM, EDTA 10 mM. En abscisse : le temps, exprimé en secondes; en ordonnées, signal de résonance, exprimé en Unités de résonance arbitraires.

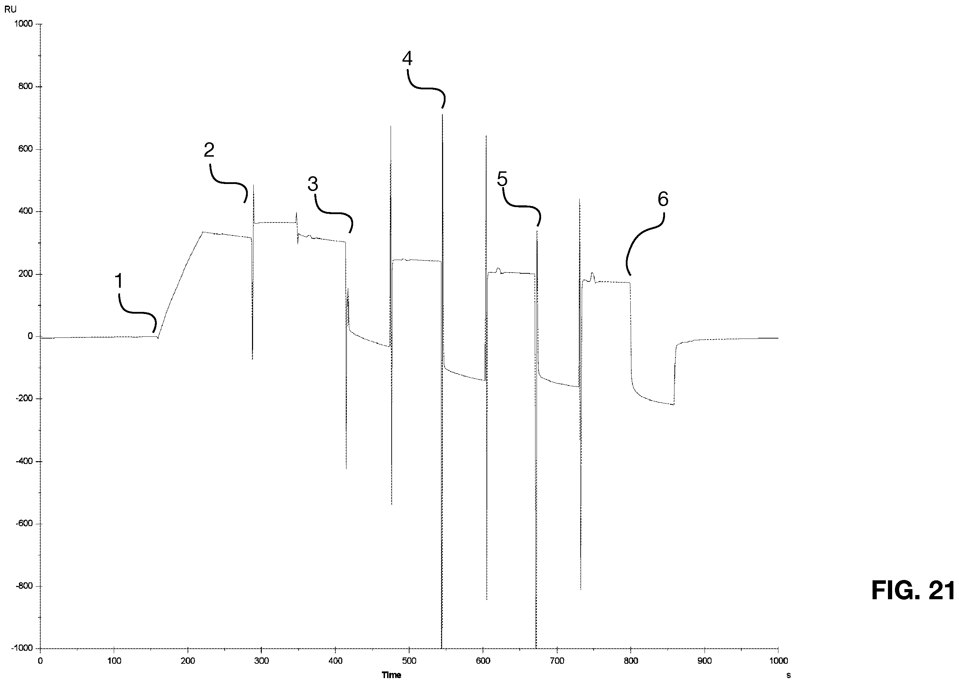

La **Figure 21** illustre les courbes de liaison de l'aptamère Mapt-2 immobilisé sur un support au Facteur VII humain plasmatique, dans un essai selon la technique de résonnance plasmonique de surface. Les courbes correspondent à la résistance de la liaison du du FVII plasmatique à Mapt-2 lors de l'utilisation de différents tampons de lavage : (1) : injection de FVII ; (2) : tampon éthanol à 10%, (3) tampon Tris 50 mM, CaCl₂ 10 mM, MgCl₂ 4 mM, NaCl 1M, pH 7,5, (4) : tampon Tris 50 mM, CaCl₂ 10 mM, MgCl₂ 4 mM, NaCl 2M, pH 7,5 ; (5) : tampon Tris 50 mM, CaCl₂ 10 mM, MgCl₂ 4 mM, NaCl 3M, pH 7,5 ; et (6) tampon Tris 50 mM, EDTA 10 mM. En abscisse : le temps, exprimé en secondes; en ordonnées, signal de résonance, exprimé en Unités de résonance arbitraires.

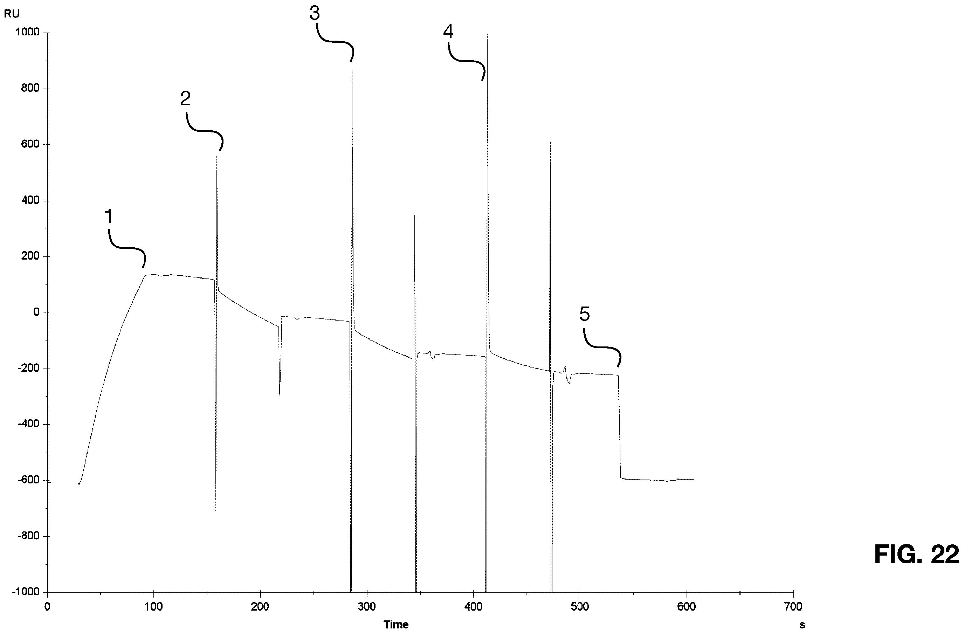

La **Figure 22** illustre les courbes de liaison de l'aptamère Mapt-1 immobilisé sur un support au Facteur VII humain recombinant, dans un essai selon la technique de résonnance plasmonique de surface. Les courbes correspondent à la résistance de la liaison du FIX recombinant à Mapt-1 lors de l'utilisation de différents tampons de lavage : (1) : injection de FVII recombinant; (2) : tampon Tris 50 mM, CaCl₂ 10 mM, MgCl₂ 4 mM, NaCl 1M, pH 7,5, (3) tampon Tris 50 mM, CaCl₂ 10 mM, MgCl₂ 4 mM, NaCl 2M, pH 7,5 ; (4) tampon Tris 50 mM, CaCl₂ 10 mM, MgCl₂ 4 mM, NaCl 3M, pH 7,5 et (5) tampon Tris 50 mM, EDTA 10 mM.. En abscisse : le temps, exprimé en secondes; en ordonnées, signal de résonance, exprimé en Unités de résonance arbitraires.

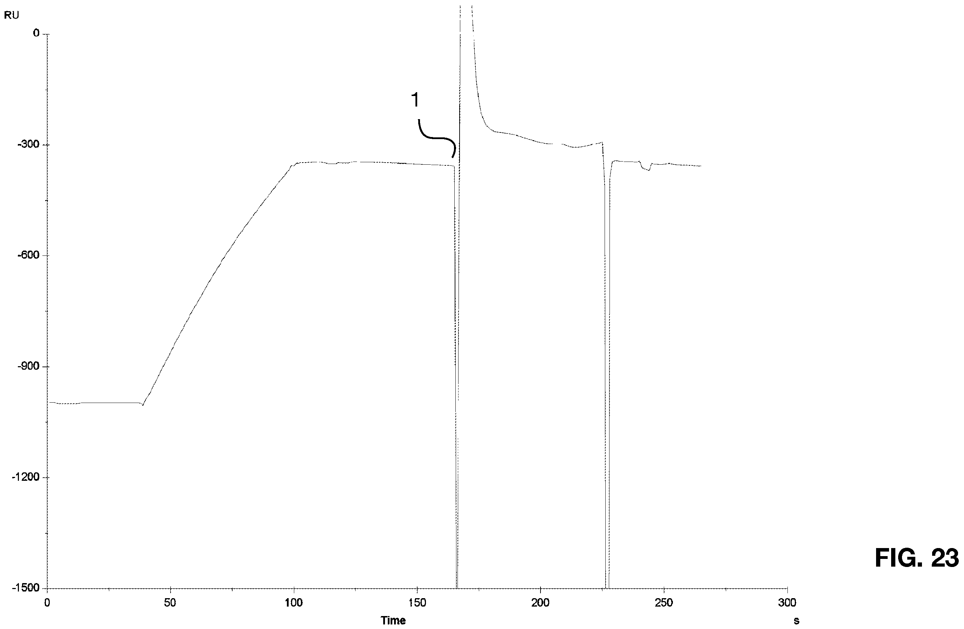

La **Figure 23** illustre les courbes de liaison de l'aptamère Mapt-2 immobilisé sur un support au Facteur IX humain recombinant, dans un essai selon la technique de résonnance plasmonique de surface. Les courbes correspondent à la résistance de la liaison du FIX recombinat à Mapt-1 lors de l'utilisation du tampon de lavage: Tris 50 mM, CaCl₂ 10 mM, propylène glycol à 50%, à pH 7,5. En abscisse : le temps, exprimé en secondes; en ordonnées, signal de résonance, exprimé en Unités de résonance arbitraires. (1) : injection de propylène glycol à 50% dans le Tampon 5.

### DESCRIPTION DETAILLEE DE L'INVENTION

Le demandeur s'est attaché à concevoir de nouveaux procédés pour purifier des protéines de la coagulation à domaine GLA, c'est-à-dire de nouveaux procédés adaptés pour l'obtention de protéine(s) de la coagulation à domaine GLA purifiées à partir d'un échantillon de départ comprenant une protéine de la coagulation à domaine GLA ou une pluralité de protéines de la coagulation à domaine GLA. En d'autres termes, le demandeur a cherché à mettre au point des procédés d'enrichissement ou de purification qui soit à la fois (i) sélectif pour les protéines de la coagulation à domaine GLA et (ii) non sélectif d'une protéine de la coagulation à domaine GLA spécifique.

Afin de mettre au point ces procédés de purification, le demandeur a isolé et caractérisé de nouveaux ligands qui (i) possèdent l'aptitude à se lier spécifiquement aux protéines de la coagulation à domaine GLA et (ii) qui ne sont pas spécifiques d'une protéine de la coagulation à domaine GLA en particulier.

Ces nouveaux ligands, dont les caractéristiques sont détaillées plus loin dans la présente description, consistent en des acides nucléiques, aussi appelés « aptamères nucléiques », se liant aux protéines de la coagulation à domaine GLA, et qui ne sont pas spécifiques d'une protéine de la coagulation à domaine GLA particulière.

La présente description fournit des procédés de purification de protéines de la coagulation à domaine GLA, dans lesquels il est tiré bénéfice des propriétés de liaison de ces nouveaux ligands du type acide nucléique.

Il est décrit ici aussi des procédés permettant l'obtention d'aptamères nucléiques se liant spécifiquement aux protéines de la coagulation à domaine GLA, dans l'objectif d'utiliser lesdits aptamères nucléiques dans des procédés de purification.

Selon l'invention, on a obtenu des aptamères nucléiques se liant spécifiquement aux protéines de la coagulation à domaine GLA. Plus précisément, le demandeur a obtenu et caractérisé des aptamères nucléiques qui se lient exclusivement à une variété de protéines de la coagulation à domaine GLA, c'est à dire à une variété de protéines de la coagulation possédant un domaine GLA dont les résidus glutamine caractéristiques du domaine GLA peuvent être gamma-carboxylés.

Comme cela est montré dans les exemples, un aptamère nucléique décrit ici, qui est spécifique des protéines de la coagulation à domaine GLA, est capable de se lier indifféremment à une variété de protéines de la coagulation vitamine K-dépendantes présentant une caractéristique commune conservée qui est le domaine GLA.

On a notamment montré dans les exemples qu'un aptamère spécifique des protéines de la coagulation à domaine GLA est capable de se lier à une pluralité de protéines comme le Facteur IX, le Facteur VII et le Facteur X.

On connaît déjà dans l'état de la technique des aptamères nucléiques qui reconnaissent spécifiquement et individuellement des protéines de la coagulation à domaine GLA tels que le Facteur II, le Facteur VII, le Facteur IX ou le Facteur X, y compris des aptamères liant la thrombine (Zhao et al., 2008, Anal Chem, Vol. 80(19) : 7586-7593), des aptamères liant le Facteur IX/IXa (Subash et al., 2006, Thromb Haemost, Vol. 95 : 767-771; Howard et al., 2007, Atherioscl Thromb Vasc Biol, Vol. 27 : 722-727; Demande PCT n°WO 2002/096926; Brevet aux Etats-Unis n°US 7,312,325), des aptamères liant le Facteur X/Xa (Demande PCT n°WO 2002/096926; Brevet aux Etats-Unis n°US 7,312,325) ou encore des aptamères liant le facteur VII/VIIa humain (Rusconi et al., 2000, Thromb Haemost, Vol. 84(5) : 841-848; Layzer et al., 2007, Spring, Vol. 17 : 1-11).

On précise qu'aucun des aptamères ci-dessus n'est décrit pour son utilisation pour purifier la protéine cible sur laquelle il se lie. De plus, les aptamères ci-dessus se lient exclusivement à une seule protéine de la coagulation à domaine GLA, sans réaction croisée avec une autre protéine de la coagulation à domaine GLA.

Un tel type d'aptamère, spécifique d'une protéine de la coagulation à domaine GLA donnée, et qui ne présente pas la capacité de se lier à une autre protéine, y compris à une autre protéine de la coagulation à domaine GLA, est par exemple décrit par Layzer et al. (2007, Oligonucleotides, Vol. 17 : 1-11).

La disponibilité d'aptamères nucléiques se liant spécifiquement aux protéines de la coagulation à domaine GLA a permis la mise au point de procédés de purification de ces protéines, notamment dans l'objectif d'obtenir un produit final purifié utilisable en tant que principe actif d'un médicament.

Il est ici décrit un procédé pour la purification de protéines de la coagulation à domaine GLA comprenant les étapes suivantes :
a) mettre en contact un échantillon contenant une ou plusieurs protéines de la coagulation à domaine GLA avec un support d'affinité sur lequel sont immobilisés des aptamères nucléiques se liant spécifiquement aux protéines de la coagulation à domaine GLA, afin de former des complexes entre (i) lesdits aptamères nucléiques et (ii) ladite ou lesdites protéine(s) de la coagulation à domaine GLA,
b) libérer la ou les protéine(s) de la coagulation à domaine GLA à partir des complexes formés à l'étape a), et
c) récupérer ladite ou lesdites protéine(s) de la coagulation à domaine GLA sous une forme purifiée.

Il est aussi décrit ici un procédé pour la purification de protéines de la coagulation à domaine GLA comprenant les étapes suivantes :
a) mettre en contact un échantillon contenant une ou plusieurs protéines de la coagulation à domaine GLA avec un support d'affinité sur lequel sont immobilisés des aptamères nucléiques se liant spécifiquement à une pluralité de protéines de la coagulation à domaine GLA, afin de former des complexes entre (i) lesdits aptamères nucléiques et (ii) ladite ou lesdites protéine(s) de la coagulation à domaine GLA,
b) libérer la ou les protéine(s) de la coagulation à domaine GLA à partir des complexes formés à l'étape a), et
c) récupérer ladite ou lesdites protéine(s) de la coagulation à domaine GLA sous une forme purifiée.

Le terme « protéine de la coagulation à domaine GLA » englobe toute protéine de la coagulation comprenant une région, désignée domaine GLA, comprenant une pluralité de résidus glutamine qui sont gamma-carboxylés au cours de la synthèse protéique. Les protéines de la coagulation à domaine GLA englobent les protéines de la coagulation possédant un domaine GLA vers l'extrémité N-terminale, ledit domaine GLA étant localisé généralement en aval, c'est-à-dire du côté C-terminal, d'un propeptide qui est naturellement hydrolysé au cours de la synthèse. Les protéines de la coagulation à domaine GLA englobent les protéines de la coagulation dans lesquelles le domaine GLA consiste en une région d'environ 45 acides aminés comprenant de 9 à 12 résidus glutamine dont au moins une partie d'entre eux sont normalement carboxylés en résidus GLA lors du processus de synthèse protéique dans les cellules. Les facteurs de la coagulation à domaine GLA, qui consistent en des protéines vitamine K-dépendantes, englobent la prothrombine (Facteur II), le Facteur VII, le Facteur IX, le Facteur X, la Protéine C et la Protéine S. Les protéines de la coagulation à domaine GLA englobent les protéines non recombinantes provenant de sources naturelles, comme le plasma sanguin, ainsi que les protéines recombinantes qui peuvent être produites *in vitro* par des cellules transfectées ou transformées avec un ADN codant ladite protéine de la coagulation ou qui peuvent être produites *in vivo* par des animaux dans lequel on a introduit un transgène codant ladite protéine de la coagulation. Les protéines de la coagulation à domaine GLA produites par des animaux transgéniques peuvent aussi être appelées « protéines transgéniques » dans la présente description.

Par « aptamère nucléique », on entend selon l'invention un acide nucléique simple brin se liant spécifiquement aux protéines de la coagulation à domaine GLA, y compris un acide nucléique se liant spécifiquement à une pluralité de protéines à domaine GLA, qui peut être aussi désigné dans la présente description par le terme « aptamère anti-GLA ». Les aptamères décrits ici englobent donc ceux pour lesquels on peut détecter des complexes avec une variété de protéines de la coagulation à domaine GLA données, après une étape préalable de mise en contact des partenaires respectivement nucléique et protéique.

La détection de complexes formés entre un aptamère anti-GLA utile selon l'invention et une protéine de la coagulation à domaine GLA peut être aisément réalisée par l'homme du métier, par exemple en mettant en oeuvre une technique de détection par résonance plasmonique de surface, y compris la technique Biacore®, comme cela est illustré dans les exemples. L'homme du métier peut aussi aisément détecter la formation de complexes entre un aptamère anti-GLA utile selon l'invention et une protéine de la coagulation à domaine GLA par des techniques classiques du type ELISA, comme cela est connu par l'homme du métier.

On a montré dans les exemples qu'un aptamère anti-GLA tel que décrit ici est capable de se lier respectivement à une pluralité de protéines de la coagulation à domaine GLA distinctes, et en particulier à une pluralité de protéines humaines de la coagulation à domaine GLA. On a montré notamment qu'un aptamère anti-GLA tel que décrit ici est capable de se lier respectivement au Facteur VII, au Facteur IX et au Facteur X humains.
Selon l'invention, les protéines de la coagulation à domaine GLA sont les facteurs de la coagulation vitamine K dépendants que sont le facteur VII, le facteur IX, et le facteur X.

Selon l'invention, le procédé de purification ci-dessus est adapté pour purifier simultanément le facteur VII, le facteur IX et le facteur X.

La purification simultanée de plus d'une protéine de la coagulation à domaine GLA dépend notamment du type d'échantillon de départ qui est utilisé pour la mise en oeuvre du procédé de purification. En particulier, le nombre et l'identité des protéines de la coagulation à domaine GLA qui sont obtenues sous forme purifiée à l'issue du procédé sont logiquement limités par le nombre et l'identité des protéines de la coagulation à domaine GLA qui sont présentes initialement dans l'échantillon de départ.

Selon l'invention, les aptamères anti-GLA consistent en des molécules d'ADN (acide désoxyribonucléique) qui ont la capacité à se lier à une protéine à domaine GLA, supérieure à la capacité à se lier à une protéine qui ne comprend pas de domaine GLA.

Un aptamère anti-GLA peut être obtenu par des procédés qui ont été spécialement mis au point pour les besoins de l'invention, et qui sont détaillés plus loin dans la présente description.

Dans certains modes de réalisation, les aptamères anti-GLA utilisés selon l'invention possèdent diverses caractéristiques structurelles communes, y inclus une séquence comprenant, de l'extrémité 5' vers l'extrémité 3', successivement (i) une séquence nucléotidique spécifique invariable d'environ 20 nucléotides de longueur, suivie de (ii) une séquence nucléotidique variable d'environ 40 à 50 nucléotides de longueur, suivie de (iii) une séquence nucléotidique spécifique invariable d'environ 20 nucléotides de longueur. On précise que les séquences nucléotidiques variables (ii) peuvent posséder entre elles une très forte identité de séquence en nucléotides.

Les procédés de sélection d'aptamères anti-GLA qui sont spécifiés dans la présente description sont du type permettant l'obtention d'une famille d'aptamères anti-GLA, aptes à reconnaître une pluralité de protéines de la coagulation à domaine GLA, en particulier de protéines humaines de la coagulation à domaine GLA.

D'un point de vue structurel, chaque élément de la famille d'acides nucléiques, ou aptamères nucléiques, susceptibles d'être obtenus par les procédés décrits ici, qui se lient spécifiquement
aux protéines de la coagulation à domaine GLA, comprend au moins 15 nucléotides consécutifs d'un polynucléotide ayant au moins 40% d'identité en nucléotides avec l'acide nucléique de formule (I) suivante :

5'-[SEQ ID N°1]x-[SEQ ID N°X]-[SEQ ID N°2]y-3' (I),

dans laquelle :
- « SEQ ID N°X » consiste en un acide nucléique de séquence SEQ ID N°3,
- « x » est un entier égal à 0 ou 1, et
- « y » est un entier égal à 0 ou 1.

Dans certains modes de réalisation, l'acide de séquence SEQ ID N°X a une longueur de 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, ou 50 nucléotides.

Dans d'autres modes de réalisation, l'acide nucléique de séquence SEQ ID N°X a une longueur de 43, 44, 45, 46, 47, 48 ou 49 nucléotides de longueur.

Dans certains autres modes de réalisation préférés, l'acide nucléique de séquence SEQ ID N°X a une longueur de 43, 44 ou 45 nucléotides de longueur.

Comme déjà mentionné précédemment, l'acide nucléique de formule (I) a au moins 15 nucléotides de longueur.

Dans certains modes de réalisation, l'acide nucléique de formule (I) a au moins 15, 16, 17, 18, 19, 20, 21, 22, 23, 24, 25, 26, 27, 28, 29, 30, 31, 32, 33, 34, 35, 36, 37, 38, 39, 40,41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80 ou 81 nucléotides de longueur, ce qui englobe les acides nucléiques possédant exactement chacune des longueurs spécifiées.

Lorsque l'entier « x » est égal à 0 et l'entier « y » est égal à 1, les aptamères nucléiques décrits ici, englobent les acides nucléiques comprenant au moins 15 nucléotides consécutifs d'un polynucléotide ayant au moins 40% d'identité en nucléotides avec l'acide nucléique de formule (I-1) suivante

5' -[SEQ ID N°X]-[SEQ ID N°2] -3' (I-1)

Lorsque l'entier « x » est égal à 1 et l'entier « y » est égal à 0, les aptamères nucléiques décrits ici, englobent les acides nucléiques comprenant au moins 15 nucléotides consécutifs d'un polynucléotide ayant au moins 40% d'identité en nucléotides avec l'acide nucléique de formule (I-2) suivante

5'-[SEQ ID N°1] -[SEQ ID N°X]-3' (I-2)

Lorsque l'entier « x » est égal à 0 et l'entier « y » est égal à 0, les aptamères nucléiques décrits ici, englobent les acides nucléiques comprenant au moins 15 nucléotides consécutifs d'un polynucléotide ayant au moins 40% d'identité en nucléotides avec l'acide nucléique de formule (I-3) suivante :

5'-[SEQ ID N°X]-3' (I-3)

Les aptamères nucléiques ci-dessus englobent donc les acides nucléiques comprenant au moins 15 nucléotides consécutifs d'un polynucléotide ayant au moins 40% d'identité en nucléotides avec un acide nucléique de séquence SEQ ID N°3.

De manière générale, un premier polynucléotide ayant au moins 40% d'identité en nucléotides avec un second polynucléotide ou acide nucléique possède au moins 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% ou 100% d'identité en nucléotides avec ledit second polynucléotide ou acide nucléique.

Il est décrit ici un acide nucléique comprenant la séquence SEQ ID N°X, ladite séquence SEQ ID N°X est choisie dans le groupe constitué des acides nucléiques ayant au moins 15 nucléotides consécutifs d'une séquence possédant au
moins 40% d'identité en nucléotides avec une séquence choisie parmi les séquences SEQ ID N°3, 6 à 35, 37 et 38.

Il est aussi décrit ici un acide nucléique comprenant la séquence SEQ ID N° X, ladite séquence SEQ ID N°X est choisie dans le groupe constitué des
acides nucléiques possédant au moins 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% ou 100% d'identité en nucléotides avec une séquence choisie parmi les séquences SEQ ID N°3, 6 à 35, 37 et 38.

Selon certains aspects de la présente description, ledit aptamère est choisi parmi les acides nucléiques comprenant une séquence d'au moins 15 nucléotides consécutifs d'une séquence possédant au moins 40% d'identité en nucléotides avec une séquence choisie parmi les séquences SEQ ID N°3, 4, 6 à 39.

Selon certains aspects de la présente description , ledit aptamère est choisi parmi les acides nucléiques comprenant une séquence d'au moins 15 nucléotides consécutifs d'une séquence choisie parmi les séquences SEQ ID N°3, 4, 6 à 39.

Selon certains aspects de la présente description , ledit aptamère est choisi parmi les acides nucléiques comprenant une séquence possédant au
moins 40% d'identité en nucléotides avec une séquence choisie parmi les séquences SEQ ID N°3, 4, 6 à 39.

Selon certains aspects de la présente description , ledit aptamère est choisi parmi les acides nucléiques comprenant une séquence choisie parmi les séquences SEQ ID N°3, 4, 6 à 39.

Selon l'invention, ledit aptamère est choisi parmi les acides nucléiques consistant en une séquence choisie parmi les séquences SEQ ID N°3, 4, 6 à 36.

De manière générale, il est décrit ici une famille d'acides nucléiques monobrin ayant au moins 15 nucléotides consécutifs de l'enchaînement de formule (I) défini ci-dessus.

Le « pourcentage d'identité » entre deux séquences d'acides nucléiques, au sens de la présente description, est déterminé en comparant les deux séquences alignées de manière optimale, à travers une fenêtre de comparaison.

La partie de la séquence nucléotidique dans la fenêtre de comparaison peut ainsi comprendre des additions ou des délétions (par exemple des « gaps ») par rapport à la séquence de référence (qui ne comprend pas ces additions ou ces délétions) de manière à obtenir un alignement optimal entre les deux séquences.

Le pourcentage d'identité est calculé en déterminant le nombre de positions auxquelles une base nucléique identique est observé pour les deux séquences comparées, puis en divisant le nombre de positions auxquelles il y a identité entre les deux bases nucléiques par le nombre total de positions dans la fenêtre de comparaison, puis en multipliant le résultat par cent afin d'obtenir le pourcentage d'identité en nucléotides des deux séquences entre elles.

L'alignement optimal des séquences pour la comparaison peut être réalisé de manière informatique à l'aide d'algorithmes connus.
De manière tout à fait préférée, le pourcentage d'identité de séquence est déterminé à l'aide du logiciel CLUSTAL W (version 1.82) les paramètres étant fixés comme suit : (1) CPU MODE = ClustalW mp ; (2) ALIGNMENT = « full » ; (3) OUTPUT FORMAT = « aln w/numbers » ; (4) OUTPUT ORDER = « aligned » ; (5) COLOR ALIGNMENT = « no » ; (6) KTUP (word size) = « default » ; (7) WINDOW LENGTH = « default » ; (8) SCORE TYPE = « percent » ; (9) TOPDIAG = « default » ; (10) PAIRGAP = « default » ; (11) PHYLOGENETIC TREE/TREE TYPE = « none » ; (12) MATRIX = « default » ; (13) GAP OPEN = « default » ; (14) END GAPS = « default »; (15) GAP EXTENSION = « default »; (16) GAP DISTANCES = « default » ; (17) TREE TYPE = « cladogram » et (18) TREE GRAP DISTANCES = « hide ».
A titre illustratif et pour certains modes de réalisation d'un aptamère nucléique utiles dans l'invention, l'homme du métier peut aisément, sur la base de la définition structurelle ci-dessus des aptamères nucléiques de formule (I), générer de manière automatique, par exemple à l'aide d'un calculateur numérique dont la mémoire est chargée avec un ensemble d'instructions approprié, l'ensemble des séquences SEQ ID N°X possibles. Le cas échéant, l'homme du métier peut ensuite déterminer automatiquement, à l'aide dudit calculateur numérique, respectivement (i) celles des séquences dont le modèle de structure dans l'espace est similaire ou identique à celle de l'aptamère nucléique anti-GLA de séquence SEQ ID N°4, et (ii) celles dont la structure de l'aptamère nucléique est distincte de celle de l'aptamère nucléique anti-GLA de séquence SEQ ID N°4.

Les aptamères nucléiques ayant une structure dans l'espace similaire ou identique à celle de l'aptamère nucléique anti-GLA de séquence SEQ ID N° 4 englobent ceux qui comprennent un enchaînement de boucles et de tiges similaire ou identique à celui-ci.

Pour déterminer la structure secondaire dans l'espace d'un acide nucléique de formule (I), l'homme du métier peut notamment, sur la base de la description de sa séquence en nucléotides, générer une modèlisation en utilisant le programme d'ordinateur mFold® décrit par Zuker (2003, Nucleic Acids Research, Vol. 231(13) : 3406-3413), et qui peut aussi être utilisé à l'adresse Web suivante : http://mfold.bioinfo.rpi.edu/.

Préférentiellement, on utilise le programme d'ordinateur mFold , selon les paramètres suivants : (i) ADN à séquence linéaire, (ii) température de repliement : 25°C, (iii) conditions ioniques : [Na⁺] : 150 mM; [Mg⁺⁺] : 4 mM, (iv) type de correction : Oligomère, (v) nombre du pourcentage de « suboptimality » : 2, (vi) limite supérieure du nombre de repliements calculés : 50, (vii) distance maximale entre deux paires de bases : pas de limite, et (viii) utilisation des valeurs par défaut pour les autres paramètres.

Puis, l'homme du métier réalise une étape de comparaison entre (i) le modèle de structure de l'aptamère et (ii) le modèle de structure de l'aptamère de formule (I) qui vient d'être généré, et il sélectionne positivement l'aptamère de formule (I) qui vient d'être généré si son modèle structurel est identique ou similaire à celui de l'aptamère anti-GLA de séquence SEQ ID N°4.

Dans tous les cas, afin de confirmer la sélection positive de l'aptamère de formule (I) nouvellement généré, l'homme du métier peut vérifier ses propriétés de liaison à une protéine de la coagulation à domaine GLA, choisie parmi le Facteur VII/VIIa humain, le Facteur IX/Ixa humain et le Facteur X/Xa humain, par exemple selon l'une des méthodes spécifiées dans la présente description, en particulier dans les exemples.

Dans certains modes de réalisation préférés d'un aptamère nucléique anti-GLA décrits ici, ledit aptamère nucléique comprend au moins 15 nucléotides consécutifs d'un polynucléotide ayant au moins 80% d'identité en nucléotides avec l'acide nucléique de formule (I), ce qui englobe les aptamères comprenant 15 nucléotides consécutifs d'un polynucléotide ayant au moins 81%, 82%, 83%, 84%, 85%, 86%, 87%, 88%, 89%, 90%, 91%, 92%, 93%, 94%, 95%, 96%, 97%, 98% ou 100% d'identité en nucléotides avec ledit acide nucléique de formule (I).

Les aptamères nucléiques selon l'invention englobent l'aptamère nucléique de séquence SEQ ID N°4. On rappelle que l'aptamère de séquence SEQ ID N°4 consiste en un aptamère de formule (I) dans laquelle la séquence SEQ ID N° X consiste en la séquence SEQ ID N°3. On rappelle aussi qu'un aptamère de séquence SEQ ID N°3 consiste en un aptamère de formule (I-3) dans lequel les séquences SEQ ID N°1 et SEQ ID N°2 sont absentes.

Le demandeur a montré que l'aptamère de séquence SEQ ID N° 3 possède une capacité à se lier sélectivement aux protéines de la coagulation à domaine GLA similaire à celle de l'aptamère de séquence SEQ ID N°4. Ces ré sultats montrent le caractère essentiel de la présence de l'acide nucléique de séquence SEQ ID N°3 dans les propriétés de liaison spécifique de l'aptamère de séquence SEQ ID N° 4. De manière générale, ces résultats montrent le caractère essentiel de l'acide nucléique de séquence SEQ ID N° X dans un aptamère de formule (I), l'acide nucléique de séquence SEQ ID N°X conférant à l'aptamère de formule (I) l'aptitude à se lier sélectivement aux protéines de la coagulation à domaine GLA actives.

Les aptamères nucléiques de l'invention englobent donc aussi l'aptamère de séquence SEQ ID N°3.

On a aussi montré dans les exemples qu'une grande variété d'aptamères, incluant les aptamères de séquences SEQ ID N°6 à 35 possèdent u ne capacité à se lier sélectivement aux protéines de la coagulation à domaine GLA, le cas échéant avec des niveaux d'affinité distincts. A titre illustratif, parmi les aptamères de séquences SEQ ID N° 6 à 35, l'aptamère ayant la plus grande capacité à se lier au Facteur IX humain est l'aptamère de séquence SEQ ID N°35, qui peut être aussi désigné « Mapt-1.2.-C S ».

On a aussi identifié dans les exemples des aptamères ayant une capacité à se lier aux protéines de la coagulation à domaine GLA encore supérieure à l'aptamère Mapt-1.2.-CS ci-dessus. Un exemple d'un tel aptamère est l'aptamère de séquence SEQ ID N°36, qui peut être aussi désigné « Mapt-1.2.-CSO ».

L'aptamère Mapt-1.2.-CSO de séquence SEQ ID N°36 est un aptamère comprenant au moins 15 nucléotides consécutifs d'un polynucléotide ayant au moins 40% d'identité en nucléotides avec l'acide nucléique de 62 nucléotides de longueur de formule (I), suivante :

5'-[SEQ ID N°1]-[SEQ ID N35]-3',

et consistant en l'acide nucléique allant du nucléotide en position 10 jusqu'au nucléotide en position 49 dudit acide nucléique de formule (I).

On a aussi montré dans les exemples que chacun des acides nucléiques de séquences SEQ ID N°37 à 39 possédait la capacité à se lier aux protéines de la coagulation à domaine GLA. L'aptamère de séquence SEQ ID N°39 peut être aussi désigné « Mapt-2 ». L'aptamère de séquence SEQ ID N°37 peut être aussi désigné « Mapt-2-CS ». L'aptamère de séquence SEQ ID N° 38 peut être aussi désigné « Mapt-2.2.-CS ». On précise que la séquence de l'aptamère Mapt-2CS est incluse dans la séquence de l'aptamère Mapt-2 : il s'agit de la « core sequence » SEQ ID N°X de l'aptamère Mapt-2.

On a montré dans les exemples que les aptamères anti-GLA décrits ici peuvent être utilisés avec succès pour la fabrication de supports de chromatographie d'affinité, utiles pour séparer les protéines de la coagulation à domaine GLA d'un échantillon des autres protéines.

Pour la réalisation de supports de chromatographie d'affinité notamment, les aptamères nucléiques anti-GLA décrits ici sont préférentiellement inclus dans une structure chimique, aussi appelée « composé » dans la présente description, qui comprend notamment un moyen espaceur et, le cas échéant, un moyen d'immobilisation sur un support solide.

Dans certains modes de réalisation, l'aptamère nucléique est inclus dans la structure d'un composé de formule (II) suivante :

[IMM]ₓ-[SPAC]_{y}- [APT] (II),

dans laquelle :
- [IMM] signifie un composé d'immobilisation sur un support,
- [SPAC] signifie une chaîne espaceur,
- [APT] signifie un aptamère anti-GLA tel que défini dans la présente description,
- x est un entier égal à 0 ou 1, et
- y est un entier égal à 0 ou 1.

Dans certains modes de réalisation du composé de formule (II), [APT] consiste en un acide désoxyribonucléique dont la séquence est choisie parmi les séquences SEQ ID N°3, 4, 6 à 36.

La « chaîne espaceur » désignée [SPAC] dans le composé de formule (II) peut être de tout type connu. Ladite chaîne espaceur a pour fonction d'éloigner physiquement l'acide nucléique de la surface du support solide sur lequel ledit composé peut être immobilisé et de permettre une mobilité relative de l'acide nucléique par rapport à la surface du support solide sur lequel il peut être immobilisé. La chaîne espaceur limite ou évite que des encombrements stériques, dus à une trop grande proximité du support solide et de l'acide nucléique, gênent les évènements de liaison entre ledit acide nucléique et des molécules de protéine de la coagulation susceptibles d'être mises en contact avec celui-ci.

Dans le composé de formule (II), la chaîne espaceur est liée préférentiellement à l'extrémité 5' ou à l'extrémité 3' de l'acide nucléique aptamère.

Avantageusement la chaîne espaceur est liée à la fois à une extrémité de l'aptamère et au support solide. Cette construction avec espaceur a pour avantage de ne pas immobiliser directement l'aptamère sur le support solide. De préférence la chaîne espaceur est un oligonucléotide non spécifique ou du Polyéthylène Glycol (PEG) ou autre chaine hydrocarbonée hydrophile. Lorsque la chaîne espaceur consiste en un oligonucléotide non spécifique, ledit oligonucléotide comprend avantageusement au moins 5 nucléotides de longueur, de préférence entre 5 et 15 nucléotides de longueur.

Dans les modes de réalisation d'un composé de formule (II) dans lesquels la chaîne espaceur consiste en un Polyéthylène Glycol, ladite chaîne espaceur englobe un polyéthylène glycol de type PEG(C18), commercialisé par exemple par la Société Sigma Aldrich.

Comme cela est illustré dans les exemples, la purification de protéines de la coagulation à domaine GLA est réalisée à la fois avec des composés de formule (II) comprenant une chaîne espaceur [SPAC] et avec des composés de formule (II) ne possédant pas de chaîne espaceur [SPAC].

Pour immobiliser l'aptamère sur la chaîne espaceur, l'acide nucléique peut être modifié chimiquement avec différents groupements chimiques tels que des groupements permettant d'immobiliser ledit acide nucléique de façon covalente, tels que des thiols, des amines ou tout autre groupement capable de réagir avec des groupements chimiques présents sur le support solide.

Dans le composé de formule (II) le composé [IMM] consiste en un composé choisi parmi (i) un composé capable de former une ou plusieurs liaisons covalente(s) avec le matériau de support solide et (ii) un composé capable de se lier spécifiquement sur le support solide par l'intermédiaire de liaison faibles non covalentes, y compris des liaisons hydrogènes, des forces électrostatiques ou des forces de Van der Waals.

Dans certains cas, le composé [IMM], du fait qu'il est constitué d'une chaîne d'atomes liés entre eux par des liaisons covalentes, peut se comporter comme une chaîne espaceur. Toutefois, par définition, un composé [IMM] ne peut jamais signifier une chaîne [SPAC] dans un composé de formule (II) décrit ici. En d'autres termes, dans un composé de formule (II), la chaîne [SPAC], lorsqu'elle est présente, ne peut être liée directement au support de chromatographie, que ce soit par des liaisons covalentes ou des liaisons faibles non covalentes.

Le premier type de composé [IMM] englobe les agents de couplage bifonctionnels, comme le glutaraldéhyde, le SIAB ou encore le SMCC.

Le composé SIAB, décrit par Hermanson G.T. (1996, Bioconjugate techniques, San Diego : Academic Press, pp 239-242), est le composé de formule (A) suivante :

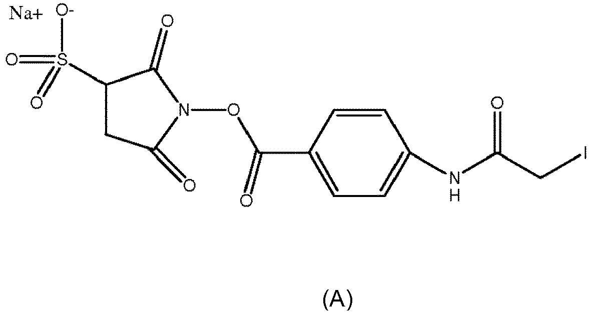

Le composé SIAB comprend deux groupes réactifs, respectivement un groupe iodoacétate et un groupe ester Sulfo-NHS, ces groupes réagissant respectivement sur des groupes amino et sulfhydryl.

Le composé SMCC, qui est décrit par Samoszuk M.K. et al. (1989, Antibody, Immunoconjugates Radiopharm., 2(1) : 37-46), est le composé de formule (B) suivante :

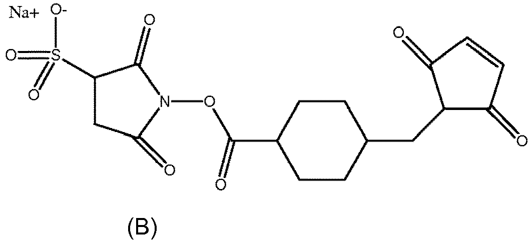

Le composé SMCC comprend deux groupes réactifs, respectivement un groupe ester Sulfo-NHS et un groupe maléimide, qui réagissent respectivement avec un groupe amino et un groupe sulfhydryl.

Le second type de composé [IMM] englobe la biotine, qui est capable de se lier de manière spécifique non covalente à des molécules d'avidine ou de streptavidine présentes sur le support solide.

Une fois immobilisé sur le support solide via l'espaceur, l'aptamère anti-GLA est avantageusement modifié à son extrémité libre (extrémité non liée à l'espaceur) grâce à, et sans s'y limiter, un nucléotide chimiquement modifié (tel que 2'omethyl ou 2'fluoropyrimidine, 2' ribopurine, phosphoramidite), un nucléotide inversé ou un groupement chimique (PEG, polycations, cholestérol). Ces modifications permettent de protéger l'aptamère anti-GLA des dégradations enzymatiques.

Le support solide peut être une colonne de chromatographie d'affinité composée d'un gel dérivé de l'agarose, de la cellulose ou d'un gel synthétique tel qu'un dérivé acrylamide, méthacrylate ou polystyrène, une puce telle qu'une puce adaptée à la résonance plasmonique une bille magnétique ou paramagnétique.

Il est également décrit ici un complexe entre :
(i) un aptamère nucléique choisi parmi un acide nucléique de formule (I), et un composé de formule (II), et
(ii) une protéine de la coagulation à domaine GLA,

Il est aussi décrit ici un support pour l'immobilisation d'une protéine
de la coagulation à domaine GLA, caractérisé en ce qu'il comprend un matériau support solide sur lequel sont greffées une pluralité de molécules consistant chacune en, ou comprenant chacune, un aptamère nucléique, lesdites molécules étant choisies parmi (a) un acide nucléique de formule (I), et (b) un composé de formule (II).

Le support ci-dessus peut être utilisé pratiquement dans toutes les applications pour lesquelles on cherche à immobiliser une protéine de la coagulation à domaine GLA, y compris une protéine de la coagulation à domaine GLA humaine, ce qui englobe les applications à des fins de purification de ladite protéine de la coagulation à domaine GLA et les applications à des fins de détection de ladite protéine de la coagulation à domaine GLA.
La réalisation de supports sur lesquels sont immobilisés des aptamères nucléiques utilses dans l'invention se liant spécifiquement à une protéine de la coagulation à domaine GLA sont largement illustrés dans les exemples, dans lesquels les aptamères utiles dans l'invention sont utilisés notamment comme agents de capture, utilisables pour la purification ou pour la détection d'une protéine de la coagulation à domaine GLA, par exemple une protéine humaine de la coagulation à domaine GLA, dans des échantillons.

La présente description fournit donc aussi un procédé pour l'immobilisation d'une protéine de la coagulation à domaine GLA sur un support, comprenant une étape au cours de laquelle on met en contact un échantillon comprenant au moins une protéine de la coagulation à domaine GLA avec un support solide sur lequel a été préalablement immobilisée une substance choisie parmi un acide nucléique de formule (I), et un composé de formule (II). Ledit procédé peut comprendre, selon les objectifs techniques poursuivis, une étape additionnelle de récupération des molécules de protéine(s) de la coagulation à domaine GLA immobilisées, complexées avec les molécules d'acides nucléiques de formule (I). L'étape additionnelle de récupération de la protéine de la coagulation à domaine GLA, ou des protéines à domaine GLA, consiste préférentiellement en une étape de mise en contact des complexes de protéine(s) de la coagulation à domaine GLA avec les acides nucléiques de formule (I) avec un agent chélateur des cations métalliques, tel que l'EDTA.

Pour la réalisation de procédés de purification de protéines par chromatographie d'affinité en utilisant des supports solides sur lesquels sont immobilisés les aptamères d'intérêt, l'homme du métier peut se référer aux travaux décrits par Romig et al. (1999, J Chomatogr B Biomed Sci Apl, Vol. 731(2) : 275-284).

De plus, la fabrication d'un support d'affinité comprenant un aptamère nucléique utile dans l'invention et la réalisation d'un procédé de purification du Facteur IX humain avec ledit support d'affinité est illustré dans les exemples.

De manière générale, les supports solides sur lesquels peuvent être immobilisés les aptamères décrits ici englobe tout type de support ayant la structure et la composition retrouvée communément pour les supports de filtre, de support de silicium pour des puces, des membranes, etc. Les supports solides englobent notamment les résines, les résines pour colonne de chromatographie d'affinité, les billes de polymères, les billes magnétiques, etc. Les supports solides englobent aussi notamment les matériaux à base de verre ou de métal, tels que l'acier, l'or, l'argent, l'aluminium, le cuivre, le silicium, le verre, la céramique. Les supports solides englobent aussi notamment des matériaux polymères, tels qu'un polyéthylène, un polypropylène, un polyamide, un fluorure de polyvinylidène, et des combinaisons de ceux-ci.

Le support solide peut être revêtu avec un matériau facilitant l'attachement, la fixation, la formation de complexes, l'immobilisation ou l'interaction avec les aptamères.

Dans certains modes de réalisation, le support solide est une lame de verre dont la surface est revêtue d'une couche d'or, d'une couche ayant subi un traitement par carboxyméthylation, d'une couche de dextran, de collagène, d'avidine, de streptavidine, etc.

De cette manière, les aptamères utiles dans l'invention peuvent être immobilisés sur le support solide par l'intermédiaire d'un revêtement d'attachement, comme par exemple décrit ci-dessus, soit par réaction chimique avec création de liaisons covalentes, soit par association par des liaisons non covalentes telles que des liaisons hydrogène, des forces électrostatiques, des forces de Van der Waals, etc.

Les exemples décrivent des modes de réalisation de supports solides sur lesquels sont immobilisés, par des liaisons non covalentes, les aptamères utiles dans l'invention.

Dans les exemples, on décrit notamment des supports solides consistant en une lame de verre revêtue d'une couche de molécules de streptavidine, et des aptamères utiles dans l'invention conjugués à une molécule de biotine qui sont immobilisé sur le support par association non covalente biotine/streptavidine.

Dans les exemples, on décrit aussi des supports solides consistant en un matériau de polystyrène revêtu d'une couche de molécules de streptavidine, et des aptamères utiles dans l'invention conjugués à une molécule de biotine qui sont immobilisé sur le support par association non covalente biotine/streptavidine.

Dans certains modes de réalisation, les aptamères utiles dans l'invention peuvent être immobilisés sur un support solide adapté pour la chromatographie d'affinité, l'électrochromatographie et l'électrophorèse capillaire, comme décrit par exemple par Ravelet et al. (2006, J Chromatogr A, Vol. 117(1): 1-10), Connor et al. (2006, J Chromatogr A, Vol. 111(2): 115-119), Cho et al. (2004, Electrophoresis, Vol. 25 (21-22) : 3730-3739) ou encore Zhao et al. (2008, Anal Chem, Vol. 80(10) : 3915-3920).

Un aptamère de formule (I) ayant au moins 15 nucléotides de longueur et se liant spécifiquement aux protéines de la coagulation à domaine GLA, ou un composé de formule (II), peut être aussi avantageusement utilisé comme agent de capture de protéine(s) de la coagulation à domaine GLA, dans des procédés et dispositifs de détection ou de diagnostic.

Selon encore un autre aspect, la présente description fournit aussi un procédé pour détecter la présence d'une ou plusieurs protéine(s) à domaine GLA dans un échantillon comprenant les étapes suivantes :
a) mettre en contact (i) un acide nucléique de formule (I), ou un composé de formule (II) ou un support solide sur lequel sont immobilisés une pluralité de molécules dudit acide nucléique ou dudit composé, avec (ii) ledit échantillon; et
b) détecter la formation de complexes entre (i) ledit acide nucléique de formule (I), ou ledit composé de formule (II) ou ledit support et (ii) la ou les protéine(s) de la coagulation à domaine GLA.

Les exemples de la présente demande de brevet fournissent divers modes de réalisation de procédés de détection du FIX/FIXa humain avec des aptamères tels que décrits ici, préalablement immobilisés sur un support solide.

Pour la réalisation d'un procédé de détection tel que décrit ici, le support solide utilisé peut être un support solide choisi parmi les supports solides décrits précédemment en relation avec le procédé de purification de protéines de la coagulation à domaine GLA.

Pour la réalisation d'un procédé ou d'un dispositif de détection de protéines de la coagulation à domaine GLA, l'homme du métier peut se référer notamment aux techniques décrites dans la demande de brevet européen n° EP 1 972 693, la demande PCT n° WO 2008/038696, la demande PCT n° WO 2008/025830, ou encore la demande PCT n° WO 2007/0322359.

Dans certains modes de réalisation, l'étape b) de détection de la formation de complexes entre (i) ledit acide nucléique ou ledit support solide et (ii) la ou les protéine(s) de la coagulation à domaine GLA peut être réalisé par mesure du signal de résonance plasmonique de surface, comme cela est décrit dans les exemples.

Dans certains autres modes de réalisation, l'étape b) de détection de la formation de complexes entre (i) ledit acide nucléique ou ledit support solide et (ii) la ou les protéine(s) de la coagulation à domaine GLA peut être réalisée par la mise en contact desdits complexes éventuellement formés avec un ligand spécifique d'une protéine de la coagulation à domaine GLA, ledit ligand étant détectable. Dans les exemples, on décrit ces modes de réalisation dans lesquels on utilise, comme ligand détectable d'une protéine de la coagulation à domaine GLA, des anticorps monoclonaux ou polyclonaux anti-protéine de la coagulation à domaine GLA, marqués avec une enzyme, en l'occurrence la peroxydase de raifort, comme cela est
conventionnellement utilisé dans les tests de type ELISA. Comme anticorps spécifiques de protéines de la coagulation à domaine GLA, on peut utiliser, selon les objectifs qui sont poursuivis, (i) soit des anticorps dirigés spécifiquement contre une protéine de la coagulation à domaine GLA déterminée, par exemple des anticorps anti-FII humain, anti-FVII humain, anti-FIX humain, anti-FX humain, anti-protéine C humaine ou anti-protéine S humaine (ii) soit des anticorps dirigés contre un domaine GLA de protéine de coagulation à domaine GLA, capables de reconnaître une pluralité de protéines de la coagulation à domaine GLA, comme décrits par exemple dans le brevet américain N°US 7,439,025.

De manière surprenante, il est montré que l'on peut fabriquer un support d'affinité tel que défini dans la présente description en utilisant, comme aptamères
nucléiques anti-GLA, des aptamères ADN, pourtant considérés dans l'état de la technique comme des acides nucléiques ligands dont la mise en oeuvre est malaisée, et dont la spécificité pour la protéine cible est moindre que la spécificité de la molécule d'ARN de séquence correspondante. Notamment, il est admis dans l'état de la technique que le ADN ligands ont une flexibilité moindre que l'ARN correspondant et qu'en conséquence sont moins aptes que les ARN ligands à subir des changements de conformation. On rappelle que lorsqu'un aptamère nucléique se lie à la protéine cible, il se produit un changement de conformation. Il a aussi été décrit que plus le changement de conformation de l'aptamère nucléique est rapide et plus l'affinité dudit aptamère nucléique pour la protéine cible est élevée (Michaud et al., 2003, Anal Chem, Vol. 76 : 1015-1020 ; Brumbt et al., 2005, Anal Chem, Vol. 77 : 1993-1998).

Ainsi, dans les supports d'affinité utiles dans l'invention, les aptamères anti-GLA consistent en des aptamères ADN.

En conséquence, dans les supports d'affinité décrits ici, lesdits acides nucléiques immobilisés, le cas échéant inclus dans la structure d'un
composé de formule (I), consistent en des acides désoxyribonucléiques.

Dans certains aspects de la description, lesdits acides nucléiques consistent, pour une première partie d'entre eux, en des acides désoxyribonucléiques, et pour la partie restante en des acides ribonucléiques.

Un autre avantage des aptamères nucléiques concerne leur facilité de fabrication, comparée aux difficultés de synthèse des aptamères ARN, ainsi que leur prix de revient qui est significativement plus réduit que le prix de revient d'un aptamère ARN.

Ces modes de réalisation spécifiques sont illustrés dans les exemples.

La présente description fournit aussi un dispositif de chromatographie d'affinité pour la purification d'une protéine de la coagulation à domaine GLA, comprenant un conteneur dans lequel est disposé une quantité adaptée d'un support d'affinité tel que défini dans la présente description.

Des formes variées de conteneurs pour supports de chromatographie sont connues dans l'état de la technique et sont englobées par la signification du terme « conteneur » ci-dessus. Les caractéristiques importantes d'un tel conteneur englobent la présence d'un moyen d'alimentation du dispositif de chromatographie d'affinité en échantillon de départ et d'un moyen de sortie du liquide après sa mise en contact avec le support d'affinité.

Il est aussi décrit ici un procédé pour immobiliser une protéine de la coagulation sur un support, comprenant une étape au cours de laquelle on met en contact un échantillon contenant ladite protéine de la coagulation avec un support d'affinité tel que défini ci-dessus.

Dans certains modes de réalisation du procédé de purification ci-dessus, ledit échantillon consiste en une solution biologique liquide provenant d'un animal tel que du sang, un dérivé du sang (dérivé sanguin).
Ledit échantillon peut consister en du plasma, du cryoprécipité du plasma.

Selon l'invention, le procédé de purification décrit ici est également parfaitement adapté à l'obtention d'une protéine de la coagulation à domaine GLA purifiée à partir d'un échantillon de plasma sanguin humain, ou d'une fraction de plasma sanguin humain, par exemple la fraction cryoprécipitée de plasma sanguin humain.

Dans certains modes de réalisation du procédé de purification ci-dessus, la protéine de la coagulation à domaine GLA cible est humaine.

Dans certains modes de réalisation du procédé de purification ci-dessus, l'échantillon comprend au moins une protéine de la coagulation à domaine GLA non-humaine.

Dans certains modes de réalisation du procédé de purification ci-dessus, ladite protéine de la coagulation à domaine GLA humaine est homologue à ladite protéine de la coagulation à domaine GLA non-humaine.

Dans certains modes de réalisation du procédé de purification ci-dessus, ladite protéine de la coagulation à domaine GLA humaine est l'homologue de ladite protéine de la coagulation à domaine GLA non-humaine

Dans certains modes de réalisation du procédé de purification ci-dessus, l'échantillon de départ peut consister en le matériel brut, soit l'échantillon de plasma sanguin humain, ou une fraction de celui-ci, soit le fluide corporel d'un mammifère non humain transgénique pour la protéine de la coagulation à domaine GLA d'intérêt, et qui contient ladite protéine de la coagulation à domaine GLA d'intérêt à purifier.

Toutefois, le mode de réalisation ci-dessus n'est pas le mode de réalisation privilégié du procédé de purification décrit ici, notamment en raison du risque de colmatage du support d'affinité par les nombreuses protéines présentes dans l'échantillon brut de départ.

Dans des modes de réalisation préférés, ledit échantillon de départ consiste en une solution liquide contenant la protéine de la coagulation à domaine GLA d'intérêt en suspension dans ladite solution, ladite solution liquide consistant en un produit intermédiaire généré au cours d'un procédé de purification multi-étapes d'une protéine de la coagulation à domaine GLA, par exemple d'une protéine de la coagulation.

A titre illustratif, pour un procédé de purification d'une protéine de la coagulation à domaine GLA à partir d'un fluide corporel d'un mammifère non humain transgénique pour ladite protéine, l'échantillon de départ peut consister en un éluat d'une étape de chromatographie d'interaction hydrophobe, telle qu'une étape de chromatographie dans laquelle on utilise un support chromatographique du type MEP HyperCel®. Ce mode particulier de réalisation d'un procédé de purification décrit ici est illustré dans les exemples.

De la même manière, pour un procédé de purification de la protéine de la coagulation à domaine GLA d'intérêt à partir de plasma humain, l'échantillon de départ peut consister en un filtrat d'une étape de filtration en profondeur pratiquée sur la fraction cryoprécipitée d'un échantillon de plasma humain.

De manière générale, les conditions d'utilisation du support d'affinité pour réaliser le procédé de purification décrit ici sont très similaires aux conditions d'utilisation conventionnelles d'un support de chromatographie classique, par exemple du type support d'immuno-affinité sur lequel sont immobilisé des anticorps ligands. L'homme du métier peut par
exemple se référer à l'ouvrage de Bailon et al. (Pascal Bailon, George K. Ehrlich, Wen-Jian Fung and Wolfgang Berthold, An Overview of Affinity Chromatography, Humana Press, 2000).

Toutefois, comme cela sera détaillé dans la suite de la description, les conditions de l'étape c) d'élution du procédé décrit ici sont très avantageuses, pour la purification d'une protéine de la coagulation à domaine GLA.

A l'étape a), un volume approprié de l'échantillon à purifier est mis en contact avec le support d'affinité. Des complexes sont formés entre (i) les aptamères anti-GLA immobilisés sur ledit support d'affinité et (ii) la protéine de la coagulation à domaine GLA d'intérêt contenue dans l'échantillon à purifier.

On a montré dans les exemples que les conditions de liaison des aptamères anti-GLA aux protéines de la coagulation à domaine GLA sont favorisées en présence de calcium, par exemple de calcium sous la forme de chlorure de calcium.

Ainsi, dans certains modes de réalisation de l'étape a), on utilise une solution tampon comprenant une concentration finale en CaCl₂ d'au moins 1 mM, ce qui englobe au moins 2 mM, 3 mM, 4 mM, 5 mM, 6 mM, 7 mM, 8 mM, 9 mM, 10 mM, 11 mM, 12 mM, 13 mM, 14 mM et
15 mM. A l'étape a) on utilise avantageusement une solution tampon comprenant une concentration finale en CaCl₂ d'au plus 50 mM.

On a aussi montré dans les exemples que les conditions de liaison des aptamères anti-GLA aux protéines de la coagulation à domaine GLA sont favorisées en l'absence de chlorure de magnésium.

Ainsi, dans certains modes de réalisation de l'étape a), on utilise une solution tampon qui ne comprend pas de chlorure de magnésium.

A titre illustratif, on a montré dans les exemples qu'un support de chromatographie sur lequel est immobilisé un aptamère anti-GLA décrit ici, l'affinité dudit support pour une
protéine de la coagulation à domaine GLA est accrue pratiquement d'un facteur dix lorsqu'on utilise à l'étape a) une solution tampon dépourvue de chlorure de magnésium, en l'occurrence une solution tampon de Tris-HCl 50mM et CaCl₂ à pH 7,5, par comparaison avec une solution tampon identique mais comprenant du chlorure de magnésium..

Dans des modes de réalisation préférés du procédé, on procède, à la fin de l'étape a), et préalablement à l'étape b) décrite en détail plus loin, à une ou plusieurs étapes de lavage du support d'affinité de manière à éliminer les substances qui ne sont pas retenues de manière spécifique, par exemple les substances simplement adsorbées sur ledit support. On peut utiliser un tampon de lavage conventionnel, bien connu de l'homme du métier.

Toutefois, dans certains modes de réalisation de la ou des étape(s) de lavage, on peut utiliser un tampon de lavage comprenant du NaCl, et/ou de l'éthylène glycol, et/ou du propylène glycol, et/ou de l'éthanol.

On a montré dans les exemples que l'utilisation d'une solution de lavage comprenant du NaCl n'entraîne pas d'altération dans la liaison spécifique des protéines de la coagulation à domaine GLA aux aptamères anti-GLA immobilisés. On a montré en particulier qu'une concentration finale de NaCl de 3M ne perturbe pas ladite liaison spécifique.

Ainsi, dans certains modes de réalisation de la ou des étapes de lavage préalables à l'étape b), on utilise une solution de lavage ayant une concentration finale en NaCl d'au moins 0,5 M, ce qui inclut au moins 1 M, 1,5 M, 2 M, 2,5 M et 3,0 M. La concentration finale de NaCl est avantageusement d'au plus 4M.

Les résultats des exemples montrent ainsi que la capacité des aptamères anti-GLA décrits ici à se lier aux protéines de la coagulation à domaine GLA n'est pas altérée lorsqu'on soumet les complexes formés entre les aptamères immobilisés et la ou les protéines à domaine GLA à une environnement de haute force ionique, ce qui est un résultat tout à fait inattendu. On rappelle en effet que l'on a recours couramment à un tampon de haute force ionique comme tampon pour l'élution de substances préalablement complexées à des ligands immobilisés d'un support d'affinité, y compris un support d'affinité comprenant des aptamères immobilisés.

Les résultats des exemples montrent ainsi que les aptamères anti-GLA décrits ici ont des caractéristiques spécifiques et inattendues, concernant l'absence d'effet d'une haute force ionique sur leur capacité à se lier à une protéine de la coagulation à domaine GLA.

Sans vouloir être lié par une quelconque théorie, le demandeur pense que ces propriétés inattendues des aptamères anti-GLA décrits ici résultent de la capacité desdits aptamères anti-GLA à se lier spécifiquement au domaine GLA qui est commun aux protéines de la coagulation à domaine GLA. En particulier, le demandeur pense que les aptamères anti-GLA décrits ici se lient aux protéines cibles par l'intermédiaire de ponts non-covalents divalents qui ne peuvent être générés qu'au niveau du domaine GLA, et que la création desdits ponts divalents prévient la fixation subséquente des ions provenant du NaCl sur le domaine GLA.

On a aussi montré dans les exemples que l'utilisation d'une solution de lavage comprenant un alkylène glycol n'entraîne pas d'altération dans la liaison spécifique des protéines de la coagulation à domaine GLA aux aptamère anti-GLA immobilisés.

On a aussi montré dans les exemples que l'utilisation d'une solution de lavage comprenant de l'éthylène glycol n'entraîne pas d'altération dans la liaison spécifique des protéines de la coagulation à domaine GLA aux aptamère anti-GLA immobilisés. On a montré en particulier qu'une concentration finale d'éthylène glycol de 1,5 M ne perturbe pas ladite liaison spécifique.

Ainsi, dans certains modes de réalisation de la ou des étapes de lavage préalables à l'étape b), on utilise une solution de lavage ayant une concentration finale en éthylène glycol d'au moins 0,5 M, ce qui inclut au moins 1 M, et 1,5 M.

On a aussi montré dans les exemples que l'utilisation d'une solution de lavage comprenant du propylène glycol n'entraîne pas d'altération dans la liaison spécifique des protéines de la coagulation à domaine GLA aux aptamère anti-GLA immobilisés. On a montré en particulier qu'une concentration finale de propylène glycol à 50% (v/v) ne perturbe pas ladite liaison spécifique.

Ainsi, dans certains modes de réalisation de la ou des étapes de lavage préalables à l'étape b), on utilise une solution de lavage ayant une concentration finale en propylène glycol d'au moins 10% (v/v), ce qui inclut au moins 15%, 20%, 25%, 30%, 35%, 40%, 45% et 50%.

On a aussi montré dans les exemples que l'utilisation d'une solution de lavage comprenant de l'éthanol n'entraîne pas d'altération dans la liaison spécifique des protéines de la coagulation à domaine GLA aux aptamère anti-GLA immobilisés. On a montré en particulier qu'une concentration finale d'éthanol à 10% (v/v) ne perturbe pas ladite liaison spécifique.

Ainsi, dans certains modes de réalisation de la ou des étapes de lavage préalables à l'étape b), on utilise une solution de lavage ayant une concentration finale en éthanol d'au moins 1% (v/v), ce qui inclut au moins 1,5%, 2,0%, 2,5%, 3,0%, 3,5%, 4,0%, 4,5%, 5,0%, 5,5%, 6,0%, 6,5%, 7,0%, 7,5%, 8,0%, 9,0%, 9,5% et 10,0%.

On a montré qu'un tel tampon de lavage entraîne des conditions drastiques d'élimination des substances qui ne sont pas retenues de manière spécifique sur les aptamères, tout en préservant la liaison spécifique des molécules de la ou des protéines de la coagulation à domaine GLA aux aptamères immobilisés sur le support d'affinité. On rappelle qu'un tel avantage technique lié à l'élimination exclusive ou quasi-exclusive des substances non liées spécifiquement aux ligands immobilisés sur le support d'affinité ne peut être aisément réalisé avec un support d'affinité sur lequel sont immobilisés des anticorps.

L'étape b) consiste en une étape d'élution des molécules de la protéine de la coagulation à domaine GLA d'intérêt ayant formé des complexes avec les aptamères nucléiques anti-GLA au cours de l'étape a).

Comme cela est illustré dans les exemples, un avantage spécifique du procédé de purification ci-dessus est la possibilité de réaliser l'étape d'élution par mise en contact des complexes formés entre (i) les aptamères nucléiques anti-GLA immobilisés sur ledit support d'affinité et (ii) ladite protéine de la coagulation à domaine GLA, avec un agent chélateur des ions divalents, comme l'EDTA.

Cet avantage technique, qui est rendu possible, grâce aux caractéristiques du support d'affinité décrit ici, permet l'élution de la protéine de la coagulation à domaine GLA sans nécessiter un quelconque recours à l'utilisation de conditions drastiques d'élution, susceptibles de dénaturer, au moins partiellement, la protéine de la coagulation à domaine GLA d'intérêt. Lesdites conditions drastiques qui sont évitées englobent l'utilisation d'un pH acide pour l'étape d'élution, ce qui est couramment pratiqué pour les procédés de purification de protéines sur les supports d'affinité connus, et tout particulièrement sur les supports d'affinité comprenant des anticorps immobilisés.

Ainsi, dans certains modes de réalisation du procédé de purification ci-dessus, l'étape b) est réalisée par mise en contact du support d'affinité avec un tampon d'élution contenant un agent chélateur des ions divalents, de préférence l'EDTA.

A titre illustratif, le tampon d'élution peut contenir une concentration finale d'EDTA d'au moins 1 mM et d'au plus 100 mM.

L'expression « au moins 1 mM » englobe au moins 2, 3, 4, 5, 6, 7, 8, 9 ou 10 mM.

L'expression « au plus 100 mM » englobe au plus 29, 28, 27, 26, 25, 24, 23, 22, 21, 20, 19, 18, 17, 16, 15, 14, 13, 12 ou 11 mM.

A l'étape c), on récupère la protéine de la coagulation à domaine GLA d'intérêt est purifiée en collectant le liquide d'éluat obtenu à la fin de l'étape b).

A la fin de l'étape c), on obtient une composition liquide purifiée de la protéine de la coagulation d'intérêt. Ladite composition liquide purifiée peut ensuite être traitée de manière appropriée, selon toute technique connue de conditionnement et de conservation des protéines, y compris par flaconnage direct ou après dilution avec une solution adaptée, ou encore par lyophilisation, préférentiellement dans des conditions stériles et apyrogènes, puis conservation dans des conditions appropriées, à température ambiante, à -4°C ou bien à basse température, selon le type de conditionnement choisi.

Comme cela a déjà été mentionné précédemment dans la présente description, le support d'affinité décrit ici peut, avec les cycles successifs d'utilisation pour la purification d'une protéine de la coagulation à domaine GLA d'intérêt, subir une réduction de sa capacité d'absorption, par exemple du fait que l'étape c) d'élution ne permet pas systématiquement de libérer la totalité des molécules de protéine de la coagulation, ce qui réduit le nombre de sites aptamères libres pour les cycles ultérieurs de purification.

Comme pour la totalité des supports de chromatographie connus, il est donc nécessaire, à des moments appropriés, de réaliser une étape de régénération du support d'affinité, afin de libérer la totalité des molécules de protéine de la coagulation à domaine GLA dudit support, et d'éliminer toute substance pouvant être liée au matériau solide du support d'affinité, en général par fixation non spécifique.

Ainsi, dans certains modes de réalisation, le procédé de purification décrit ici comprend une étape additionnelle d) de régénération du support d'affinité, par mise en contact dudit support d'affinité avec une solution de régénération.

Des tampons variés pour la régénération de support de chromatographie, en particulier de supports de chromatographie d'affinité sont bien connus par l'homme du métier, qui peuvent être utilisés à l'étape d) du procédé. L'homme du métier peut se référer par exemple à l'ouvrage de Mohr et al. (Affinity Chromatography: Practical and Theoretical Aspects, Peter Mohr, Klaus Pommerening, Edition: illustrated, CRC Press, 1985).

A titre illustratif, l'étape d) de régénération du support d'affinité peut être réalisée par mise en contact dudit support avec une solution tampon de Tris 20 mM, Polyéthylène Glycol 5%, NaCl 1M, par exemple à pH 7,5, comme cela est illustré dans les exemples.

Le procédé de purification ci-dessus permet l'obtention d'une protéine de la coagulation à domaine GLA à un très haut degré de pureté, éventuellement à une degré de pureté supérieur à 99,95% en poids, par rapport au poids total des protéines contenues dans le produit final purifié.

Un autre avantage du procédé de purification ci-dessus, en particulier dans les modes de réalisation dans lesquels l'échantillon de départ consiste en un échantillon comprenant la protéine humaine de la coagulation à domaine GLA d'intérêt sous forme recombinante en mélange avec des protéines produites naturellement par le mammifère transgénique non humain, est que la composition finale comprenant la protéine humaine recombinante d'intérêt à haut degré de pureté est sensiblement exempte de protéines provenant dudit mammifère transgénique, et en particulier sensiblement exempte de protéines dudit mammifère, homologues de ladite protéine humaine de la coagulation à domaine GLA recombinante.

La présente description fournit aussi un aptamère nucléique se liant spécifiquement aux protéines à domaine GLA, dont la capacité à se lier à une protéine cible à domaine GLA n'est pas altérée par un environnement de haute force ionique.

Il est décrit un aptamère nucléique se liant spécifiquement aux
protéines à domaine GLA, dont la capacité à se lier à une protéine cible à domaine GLA n'est pas altérée par un environnement de haute force ionique possédant une concentration finale en NaCl d'au moins 0,5M.

Les protéines à domaine GLA englobent les protéines de la coagulation à domaine GLA.

Ledit « environnement » à haute force ionique englobe une solution tampon à haute force ionique.

Un environnement de haute force ionique inclut un environnement possédant une concentration finale en NaCl d'au moins 1 M, 1,5 M, 2 M, 2,5 M et 3 M. La concentration finale de NaCl est avantageusement d'au plus 4 M.

L'expression « n'est pas altéré » signifie que la liaison entre la protéine cible à domaine GLA et l'aptamère nucléique se liant spécifiquement aux protéines à domaine GLA résiste à un environnement de haute force ionique. Par exemple, au moins 80% des protéines cibles à domaine GLA restent liées à l'aptamère nucléique se liant spécifiquement aux protéines à domaine GLA dans un environnement à haute force ionique, de préférence 85%, 90%, 95% , 96%, 97%, 98%. En particulier, cela signifie qu'une étape de lavage dans un environnement de haute force ionique ne conduit pas à l'élution de plus de 20% des protéines à domaine GLA liées à l'aptamère nucléique se liant spécifiquement aux protéines à domaine GLA, de préférence de plus de 10%, 5% , 4%, 3%, 2%, 1%.

Dans un mode de réalisation particulier, l'aptamère nucléique décrit ici a également la capacité à se lier à une protéine cible à domaine GLA sans être altéré par un environnement hydrophobe tel qu'une solution 10% éthanol ou 50% propylèneglycol.

Ces propriétés peuvent avantageusement être utilisées pour laver très efficacement un support d'affinité sur lequel est immobilisé un aptamère nucléique décrit ici, ce qui permet d'obtenir une meilleure élimination des contaminants liés à la colonne de façon non spécifique. L'amélioration de l'efficacité du lavage permet d'augmenter la pureté des protéines à
domaine GLA que l'on cherche à purifier.

Comme cela a été décrit précédemment dans la présente description et est aussi illustré dans les exemples, on peut dissocier les complexes formés entre les aptamères nucléiques décrits ici et les protéines cibles à domaine GLA par mise en contact desdits complexes avec une solution comprenant un chélateur d'ions divalents, par exemple de l'EDTA. Ainsi, selon une autre de leurs caractéristiques, les aptamères nucléiques décrits ici consistent en des aptamères nucléiques permettant la formation de complexes avec des protéines cibles à domaine GLA, lesdits complexes pouvant être dissociés, avec libération des protéines cibles à domaine GLA, par mise en contact desdits complexes avec un milieu comprenant un chélateur d'ions divalents, par exemple de l'EDTA.

Comme déjà précisé précédemment, un complexe entre un aptamère nucléique décrit ici et une protéine cible à domaine GLA peut être dissocié en mettant en contact ledit complexe avec un milieu, y compris une solution tampon, comprenant une concentration finale d'EDTA d'au moins 1 mM et d'au plus 100 mM.

Comme cela est illustré dans les exemples, on tire parti de l'aptitude des aptamères anti-GLA décrits ici à se lier à une diversité de protéines de la coagulation à domaine GLA, pour purifier simultanément une pluralité de protéines de la coagulation à domaine GLA susceptibles d'être contenues dans l'échantillon de départ, en utilisant un unique support de chromatographie d'affinité. Par exemple, on peut utiliser le procédé de purification d'une protéine de la coagulation à domaine GLA ci-dessus pour la purification simultanée de plusieurs
facteurs de la coagulation à domaine GLA contenus dans l'échantillon de départ, par exemple pour la purification simultanée des facteurs VII, IX et X contenus dans une fraction de cryoprécipité de plasma sanguin humain.

Il est aussi décrit ici une composition purifiée d'une protéine de la coagulation à domaine GLA humaine recombinante comprenant au moins 99,9% en poids de
ladite protéine de la coagulation à domaine GLA humaine recombinante et qui est sensiblement exempte de protéines non humaines.

Il est aussi décrit une composition purifiée d'une protéine de la coagulation à domaine GLA humaine recombinante comprenant au moins 99,9% en poids de
ladite protéine de la coagulation à domaine GLA humaine recombinante et au plus 0,1 % en poids de protéines non humaines, les pourcentages en poids étant exprimés par rapport au poids total en protéines de ladite composition purifiée.

Dans la composition purifiée ci-dessus, « au moins 99,9% » englobe au moins 99,91%, 99,92%, 99,93%, 99,94%, 99,95%, 99,96%, 99,97%, 99,98% et 99,99%.

Dans la composition purifiée ci-dessus, « au plus 0,1% » englobe au plus 0,09%, 0,08%, 0,07%, 0,06%, 0,05%, 0,04%, 0,03%, 0,02% et 0,01%.

Il est aussi décrit ici une composition purifiée telle que définie ci- dessus, utilisable comme médicament.

La description divulgue encore une composition pharmaceutique comprenant une composition purifiée d'une protéine de la coagulation à domaine GLA humaine recombinante telle que définie ci-dessus, en combinaison avec un ou plusieurs excipients pharmaceutiquement acceptables.

Il est décrit aussi une composition purifiée telle que définie ci-dessus pour le traitement des troubles de la coagulation.

La description divulgue également l'utilisation d'une composition purifiée telle que définie ci-dessus pour la fabrication d'un médicament pour le traitement des troubles de la coagulation.

### Procédés d'obtention d'aptamères anti-GLA

De manière générale, un aptamère anti-GLA décrit ici peut être obtenu selon un procédé basé sur les principes généraux de la technique appelée SELEX (Systematic Evolution of Ligands by Exponential Enrichment, qui a été initialement décrite notamment dans la demande PCT N°WO 1991/019813). Le procédé SELEX de sélection des aptamères consiste à mettre en présence une protéine avec une librairie combinatoire d'acides nucléiques, ADN ou ARN (en général 10¹⁵ molécules), les acides nucléiques ne se liant pas à la cible sont éliminés, les acides nucléiques se liant à la cible sont isolés et amplifiés par PCR. Le procédé est répété jusqu'à ce que la solution soit suffisamment enrichie avec les acides nucléiques ayant une bonne affinité pour la protéine d'intérêt (Tuerk et Gold, « Systematic evolution of ligands by exponential enrichment : RNA ligands to bacteriophage T4 DNA polymerase » (1990) Science, 249(4968) :505-10 et Ellington et Szostak, « In vitro selection of RNA molecules that bind specific ligands », (1990) Nature Aug 30;346(6287):818-22). D'autres exemples de procédé SELEX sont donnés dans les documents EP 0 786 469, EP 668 931, EP 1 695 978, EP 1 493 825 dont les enseignements peuvent être repris dans la réalisation du procédé de sélection d'un aptamère nucléique utilisé selon l'invention. Certaines variantes du procédé SELEX comprennent des étapes de contre-sélection d'aptamères précédemment sélectionnés par liaison à la protéine cible d'intérêt. La ou les étapes de contre-sélection peuvent consister en une étape dans laquelle une collection d'aptamères, qui a été précédemment sélectionnée avec la protéine cible d'intérêt, est mise en contact avec des molécules non-cibles, de manière à éliminer de la collection d'aptamères de départ ceux qui se lient aux molécules non-cibles. La mise en oeuvre d'une telle étape de contre-sélection, dans un procédé d'obtention d'aptamères nucléiques, est susceptible d'accroître la spécificité ou l'affinité des aptamères sélectionnés à la fin du procédé.

Un premier procédé pour l'obtention d'un aptamère anti-GLA peut consister en un procédé comprenant les étapes suivantes :
a) fournir un mélange d'acides nucléiques, aussi appelé « collection » d'acides nucléiques de séquences distinctes,
b) mettre en contact le mélange d'acides nucléiques fourni à l'étape a), ou le mélange d'acides nucléiques obtenu à la fin de l'étape d) lorsque l'étape b) est répétée, avec une première protéine cible de la coagulation à domaine GLA, dans des conditions permettant la formation de complexes entre des acides nucléiques dudit mélange avec ladite première protéine cible de la coagulation à domaine GLA,
c) réaliser une séparation entre (i) le ou les acides nucléiques ayant formé des complexes avec ladite première protéine cible de la coagulation à domaine GLA et (ii) les acides nucléiques n'ayant pas formé de complexes avec ladite première protéine cible de la coagulation à domaine GLA,
d) amplifier les acides nucléiques ayant formé des complexes avec ladite première protéine cible de la coagulation à domaine GLA, de manière à obtenir un mélange ou une collection d'acides nucléiques se liant à ladite première protéine cible de la coagulation à domaine GLA,
e) réitérer les étapes b) à d) un nombre de fois suffisant pour l'obtention d'une collection d'acides nucléiques ayant la capacité de liaison désirée à ladite première protéine cible de la coagulation à domaine GLA,
f) mettre en contact le mélange d'acides nucléiques fourni à l'étape e), ou le mélange d'acides nucléiques obtenu à la fin de l'étape h) lorsque l'étape f) est répétées, avec une seconde protéine cible de la coagulation à domaine GLA, dans des conditions permettant la formation de complexes entre des acides nucléiques dudit mélange avec ladite seconde protéine cible de la coagulation à domaine GLA,
g) réaliser une séparation entre (i) le ou les acides nucléiques ayant formé des complexes avec ladite seconde protéine cible de la coagulation à domaine GLA et (ii) les acides nucléiques n'ayant pas formé de complexes avec ladite seconde protéine cible de la coagulation à domaine GLA,
h) amplifier les acides nucléiques ayant formé des complexes avec ladite seconde protéine cible de la coagulation à domaine GLA, de manière à obtenir un mélange ou une collection d'acides nucléiques se liant à ladite seconde protéine cible de la coagulation à domaine GLA,
i) réitérer les étapes f) à h) un nombre de fois suffisant pour l'obtention d'une collection d'acides nucléiques ayant la capacité de liaison désirée à ladite seconde protéine cible de la coagulation à domaine GLA, lesdits acides nucléiques ayant la capacité à se lier à la fois à ladite première protéine cible de la coagulation à domaine GLA et à la seconde protéine cible de la coagulation à domaine GLA,
j) facultativement, réitérer les étapes f) à i) avec une ou plusieurs autres protéines cibles de la coagulation à domaine GLA distinctes, de manière à obtenir, à la fin du procédé, un mélange ou une collection d'acides nucléiques dits « aptamères anti-GLA » ayant la capacité à se lier aux protéines de la coagulation à domaine GLA.

De manière générale, les protocoles détaillés de mise en oeuvre des étapes du procédé ci-dessus peuvent être retrouvés par l'homme du métier dans les nombreuses publications relatives à la technique SELEX, y compris dans les références citées précédemment dans la présente description.

Dans certains modes de réalisation du procédé d'obtention d'aptamères anti-GLA ci-dessus, on utilise successivement de 2 à 6 protéines cibles de la coagulation à domaine GLA pour sélectionner des aptamères anti-GLA, c'est-à-dire d'aptamères nucléiques spécifiques des protéines de la coagulation à domaine GLA, mais non-spécifiques d'une protéine de la coagulation à domaine GLA donnée. Dans la pratique, on a montré que l'utilisation, dans le procédé ci-dessus, successivement de deux protéines cibles de la coagulation à domaine GLA distinctes permettait l'obtention d'aptamères anti-GLA.

Pour la mise en oeuvre du procédé d'obtention d'aptamères anti-GLA ci-dessus, les protéines cibles de la coagulation à domaine GLA peuvent être choisies dans le groupe constitué du Facteur II, du Facteur VII, du Facteur IX, du Facteur X, de la protéine C et de la protéine S. L'ordre d'utilisation des protéines cibles n'est pas une caractéristique essentielle du procédé ci-dessus.

Selon une alternative, les aptamères nucléiques anti-GLA peuvent être obtenus selon un second procédé comprenant les étapes suivantes :
a) fournir un mélange d'acides nucléiques, aussi appelé « collection » d'acides nucléiques de séquences distinctes,
b) mettre en contact le mélange d'acides nucléiques fourni à l'étape a), ou le mélange d'acides nucléiques obtenu à la fin de l'étape d) lorsque l'étape b) est répétée, avec le domaine GLA d'une première protéine cible de la coagulation à domaine GLA, dans des conditions permettant la formation de complexes entre des acides nucléiques dudit mélange avec le domaine GLA de ladite première protéine cible de la coagulation à domaine GLA,
c) réaliser une séparation entre (i) le ou les acides nucléiques ayant formé des complexes avec le domaine GLA de ladite première protéine cible de la coagulation à domaine GLA et (ii) les acides nucléiques n'ayant pas formé de complexes avec le domaine GLA de ladite première protéine cible de la coagulation à domaine GLA,
d) amplifier les acides nucléiques ayant formé des complexes avec le domaine GLA de ladite première protéine cible de la coagulation à domaine GLA, de manière à obtenir un mélange ou une collection d'acides nucléiques se liant au domaine GLA de ladite première protéine cible de la coagulation à domaine GLA,
e) réitérer les étapes b) à d) un nombre de fois suffisant pour l'obtention d'une collection d'acides nucléiques ayant la capacité de liaison désirée au domaine GLA de ladite première protéine cible de la coagulation à domaine GLA,
f) mettre en contact le mélange d'acides nucléiques fourni à l'étape e), ou le mélange d'acides nucléiques obtenu à la fin de l'étape h) lorsque l'étape f) est répétée, avec le domaine GLA d'une seconde protéine cible de la coagulation à domaine GLA, dans des conditions permettant la formation de complexes entre des acides nucléiques dudit mélange avec le domaine GLA de ladite seconde protéine cible de la coagulation à domaine GLA,
g) réaliser une séparation entre (i) le ou les acides nucléiques ayant formé des complexes avec le domaine GLA de ladite seconde protéine cible de la coagulation à domaine GLA et (ii) les acides nucléiques n'ayant pas formé de complexes avec le domaine GLA de ladite seconde protéine cible de la coagulation à domaine GLA,
h) amplifier les acides nucléiques ayant formé des complexes avec le domaine GLA de ladite seconde protéine cible de la coagulation à domaine GLA, de manière à obtenir un mélange ou une collection d'acides nucléiques se liant au domaine GLA de ladite seconde protéine cible de la coagulation à domaine GLA,
i) réitérer les étapes f) à h) un nombre de fois suffisant pour l'obtention d'une collection d'acides nucléiques ayant la capacité de liaison désirée au domaine GLA de ladite seconde protéine cible de la coagulation à domaine GLA, lesdits acides nucléiques ayant la capacité à se lier à la fois au domaine GLA de ladite première protéine cible de la coagulation à domaine GLA et au domaine GLA de la seconde protéine cible de la coagulation à domaine GLA,
j) facultativement, réitérer les étapes f) à i) avec le domaine GLA d'une ou plusieurs autres protéines cibles de la coagulation à domaine GLA distinctes, de manière à obtenir, à la fin du procédé, une mélange ou une collection d'acides nucléiques dits « aptamères anti-GLA » ayant la capacité à se lier aux protéines de la coagulation à domaine GLA.

Dans certains modes de réalisation du second procédé d'obtention d'aptamères anti-GLA ci-dessus, on utilise successivement les domaines GLA provenant de 2 à 6 protéines cibles de la coagulation à domaine GLA pour sélectionner des aptamères anti-GLA..

Pour la mise en oeuvre du second procédé d'obtention d'aptamères anti-GLA ci-dessus, les protéines cibles de la coagulation à domaine GLA dont proviennent les domaines GLA utilisés peuvent être choisies dans le groupe constitué du Facteur II, du Facteur VII, du Facteur IX, du Facteur X, de la protéine C et de la protéine S. L'ordre d'utilisation des domaines GLA cibles n'est pas une caractéristique essentielle du procédé ci-dessus.

La différence essentielle entre le second procédé d'obtention d'aptamères anti-GLA ci-dessus et le premier procédé d'obtention d'aptamères anti-GLA précédemment décrit réside dans la cible, laquelle consiste en une succession de protéines cibles de la coagulation à domaine GLA dans le premier procédé et consiste en une succession de domaines GLA de protéines de la coagulation à domaine GLA dans le second procédé.

Dans la présente description, des aptamères anti-GLA peuvent être obtenus selon un troisième procédé, dont les principes sont identiques au premier et au second procédés décrits ci-dessus, mais dans lequel les substances cibles sont alternativement des protéines de la coagulation à domaine GLA et des domaines GLA de protéines de la coagulation à domaine GLA. L'ordre d'utilisation des protéines de la coagulation à domaine GLA et des domaines GLA de protéines de la coagulation à domaine GLA est aisément déterminé par l'homme du métier, selon en particulier les substances cibles auxquelles cet homme du métier à accès.

Les domaines GLA cibles qui sont utilisés dans chacun des second et troisième procédés ci-dessus peuvent être aisément obtenus par l'homme du métier, en particulier dans les situations dans lesquelles la séquence en acides aminés de la protéine de la coagulation à domaine GLA parente, et donc aussi la séquence en acides aminés du domaine GLA considéré, sont connues.

Selon certains modes de réalisation, un domaine GLA est obtenu à partir de la protéine de la coagulation à domaine GLA parente, par protéolyse de ladite protéine de la coagulation à domaine GLA, en utilisant une ou plusieurs enzymes protéolytiques adaptées, de spécificité de coupure connue.

Selon d'autres modes de réalisation, un domaine GLA est obtenu par des techniques de synthèse peptidique connues en elles-mêmes, sur la base de leur séquence connue en acides aminés, puis par gamma-carboxylation des résidus de glutamine du domaine GLA, à l'aide d'une carboxylase, selon des techniques bien connues de l'homme du métier. A titre illustratif, un peptide comprenant le domaine GLA d'une protéine de la coagulation à domaine GLA peut être synthétisé avec un appareil synthétiseur de peptides du type Milligen® 9050 Plus (Perkin Elmer, Stockholm, Suède, par exemple en utilisant des acides aminés DPfp Fmoc commercialisés par la Société PerSeptive Biosystems® (Framingham, Massachusets, Etats-Unis). Pour l'étape de gamma-carboxylation enzymatique du domaine GLA néo-synthétisé, on peut par exemple utiliser une carboxylase semi-purifiée comme décrit par Soute et al. (1987, Thromb Haemostasis, Vol. 57 : 77-81), selon la technique décrite par Wu et al. (1990, J Biol Chem, Vol. 265(22) : 13124-13129).

Selon encore d'autres modes de réalisation, un procédé d'obtention d'un aptamère anti-GLA peut être du type de l'un quelconque des procédés décrits ci-dessus, auquel peut être ajoutée une étape de sélection de ceux des aptamères dont la capacité à se lier à la protéine cible n'est pas altérée par un environnement de haute force ionique, par exemple une solution tampon comprenant du NaCl à une concentration finale d'au moins 0,5 M, ce qui inclut au moins 1 M, 1,5 M, 2 M, 2,5 M et 3 M.

Selon encore d'autres modes de réalisation, un procédé d'obtention d'un aptamère anti-GLA peut être du type de l'un quelconque des procédés décrits ci-dessus, auquel peut être ajoutée une étape de sélection de ceux des aptamères permettant la formation de complexes avec des protéines cibles à domaine GLA, lesdits complexes pouvant être dissociés, avec libération des protéines cibles à domaine GLA, par mise en contact desdits complexes avec un milieu comprenant un chélateur d'ions divalents, par exemple de l'EDTA. L'étape de sélection de ces aptamères peut être réalisée avec un milieu, y compris une solution tampon, comprenant une concentration finale d'EDTA d'au moins 1 mM et d'au plus 100 mM.

Selon encore d'autres modes de réalisation, un procédé d'obtention d'un aptamère anti-GLA peut être du type de l'un quelconque des procédés décrits ci-dessus, auquel peut être ajoutée une étape de sélection de ceux des aptamères dont la capacité à se lier à la protéine cible n'est pas altérée par la présence d'un alkylène glycol ou d'un polyalkylène glycol.

Selon encore d'autres modes de réalisation, un procédé d'obtention d'un aptamère anti-GLA peut être du type de l'un quelconque des procédés décrits ci-dessus, auquel peut être ajoutée une étape de sélection de ceux des aptamères dont la capacité à se lier à la protéine cible n'est pas altérée par la présence d'éthylène glycol. L'étape de sélection de ces aptamères peut être réalisée avec un milieu, y compris une solution tampon, comprenant une concentration finale d'éthylène glycol d'au moins 0,5 M, ce qui inclut au moins 1 M, et 1,5 M.

Selon encore d'autres modes de réalisation, un procédé d'obtention d'un aptamère anti-GLA peut être du type de l'un quelconque des procédés décrits ci-dessus, auquel peut être ajoutée une étape de sélection de ceux des aptamères dont la capacité à se lier à la protéine cible n'est pas altérée par la présence de propylène glycol. L'étape de sélection de ces aptamères peut être réalisée avec un milieu, y compris une solution tampon, comprenant une concentration finale de propylène glycol d'au moins 10% (v/v), ce qui inclut au moins 15%, 20%, 25%, 30%, 35%, 40%, 45% et 50%..

Selon encore d'autres modes de réalisation, un procédé d'obtention d'un aptamère anti-GLA peut être du type de l'un quelconque des procédés décrits ci-dessus, auquel peut être ajoutée une étape de sélection de ceux des aptamères dont la capacité à se lier à la protéine cible n'est pas altérée par la présence d'éthanol. L'étape de sélection de ces aptamères peut être réalisée avec un milieu, y compris une solution tampon, comprenant une concentration finale en éthanol d'au moins 1% (v/v), ce qui inclut au moins 1,5%, 2,0%, 2,5%, 3,0%, 3,5%, 4,0%, 4,5%, 5,0%, 5,5%, 6,0%, 6,5%, 7,0%, 7,5%, 8,0%, 9,0%, 9,5% et 10,0%.

**Tableau des séquences**

| **SEQ ID N°** | **Type** | **Désignation** |
|---|---|---|
| 1 | Acide nucléique | Région 5' d'aptamère |
| 2 | | Région 3' d'aptamère |
| 3 | | Core sequence de Mapt-1 |
| 4 | | Aptamère Mapt-1 |
| 5 | | Aptamère non apparenté à Mapt-1 |
| 6 à 34 | | Aptamères (« Core sequences ») |
| 35 | | Aptamère Mapt-1.2.-CS |
| 36 | | Aptamère Mapt-1.2.-CSO |
| 37 | | Aptamère Mapt-2-CS |
| 38 | | Aptamère Mapt-2.2.-CS |
| 39 | | Aptamère Mapt-2 |

La présente invention est en outre illustrée par les exemples suivants.

### EXEMPLES

### Exemple 1 : préparation d'un support d'affinité

Le support d'affinité a été réalisé à partir d'un matériau de support solide consistant en une matrice sur laquelle on a greffé de la streptavidine (streptavidin-agarose - Novagen®).

On a introduit un volume de 1 ml de gel dans une conteneur consistant en une colonne (i.d. 11mm). On a lavé le gel avec de l'eau purifiée, pour éliminer le solvant de stockage

### Les caractéristiques du gel sont :

- *Capacité d'adsorption de biotine :* ≥ 85 nanomole / ml de gel
- *Test fonctionnel:* Capture >99% de thrombine biotinylée en 30 minutes à TA
- *Autres tests* : Protease-free , endo/exonucléase-free, RNase-free.
- *Conservateur:* 100 mM sodium phosphate pH7,5 + NaN3 0,02

La sortie de la colonne conditionnée (« packée ») (Hauteur de lit de gel = 1cm) est connectée à un détecteur d'absorbance UV à 254 nm et d'un enregistreur.

Les aptamères nucléiques anti-GLA biotinylés de séquence SEQ ID N°4, aussi appelés aptamères « Mapt-1 » dans la présente description, sont solubilisés dans de l'eau purifiée à la concentration finale de 0,5 mg/0,187 ml, soit une concentration molaire finale de 0,1 mM. La solution d'aptamères nucléiques a été activée à 95°C selon le cycle standard, pour l'immobilisation des aptamères sur le matériau support solide.

La solution d'aptamères nucléiques a été préalablement diluée par 4,8 ml d'eau purifiée puis 1,5 ml de tampon concentré de façon à obtenir la formulation suivante : 50mM Tris, 50mM NaCl, MgCl₂ 4mM, CaCl₂ 10mM pH= 7,5

Le détecteur d'absorbance est ajusté à 1 AUFS (Absorbance Unit Full Scale) et on enregistre la DO à 254 nm de cette solution à 0,575 UA₂₅₄.

La solution d'aptamères nucléiques biotinylés est injectée sur le gel streptavidin-agarose pré-conditionnée (« prépackée ») et mise en recirculation avec une pompe péristaltique à un débit de 2,5 ml/minute, soit un temps de contact sur le gel de 24 secondes (entrée/sortie E/S). Dans ces conditions, la DO à 254 nm se stabilise rapidement à 0,05 UA₂₅₄, soit une valeur de 91% de couplage théorique, c'est-à-dire 0,455 mg d'aptamères nucléiques par millilitre de gel.

Un lavage avec un tampon NaCl 2M est réalisé, afin d'éliminer les aptamères nucléiques qui ne sont pas fixées spécifiquement aux molécules de streptavidine greffées sur le matériau de support solide.

### Exemple 2 : Purification d'une protéine de la coagulation à domaine GLA plasmatique (Facteur IX humain plasmatique)

On a utilisé un support d'affinité du type décrit à l'exemple 1 pour purifier du Facteur IX humain plasmatique.

### A. Procédé de chromatographie

### A. 1. - Caractéristiques de la matière première (MP)

Facteur IX :C : 204 IU/ml
Protéines totales : 0,5 g/l

### A. 2. - Préparation de la matière de départ (MD)

- 0,920ml MP dilué dans 5ml de tampon d'équilibration /
- Facteur IX :C : 37,5 IU/ml
- Protéines totales : 0,092 g/l
- pH : 7,43
- Osmolalité : H2O 231 mOsmol/Kg

### A.3. - Chargement

- 1UI de FIX = 4,4 µg.
- Volume injecté - 2 g
- Chargement FIX en UI par ml de gel : 75 UI/ml de gel
- Chargement FIX en µg par ml de gel : 300 µg/ml de gel

### A.4. - Caractéristiques des tampons de chromatographie

- Tampon d'Equilibration : 50mM Tris, 50mM NaCl, 10mM CaCl2, 4mM MgCl2, pH 7,5, 218 mOsmol/kg
- Tampon Elution 1 : 20mM Tris, 10mMEDTA, pH 7,5, 47 mOsmol/kg
- Tampon Elution 2 (Régénération) : 20mM Tris, 1M NaCl, 50% Propylène glycol , pH 7,5

### A.5. - Suivi des étapes de chromatographie

| Tampons | Fractions | Débit MI/min | Poids g | Pic AU | pH | Osmolalité mOsmol/Kg |
|---|---|---|---|---|---|---|
| Equilibration | MD | 0,1 | 2 | NA | 7,43 | 231 |
| Equilibration | NR | 0,1 | 6,04 | 0,01 | 7,40 | 222 |
| Elution 1 | E1 | 0,5 | 4,17 | 0,02 | 6,02 | 120 |
| Elution 2 | E2 | 0,5 | 3,61 | 0,01 | 7,38 | NA |

### B. Caractérisation des produits purifiés

### B.1. Electrophorèse SDS-PAGE

Les résultats d'un essai d'électrophorèse sur gel SDS-PAGE sont illustrés sur la Figure 1.

Sur la ligne 3, on observe que la fraction d'élution (E1) contient une seule bande de Facteur IX humain plasmatique purifié.

### B. 2 - Bilan en protéines totales, en FIX :C, FIX :Ag et FIX :Am

Les dosages des protéines totales ont été réalisés par le laboratoire Tébu Bio. Les déterminations du taux de FIX :C ont été réalisées par le LBA et ceux de FIX :Ag et FIX :Am par le LIB.

| | Coag | | | Protéine | | | Purification | | |
|---|---|---|---|---|---|---|---|---|---|
| Etapes | [FIX :C] (UI ml) | Q FIX :C (UI) | R FIX:C (%) | [Protéine] (g/l) | Q Protéine (g) | R Protéine (%) | AS (UI/mg) | Pureté | Gain de pureté |
| MD | 26,8 | 53,6 | 100,0 | 0,125 | 0,3 | 100,0 | 214 | 94 | / |
| NR | 1,6 | 9,7 | 18,0 | 0,024 | 0,1 | 58,0 | 67 | 29 | 0,3 |
| E1 | 5,6 | 23,4 | 43,6 | 0,019 | 0,1 | 31,7 | 295 | 130 | 1,4 |

| | Am | | | Ag | | | Ratio | | |
|---|---|---|---|---|---|---|---|---|---|
| Etapes | [ FIX :Am] (UI / ml) | Q FIX :Am (UI) | R FIX:Am (%) | [FIX :Ag] (UI / ml) | Q FIX :Ag (UI) | R FIX:Ag (%) | [C]/[Ag] | [Am]/[Ag] | [C]/[ Am] |
| MD | 32,3 | 64,6 | 100 | 24,0 | 48 | 100 | 1,1 | 1,3 | 0,8 |
| NR | 1,1 | 6,5 | 10 | 1,6 | 10 | 20 | 1,0 | 0,7 | 1,5 |
| E1 | 8,3 | 34,8 | 54 | 4,8 | 20 | 42 | 1,2 | 1,7 | 0,7 |

### B.3 - Conclusions

Cet essai de chromatographie d'affinité sur Aptamère Mapt-1 (SEQ ID N°4) a été réalisé pour vérifier l'intégrité du support d'affinité et la capacité du ligand à fixer le facteur IX.

Il s'agit du second essai réalisé dans les mêmes conditions chromatographiques à partir d'un échantillon de facteur IX pré-purifié sur Superdex 75.

Le ratio du taux de facteur IX :C / Ag de l'éluat est de l'ordre de 1, les conditions opératoires appliquées lors de la chromatographie n'ont pas occasionné de dégradation de la molécule de FIX.

Une quantité de 20% du facteur IX est récupéré dans la fraction non adsorbée du gel ce qui indique probablement une saturation du gel et l'utilité de réduire la quantité d'échantillon de départ pour le chargement du support d'affinité.

L'élution avec 20mM de Tris et 10mM EDTA donne un rendement de 44% de FIX avec un gain de pureté de l'ordre de 1,4 par rapport à la matière de départ.

Dans d'autres essais, on a obtenu un rendement de 71% (résultats non présentés).

### Exemple 3 : Purification d'une protéine de la coagulation à domaine GLA recombinante (Facteur IX humain transgénique)

On a utilisé un support d'affinité du type décrit à l'exemple 1 pour purifier du Facteur IX humain recombinant produit dans le lait de porcs transgéniques pour le Facteur IX humain.

### A. Protocole de purification par chromatographie d'affinité

Les caractéristiques de mise en oeuvre de l'étape de chromatographie d'affinité de l'échantillon de départ sur un support d'affinité sur lequel sont immobilisés les aptamères anti-GLA Mapt-1 sont décrites dans les tableaux ci-dessous.

### Etape 1 : Clarification

Clarification au citrate pour d'obtenir du lait clarifié à pH 7,5 à 0,25M final de tampon citrate

### Etape 2 : MEP hypercel

MD : lait clarifé (IBF 25 - 10ml de gel)
Chargement en FIX : 243 UI/ml de gel
Tampon équilibration : 0,25M Citrate pH7,5
Tampon élution : eau

### Etape 3 : Dialyse

MD : Eluat MEP
Tampon de dialyse : 50 mM Tris - 50 mM NaCl pH 7,5
Etape 4 : MAPT-1 (3 run)
MD : Eluat MEPdialysé (IBF1,1 - 1ml de gel)
Chargement en FIX : 230 UI/ml de gel
Tampon équilibration : 50 mM tris - 50 mM NaCl - 4 mM MgCl₂ - 10 mM CaCl₂ pH 7,5
Tampon élution : 20 mM Tris - 10 mM EDTA pH 7,5
Tampon régénération : 20 mM tris - 1 M NaCl - 5% PG pH 7,5

### B. Caractéristique de la matière première (MP)

La matière première mise en oeuvre consiste en un lait brut de cochon transgénique pour le Facteur IX humain.

### C. Suivi du process

### C.1. Etape 1 : Clarification

### C.1.1. - Suivi de la clarification

- Décongélation de E0 à 37°C
- Mélange d'un aspect très laiteux : 45g (2/3) Lait de porc + 25g (1/3) de tampon citrate concentré à 0,75M
- Agitation douce de 30 min à température ambiante
- Centrifugation 30 min, 15°C, 5 000g
- Culot : petit et blanc contenant les poils de porc et les impuretés
- Surnageant : deux phases, la phase supérieure solide crémeuse et blanche constituant les corps gras
   et la phase jaunâtre qui représente le lait clarifié(E1).
- Récupération à la pompe du lait clarifié
- Filtration profondeur du lait clarifié avec un filtre Cuno BioCap 25 Filter
- Congélation à -80°C du lait filtré clarifié (E2)

### C.1.2 - Bilan en protéines totales, en FIX :C, FIX :Ag et FIX :Am

Les résultats sont présentés dans les tableaux 4 et 5 ci-dessous.

**Tableau 4**

| | | | Coagulation | | | Protéines totales | | | Purification | | |
|---|---|---|---|---|---|---|---|---|---|---|---|
| Etape | Code | Poids (g) | [ FIX :C] (UI/ml) | Q FIX :C (UI) | R FIX:C (%) | [Prot] (g/l) | Q Prot. les (g) | R Prot. (%) | AS (UI/mg) | Pureté | Gain de pureté |
| Lait brut | E0 | 45,0 | 57,2 | 2574 | 100,0 | 103,2 | 644 | 100,0 | 0,6 | 0,2 | / |
| Lait clarifié avant filtration | E1 | 65,3 | 36,0 | 2350 | 91,3 | 37,5 | 2451 | 52,8 | 1,0 | 0,4 | 1,7 |
| Lait clarifié | E2 | 63,8 | 38,2 | 2438 | 94,7 | 34,7 | 2214 | 47,7 | 1,1 | 0,5 | 2,0 |

**Tableau 5**

| | Amidolytique | | | Antigénique | | | Ratio | | |
|---|---|---|---|---|---|---|---|---|---|
| Code | [FIX :Am] (UI / ml) | Q FIX :Am (UI) | R FIX:Am (%) | [FIX :Ag] (UI / ml) | Q FIX :Ag (UI) | R FIX:Ag (%) | [C]/[Ag] | [Am]/[Ag] | |
| E0 | 40 | 1 798 | 100 | 147 | 6 615,0 | 100,0 | 0,4 | 0,3 | |
| E1 | 23 | 1 510 | 84 | 114 7 | 443,1 | 112,5 | 0,3 | 0,2 | |
| E2 | 22 | 1 426 | 79 | 95 | 6 062,0 | 91,6 | 0,4 | 0,2 | |

### C.2. Etape 2 : MEP hypercel

### C.2.1. - Caractéristiques du support de chromatographie

- Colonne : IBF 25, 2,5 cm de hauteur
- Gel : MEP 10ml de gel, lot 200920/G206-04
- Nb d'utilisation : 2ème utilisation
- Régénération du gel avant l'utilisation : 1VC NaOH 1M ; 2VC NaCl 2M ; 4VC eau

### C.3.2 - Préparation de la matière de départ (MD)

- Décongélation de E2 à 37°C
- Référence : 1UI de FIX = 4µg/ml
- Volume injecté (g) : 63,81
- Chargement FIX en UI par ml de gel (UI/ml gel) : 243 UI/ml de gel
- Chargement FIX en µg par ml de gel (µg/ml gel) : 972 µg/ml de gel

### C.2.3 - Caractéristiques des tampons de chromatographie

- Tampon d'équilibration : 0,25M Citrate, pH 7,5, 612 mOsmol/kg, 31,9 mS/cm
   Tampon d'élution MEP : eau

### C.2.4 - Suivi des étapes de chromatographie

**Tableau 6**

| Code | Etape | Débit ml/min | Poids g | Pic AU | pH | Osmolarité mosmol/Kg | Conducti. mS/cm | Observations |
|---|---|---|---|---|---|---|---|---|
| E2 | MD | 0,85 | 63,81 | NA | 7,43 | 231 | 31,9 | NA |
| E3 | NR | 0,85 | 139,61 | 2 | 7,45 | 719 | 32 | Saturation |
| E4 | E MEP | 1 | 51,68 | 2 | 7,81 | 121 | 7,11 | Saturation |

Régénération du gel avant l'utilisation : 10VC NaOH 1M ; 4VC NaCl 2M ; 10VC eau ; 10VC éthanol 20%.

### C.2.5. Bilan en protéines totales

**Tableau 7**

| | | | Coagulation | | | Protéines totales | | | Purification | | |
|---|---|---|---|---|---|---|---|---|---|---|---|
| Etapes | Code | Poids (g) | [FIX :C] (UI/ml) | Q FIX:C (%) | R FIX:C (%) | [Prot] (g/l) | Q Prot. (g) | R Prot.(%) | AS (UI/mg) | Pureté | Gain de pureté |
| Lait clarifié | E2 | 63,8 | 38,2 | 2 438 | 100,0 | 34,7 | 2 214,2 | 100,0 | 1,1 | 0,5 | / |
| Non adsorbé | E3 | 139,6 | 3,1 | 433 | 17,8 | 8,4 | 1 175,4 | 53,1 | 0,4 | 0,2 | 0,3 |
| Elution MEP | E4 | 51,7 | 20,3 | 1 049 | 43,0 | 3,6 | 186,6 | 8,4 | 5,6 | 2,5 | 5,1 |
| Eluat MEP dialysé | E5 | 51,1 | 14,5 | 741 | 30,4 | 3,3 | 169,1 | 7,6 | 4,4 | 1,9 | 4,0 |

**Tableau 8**

| | Amidolytique | | | Antigénique | | | Ratio | | |
|---|---|---|---|---|---|---|---|---|---|
| Code | [FIX :Am] (UI / ml) | Q FIX :Am (UI) | R FIX:Am (%) | [FIX :Ag] (UI / ml) | Q FIX :Ag (UI) | R FIX:Ag (%) | [C]/[Ag] | [Am]/[Ag] | |
| E2 | 22 | 1 426 | 100 | 95 | 6 062,0 | 100,0 | 0,4 | 0,2 | |
| E3 | 1 | 148 | 10 | ND | ND | ND | ND | ND | |
| E4 | 9 | 484 | 34 | 47 | 2 429,0 | 40,1 | 0,4 | 0,2 | |
| E5 | 9 | 455 | 32 | 45 | 2 298,6 | 37,9 | 0,3 | 0,2 | 1,6 |

### C.3. Etape 3 : Dialyse

Dialyse d'E4 (50 g) dans 2 bains de 1 L, toute la nuit sous agitation à 4°C

**Tableau 9**

| Echantillons | Fractions | Poids g | pH | Osmolarité mosmol/Kg | Conductivité mS/cm |
|---|---|---|---|---|---|
| E4 | Avant dialyse | 49,18 | 7,81 | 121 | 7,11 |
| E5 | Après dialyse | 51,08 | 7,81 | 149 | 7,18 |
| Tampon dialyse | | | 7,50 | 157 | 7,52 |
| 50 mM Tris - 50 mM NaCl | | | | | |

### C.4. : Chromatographie sur support d'affinité avec aptamères anti-GLA (Mapt-1)

Trois essais indépendants de purification de Facteur IX humain transgénique pré-purifié comme décrit ci-dessus ont été réalisés. Les conditions opératoires et les résultats de chacun des essais sont détaillés ci-après.

### C.4.1 - Caractéristiques du support de chromatographie

- Colonne : IBF 1,1 , 0,9 cm de hauteur
- Gel : MAPT-1, 1ml de gel
- Nb d'utilisation 2ème utilisation

### C.4.2 - Préparation de la matière de départ (MD)

- Ajustement en pH et ajout de 4mM MgCl₂ et 10mMCaCl2 final dans E5
- Répartition de E5 (48,17g) répartie en 3*16g dont deux sont mis à -80°C pour les essais suivants
- Référence : 1UI de FIX = 4µg/ml
- Volume injecté (g) : 16,14
- Chargement FIX en UI par ml de gel (UI/ml gel) : 234 UI/ml de gel
- Chargement FIX en µg par ml de gel (µg/ml gel) : 936 µg/ml de gel

### C.4.3. Caractéristiques des tampons de chromatographie

- Tampon d'équilibration : 50mM Tris, 50mM NaCl, 10mM CaCl2, 4mM MgCl2, pH 7,51, 218 mOsmol/kg, 10,77 mS/cm
- Tampon d'élution 1 : 20mM Tris, 10mMEDTA, pH 7,53, 56 mOsmol/kg, 2,64 mS/Cm
- Tampon d'élution 2 (tampon de régénération) : 20mM Tris, 1M NaCl, 50% Propylène glycol, pH 7,52, 18,27 mS/cm.

### C.4.4. Suivi des étapes de chromatographie

### Essai 1

- Colonne équilibrée à pH 7,34 et 211mOsm/Kg
- Chargement en FIX 234 UI/ml de gel,

**Tableau 10**

| Echantillons | Fractions | Débit ml/min | Poids g | Pic AU | pH | Osmolarité mosmol/Kg | Conducti. mS/cm | Observations |
|---|---|---|---|---|---|---|---|---|
| E5 | MD | 0,6 | 16,14 | Na | 7,51 | 181 | 8,18 | na |
| E6 | NR | 0,6 | 29,56 | 0,5 | 7,44 | 193 | 9,03 | Saturation |
| E7 | El MAPT-1 | 0,6 | 6,68 | 0,02 | 6,40 | 99 | na | Diminution de pH |
| E8 | E2 MAPT-1 | 0,6 | 6,66 | 0,1 | 7,41 | na | na | na |

### Essai 2

- Colonne équilibrée à pH 7,39 et 220mOsm/Kg, 3ème utilisation du gel
- Chargement en FIX 236 UI/ml de gel

**Tableau 11**

| Fractions 315 256 | Débit ml/min | Poids g | Pic AU | pH | Osmolarité mosmol/Kg | Observations |
|---|---|---|---|---|---|---|
| MD | 0,1 | 13,50 | na | 7,42 | 179 | Na |
| NR | 0,1 | 20,29 | 0,5 | 7,36 | 197 | Saturation |
| E1 MAPT-1 | 0,1 | 2,84 | 0,02 | 7,06 | 187 | Diminution de pH |
| E2 MAPT-1 | 0,1 | 3,77 | 0,1 | 6,39 | na | Diminution de pH |

### Essai 3

- Colonne équilibré à pH 7,45 et 219mOsm/Kg, 5ème utilisation du gel
- Chargement en FIX 210 UI/ml de gel

**Tableau 12**

| Fractions 315 258 | Débit ml/min | Poids g | Pic AU | pH | Osmolarité mosmol/Kg | Observations |
|---|---|---|---|---|---|---|
| MD | 0,6 | 12,71 | na | 7,47 | 180 | na |
| NR | 0,6 | 23,79 | 0,5 | 7,46 | 206 | Saturation |
| E1 MAPT-1 | 0,6 | 2,67 | 0,02 | 6,94 | 168 | Diminution de pH |
| E2 MAPT-1 | 0,6 | 4,20 | 0,1 | 7,36 | na | na |

### C.4.5. Bilan en protéines totales, en FIX :C, FIX :Ag et FIX :Am

Les dosages des protéines totales ont été réalisés par le laboratoire Tébu Bio. Les déterminations du taux de FIX :C ont été réalisées par le LBA et ceux de FIX :Ag et FIX :Am par le LIB.

### C.4.5.1 Résultats chromatographie d'affinité de l'Essai 1

**Tableau 13**

| | | | Coag | | | Protéine | | | Purification | | |
|---|---|---|---|---|---|---|---|---|---|---|---|
| Etape | Code | Poids (g) | [ FIX :C] (UI/ml) | Q FIX :C | R FIX:C (%) | [Prot. Totales] | Q Prot.totales | R Prot. totales (%) | AS (UI/mg) | Pureté | Gain de pureté |
| Injection | E5 | 16,1 | 14,5 | 234 | 100,0 | 3,310 | 53,4 | 100,0 | 4,4 | 1,9 | / |
| Non adsorbé | E6 | 29,6 | 8,9 | 263 | 112,4 | 1,360 | 40,2 | 75,3 | 6,5 | 2,9 | 1,5 |
| Elution 1 | E7 | 6,7 | 0,50 | 3,3 | 1,4 | 0,015 | 0,1 | 0,2 | 33,3 | 14,7 | 7,6 |

**Tableau 14**

| | Am | | | Ag | | | Ratio | | |
|---|---|---|---|---|---|---|---|---|---|
| Code | [FIX :Am] (UI / ml) | Q FIX :Am (UI) | R FIX:Am (%) | [FIX :Ag] (UI / ml) | Q FIX :Ag (UI) | R FIX:Ag (%) | [C]/[Ag] | [Am]/[Ag] | |
| E5 | 9 | 143,6 | 100,0 | 45 | 726,3 | 100,0 | 0,3 | 0,2 | |
| E6 | 2,7 | 78,6 | 54,7 | 26 | 768,6 | 105,8 | 0,3 | 0,1 | |
| E7 | 0,2 | 1,1 | 0,7 | 0,238 | 1,6 | 0,2 | 2,1 | 0,7 | |

### C.4.5.2. Résultats chromatographie d'affinité de l'Essai 2

**Tableau 15**

| | | | Coag | | | Protéine | | | Purification | | |
|---|---|---|---|---|---|---|---|---|---|---|---|
| Etape | Code | Poids (g) | [FIX :C] (UI / ml) | Q FIX :C | R FIX:C (%) | [Prot. Totales] | Q Prot.totales | R Prot. totales (%) | AS (UI/mg) | Pureté | Gain de pureté |
| Injection | MD | 13,5 | 17,5 | 236 | 100,0 | 3,110 | 42,0 | 100,0 | 5,6 | 2,5 | / |
| Non adsorbé | NR | 20,3 | 8,7 | 177 | 74,7 | 1,940 | 39,4 | 93,8 | 4,5 | 2,0 | 0,8 |
| Elution 1 | E1 | 2,8 | 0,32 | 0,91 | 0,4 | 0,007 | 0,0 | 0,0 | 45,7 | 20,1 | **8,1** |

**Tableau 16**

| | Am | | | Ag | | | Ratio | | |
|---|---|---|---|---|---|---|---|---|---|
| Code | [ FIX :Am] (UI / ml) | Q FIX :Am (UI) | R FIX:Am (%) | [FIX :Ag] (UI / ml) | Q FIX :Ag (UI) | R FIX:Ag (%) | [C]/[Ag] | [Am]/[Ag] | |
| MD | 9,4 | 127,4 | 100,0 | 45,0 | 607,5 | 100,0 | 0,4 | 0,2 | |
| NR | 5 | 100,4 | 78,8 | 25,0 | 507,3 | 83,5 | 0,3 | 0,2 | |
| E1 | 0,1 | 0,4 | 0,3 | 0,2 | 0,6 | 0,1 | **1,6** | **0,7** | |

### C.4.5.3. Résultats chromatographie d'affinité de l'Essai 3

**Tableau 17**

| | | | Coag | | | Protéine | | | Purification | | |
|---|---|---|---|---|---|---|---|---|---|---|---|
| Etape | Code | Poids (g) | [ FIX :C] (UI / ml) | Q FIX :C | R FIX:C (%) | [Prot. Totales] | Q Prot.totales | R Prot. totales (%) | AS (UI/mg) | Pureté | Gain de pureté |
| Injection | MD | 12,7 | 16,5 | 210 | 100,0 | 4,430 | 56,3 | 100,0 | 3,7 | 1,6 | / |
| Non adsorbé | NR | 23,8 | 8,0 | 190 | 90,8 | 2,130 | 50,7 | 90,0 | 3,8 | 1,7 | 1,0 |
| Elution 1 | E1 | 2,7 | 1,8 | 4,8 | 2,3 | 0,011 | 0,0 | 0,1 | 163,6 | 72,1 | **43,9** |

**Tableau 18**

| | Am | | | Ag | | | Ratio | | |
|---|---|---|---|---|---|---|---|---|---|
| Code | [FIX :Am] (UI / ml) | Q FIX :Am (UI) | R FIX:Am (%) | [FIX :Ag] (UI / ml) | Q FIX :Ag (UI) | R FIX:Ag (%) | [C]/[Ag] | [Am]/[Ag] | [C]/[ Am] |
| MD | 9,4 | 119,1 | 100,0 | 47 | 597,4 | 100,0 | 0,4 | 0,2 | 1,8 |
| NR | 4,4 | 105,6 | 88,7 | 23 | 547,2 | 91,6 | 0,3 | 0,2 | 1,8 |
| E1 | 0,5 | 1,4 | 1,2 | 0,80 | 2,1 | 0,4 | **2,3** | **0,7** | 3,3 |

### Exemple 4 : Purification de diverses protéines à domaine GLA avec un support d'affinité sur lequel sont immobilisés des aptamères anti-GLA

Dans l'exemple 4, on a montré qu'un support d'affinité sur lequel sont immobilisés des aptamères anti-GLA est utilisé avec succès pour la purification d'une variété de protéines de la coagulation à domaine GLA.

Plus précisément on montre dans l'exemple 4 qu'un support d'affinité sur lequel sont immobilisés des aptamères anti-GLA Mapt-1 retient sélectivement le Facteur VII, le Facteur IX et le Facteur X.

### 5.1.1. Conditions expérimentales

### Mesure de Binding : Appareil Biacore T100

- Puce : Mapt-1 immobilisé sur surface streptavidine (Chip SA, GE) à 3596 RU sur la flow cell active n°2 (FC2). Tampon de course et de dilution d es échantillons : Tris 50mM / NaCl 50mM /CaCl2 10mM/ MgCl2 4mM/ pH = 7,4
- Flux : 30µl/min injection pendant 60sc, dissociation pendant 120 sc
- Signal : Signal sur Fc2 avec soustraction du signal de la Flow cell n°1 restée vierge
- Régénération : EDTA 10mM dans Tris 50mM pH= 7,4

### 5.1.2. Résultats

Les résultats sont représentés sur la Figure 2 .

La Figure 6 montre que le support d'affinité Mapt-1 retient sélectivement une variété de protéines à domaine GLA, en l'occurrence une variété de protéines humaines de la coagulation à domaine GLA comme le Facteur VII, le Facteur IX et le Facteur X.

### Exemple 5 : Capture d'acides nucléiques aptamères selon l'invention par du Facteur IX humain immobilisé sur un support.

On a fabriqué un support solide sur lequel ont été immobilisées des molécules de Facteur IX humain recombinant purifié. On a utilisé une préparation purifiée de facteur IX humain recombinant commercialisée sous le nom Benefix® par la société Wyeth.

On réalise une immobilisation du Facteur IX humain sur dextran carboxymethyl activé par NHS-EDC et se liant aux amines libres présentes sur le FIX..

Le Facteur IX recombinant humain est ainsi immobilisé avec un taux d'immobilisation de 3153 RU (1 RU correspond approximativement à 1 pg de produit immobilisé par mm²).

Des aptamères nucléiques de l'invention (pureté : 99%), respectivement les aptamères de séquences SEQ ID N°3 et 6 à 35, ont été dilués dans du tampon de course (Tris 50 mM, CaCl₂ 10 mM, MgCl₂ 4 mM, pH 7,5) de manière à obtenir autant d'échantillons d'aptamères que d'aptamères distincts à tester.

Chaque échantillon a été injecté de façon séquentielle sur une même puce (support solide) contenant le FIX recombinant humain immobilisé. Des contrôles sont obtenus en injectant des blancs ne contenant que du tampon de course. Toutes les injections ont été réalisées avec un débit 30 µl/min pendant 60 sec, après l'injection, du tampon de course a été injecté sur la puce à un débit identique pendant 120 sec.

Du tampon d'élution (EDTA, 10 mM) a ensuite été injecté pendant 60 sec avec un débit de 30 µl/min pour décrocher l'aptamère du FIX humain immobilisé.

La puce permet d'étudier en temps réel la formation et la rupture des interactions entre le FIX recombinantr humain immobilisé et chacun des aptamères de séquences SEQ ID N°3 et 6 à 35 testés, grâce à la résonance plasmonique de surface (RPS). Une liaison sur le FIX recombinant humain immobilisé se traduit par une augmentation du signal en unité de résonance (RU) enregistré par l'appareil (Figure 3). Ces analyses sont effectuées avec
l'appareil de RPS Biacore T100 (GE). La modélisation des interactions enregistrées sont effectuées à l'aide du Logiciel Biaevaluation (GE).

Les résultats obtenus montrent que tous les aptamères nucléiques testés se lient avec une affinité significative au Facteur IX recombinant humain plasmatique.

Les résultats de la Figure 3 montrent aussi que les 30 aptamères testés peuvent être classés dans quatre groupes principaux, selon leur niveau d'affinité pour le Facteur IX humain.

On peut noter en particulier la très forte affinité pour le Facteur IX humain de l'aptamères classés dans le Groupe 4 représenté sur la Figure 3. Parmi les 30 aptamères testés, l'aptamère ayant la plus forte affinité pour le Facteur IX humain, qui est désigné Mapt-1.2, consiste en l'aptamère de séquence SEQ ID N°35 [Mapt-1.2-CS].

### Exemple 6 : Capture d'acides nucléiques aptamères selon l'invention par du Facteur IX humain immobilisé sur un support.

On a fabriqué un support solide sur lequel ont été immobilisées des molécules de Facteur IX humain recombinant purifié. On a utilisé une préparation purifiée de facteur IX humain recombinant commercialisée sous le nom Benefix® par la société Wyeth.

On réalise une immobilisation du Facteur IX humain sur dextran carboxymethyl activé par NHS-EDC et se liant aux amines libres présentes sur le FIX.

Le Facteur IX recombinant humain est ainsi immobilisé avec un taux d'immobilisation de 3153 RU (1 RU correspond approximativement à 1 pg de produit immobilisé par mm²).

Des aptamères nucléiques de l'invention (pureté : 99%), respectivement les aptamères de séquences SEQ ID N° 35 [Mapt-1,2-CS] et SEQ ID N ° 36 [Mapt-1.2-CSO], ont été dilués dans du tampon de course (Tris 50 mM, CaCl₂ 10 mM, MgCl₂ 4 mM, pH 7,5) de manière à obtenir autant d'échantillons d'aptamères que d'aptamères distincts à tester.

L'aptamère Mapt-1.2.-CSO de séquence SED ID N°36 est dérivé de l'acide nucléique possédant la structure : 5'-SEQ ID N°1-SEQ ID N°35- SEQ ID N°2 ayant 80 nucléotides de longueur. Plus précisément, l'aptamère Mapt-1.2.-CSO de séquence SED ID N°36 consiste en l'acide nucléique allant du nucléotide en position 10 et se terminant au nucléotide en position 49 de l'acide nucléique possédant la structure : 5'-SEQ ID N°1-SEQ ID N°35-SEQ ID N2.

Chaque échantillon a été injecté de façon séquentielle sur une même puce (support solide) contenant le FIX recombinant humain immobilisé. Des contrôles sont obtenus en injectant des blancs ne contenant que du tampon de course. Toutes les injections ont été réalisées avec un débit 30 µl/min pendant 60 sec, après l'injection, du tampon de course a été injecté sur la puce à un débit identique pendant 120 sec.

Du tampon d'élution (EDTA, 10 mM) a ensuite été injecté pendant 60 sec avec un débit de 30µl/min pour décrocher l'aptamère du FIX humain immobilisé.

La puce permet d'étudier en temps réel la formation et la rupture des interactions entre le FIX recombinant humain immobilisé et chacun des aptamères de séquences SEQ ID N°SEQ ID N° 35 [Mapt-1.2-CS] et SEQ ID N° 36 [Mapt-1.2-CS O] testés, grâce à la résonance plasmonique de surface (RPS). Une liaison sur le FIX recombinant humain immobilisé se traduit par une augmentation du signal en unité de résonance (RU) enregistré par l'appareil (Figure 4). Ces analyses sont effectuées avec l'appareil de RPS Biacore T100 (GE). La modélisation des interactions enregistrées sont effectuées à l'aide du Logiciel Biaevaluation (GE).

Les résultats obtenus montrent que les deux aptamères nucléiques testés se lient avec une affinité significative au Facteur IX recombinant humain plasmatique.

Les résultats de la Figure 4 montrent aussi que, parmi les deux aptamères testés, le Mapt-1.2.-CSO (SEQ ID N° 36) présente un niveau d'affinité pour le Facteur IX humain significativement meilleur que le niveau d'affinité de l'aptamère Mapt-1.2.CS (SEQ ID N°35).

Les résultats de la figure 4 montrent aussi l'excellente stabilité de la liaison de l'aptamère Mapt-1.2.-CSO (SEQ ID N°36) sur le Facteur IX.

On rappelle que l'aptamère Mapt-1.2.-CS (SEQ ID N° 35) était l'aptamère présentant le plus haut niveau d'affinité parmi les 30 aptamères testés dans l'exemple 5

### Exemple 7 : Préparation d'un support d'affinité.

Le support d'affinité a été réalisé à partir d'un matériau de support solide consistant en une matrice sur laquelle on a greffé de la streptavidine (streptavidin-agarose - Novagen®).

On a introduit un volume de 1 ml de gel dans un conteneur consistant en une colonne (i.d. 11mm). On a lavé le gel avec de l'eau purifiée, pour éliminer le solvant de stockage

### Les caractéristiques du gel sont :

- *Capacité d'adsorption de biotine :* ≥ 85 nanomole / ml de gel
- *Test fonctionnel:* Capture >99% de thrombine biotinylée en 30 minutes à TA
- *Autres tests* : Protease-free , endo/exonucléase-free, RNase-free.
- *Conservateur:* 100 mM sodium phosphate pH7,5 + NaN3 0,02

La sortie de la colonne conditionnée (« packée ») (Hauteur de lit de gel = 1cm) est connectée à un détecteur d'absorbance équipé d'un filtre UV à 254 nm et d'un enregistreur.

Les aptamères nucléiques anti-FIX humain biotinylés comprenant l'acide nucléique de séquence SEQ ID N°4 [Mapt-1-WS] sont solubilisés dans de l'eau purifiée à la concentration finale de 0,5 mg/0,187 ml, soit une concentration molaire finale de 0,1 mM. La solution d'aptamères nucléiques a été activée à 95°C selon le cycle standard, pour l'immobilisation des aptamères sur le matériau support solide..

La solution d'aptamères nucléiques a été préalablement diluée par 4,8 ml d'eau purifiée puis 1,5 ml de tampon Mg⁺⁺ (5 x concentré).

Le détecteur d'absorbance est ajusté à 1 AUFS (Absorbance Unit Full Scale) et on enregistre la DO à 254 nm de cette solution à 0,575 UA₂₅₄.

La solution d'aptamères nucléiques biotinylés est injectée sur le gel streptavidin-agarose pré-conditionnée (« prépackée ») et mise en recirculation avec une pompe péristaltique à un débit de 2,5 ml/minute, soit un temps de contact sur le gel de 24 secondes (entrée/sortie E/S). Dans ces conditions, la DO à 254 nm se stabilise rapidement à 0,05 UA₂₅₄, soit une valeur de 91% de couplage théorique, c'est-à-dire 0,455 mg d'aptamères nucléiques par millilitre de gel.

Un lavage avec un tampon 10 mM CaCl₂ + 4 mM MgCl₂ puis en NaCl 2M est réalisé, afin d'éliminer les aptamères nucléiques qui ne sont pas fixées spécifiquement aux molécules de streptavidine greffées sur le matériau de support solide.

### Exemple 8 : Procédé de purification du Facteur IX recombinant humain

### A. Utilisation d'un support d'affinité comprenant l'aptamère Mapt-1WS immobilisé

Les supports d'affinité aptamères ont été testés à partir d'une préparation purifiée en FIX humain plasmatique. On précise que la préparation purifiée de FIX humain plasmatique consiste en un concentré de FIX à 60% de pureté commercialisé sous le nome Betafact® par le Laboratoire Français du Fractionnement et des Biotechnologies (LFB).

Le support d'affinité a été préparé conformément au protocole décrit à l'Exemple 7. Les aptamères consistent en des aptamères anti-GLA biotynylés qui ne comprennent pas de chaîne espaceur et désignés Mapt-1, qui comprennent l'acide nucléique de séquence SEQ ID N°4.

Le support d'affinité utilisé pour réaliser l'exemple 8 possède une densité de ligand théorique de 0,46 mg/ml. On a utilisé un volume de gel de 1 ml.

Le support d'affinité est équilibré avec un tampon de 0,05 M Tris-HCl, 0,01 M CaCl₂ à pH de 7,5.

Une charge de FIX humain plasmatique purifié dans une quantité de 200 UI (soit 800 µg) par millilitre de support d'affinité (gel) est utilisée pour l'étape de purification du FIX humain.

La solution de FIX humain plasmatique purifié, *préalablement ajustée,* à *tris 50 mM* + *NaCl 50mM* + *4 mM MgCl₂* + *10 mM CaCl₂ à pH 7,5.*
est injectée sur le gel Aptamère-agarose (support d'affinité) avec une pompe péristaltique à un débit de 0,1 ml/minute soit un temps de contact avec le support d'affinité de 10 minutes (E/S).

Après injection, le gel est lavé en tampon *tris 50 mM* + *NaCl 50mM* + *4 mM MgCl₂* + *10 mM CaCl₂ à pH 7,5.*

Un volume de 10 ml de solution non adsorbé est collecté.

Le FIX est élué par un tampon Tris-HCl 20mM + EDTA 10 mM à pH 7,5. La collecte du pic d'élution est réalisée suivant le profil de DO.

Pour régénérer le support d'affinité, on utilise un tampon de régénération de Tris-HCl 20 mM, NaCl 1M, propylène glycol 50%, à pH 7,5.

La figure 5 illustre un profil de chromatographie du FIX humain plasmatique, avec un suivi en continu des valeurs d'absorbance (D.O.) à 254 nanomètres.

Sur la Figure 5, l'injection (1) du concentré de FIX humain plasmatique est rapidement suivie du pic d'élimination (2) de la fraction non retenue sur le support d'affinité. Le support d'affinité continue de se saturer avec la protéine de la coagulation d'intérêt : des complexes entre (i) les aptamères nucléiques anti-GLA du support d'affinité et (ii) les molécules de FIX humain plasmatique initialement contenues dans la composition à purifier ont été formés. Après passage de la composition à purifier, on procède à une étape de lavage de la colonne avec le tampon de lavage spécifié précédemment. Puis on réalise l'étape d'élution, par injection de la solution tampon d'élution comprenant une concentration finale de 10 mM en EDTA.

Le pic d'absorption (3) sur la Figure 5 illustre la libération du FIX humain plasmatique à partir des complexes Aptamères nucléiques/FIX recombinant, lors de l'étape d'élution.

On note que les molécules de FIX humain plasmatique sont libérées rapidement et donc dans un faible volume. En conséquence, grâce au support d'affinité décrit ici, on obtient une solution d'élution de forte concentration en protéine FIX humain plasmatique.

Après élution, on a réalisé une étape de régénération du support d'affinité, avec un tampon Tris-HCl 20 mM, NaCl 1M, propylène glycol 50%, à pH 7,5.

On a réalisé un chromatogramme ainsi qu'une électrophorèse en gel SDS-PAGE avec gradient de Bis Acrylamide 4-12% sans réducteur, avec coloration au Bleu de Coomassie. Les résultats sont illustrés dans la figure 6.

L'analyse du chromatogramme de la figure 6 montre que l'éluat présente une bonne pureté chromatographique et des données d'activité biologiques est caractérisé par un maintien de la fonctionnalité du Facteur IX.

Ces analyses ont montré que la valeur du rapport « activité de FIX »/ « quantité de Facteur IX » retrouvée dans la fraction d'éluat est presque identique à la valeur du rapport « activité de FIX »/ « quantité de Facteur IX » retrouvée dans le produit de départ : cette valeur est de 1,2. Ces résultats montrent que le FIX n'a pas subi d'altération pendant le procédé de purification par chromatographie d'affinité.

Les résultats de l'Exemple 8-A montrent la capacité de l'aptamère Mapt-1WS qui a été immobilisé sur le support d'affinité en l'absence d'une chaîne espaceur, par exemple en l'absence d'une chaîne espaceur de polyéthylène glycol, à purifier le Facteur IX humain à partir d'un milieu complexe de départ contenant de nombreuses impuretés dérivées du plasma.

### B. Utilisation d'un support d'affinité comprenant l'aptamère Mapt-1.2.-CSO immobilisé

On a utilisé un support d'affinité sur lequel ont été immobilisées des molécules de l'aptamère Mapt-1.2.-CSO biotinylé comprenant une chaîne espaceur de PEG(C18).

L'aptamère Mapt-1.2.-CSO comprend l'acide nucléique de séquence SEQ ID N°36.

Avec ce support d'affinité, on a réalisé une purification de facteur IX humain plasmatique.

Le support d'affinité a été préparé conformément au protocole décrit à l'Exemple 7.

Le support d'affinité utilisé pour réaliser l'exemple X4 possède une densité de ligand théorique de 0,25 mg/ml. On a utilisé un volume de gel de 1 ml.

Le support d'affinité est équilibré avec un tampon de 0,05 M Tris-HCl, 0,01 M CaCl₂ à pH de 7,5.

Une charge de FIX humain plasmatique purifié à 50% dans une quantité de 207µg par millilitre de support d'affinité (gel) est utilisée pour l'étape de purification du FIX humain.

La solution de FIX humain plasmatique purifié, *préalablement ajustée à, tris 50 mM* + *10 mM CaCl₂ à pH 7,5,* est injectée sur le gel Aptamère-agarose (support d'affinité) avec une pompe péristaltique à un débit de 0,05 ml/minute soit un temps de contact avec le support d'affinité de 20 minutes (E/S).

Après injection, le gel est lavé en tampon *tris 50 mM* + *NaCl 50mM* + *4 mM MgCl₂* + *10 mM CaCl₂ à pH 7,5.*

Un volume de 10 ml de solution non adsorbé est collecté.

Le FIX est élué par un tampon Tris-HCl 50mM + EDTA 10 mM à pH 7,5. La collecte du pic d'élution est réalisée suivant le profil de DO.

Pour régénérer le support d'affinité, on utilise un tampon de régénération de NaCl 1M, propylène glycol 50%, à pH 7,5.

Le profil chromatographique est illustré dans la Figure **7****.** Sur la Figure **7****,** l'injection du concentré de FIX humain plasmatique est suivi du pic d'élimination (1) de la fraction non retenue sur le support d'affinité. Le support d'affinité continue de se saturer avec la protéine de la coagulation d'intérêt : des complexes entre (i) les aptamères nucléiques anti-GLA du support d'affinité et (ii) les molécules de FIX humain plasmatique initialement contenues dans la composition à purifier ont été formés. Après passage de la composition à purifier, on procède à une étape de lavage de la colonne avec le tampon de lavage spécifié précédemment. Puis on réalise l'étape d'élution, par injection de la solution tampon d'élution comprenant une concentration finale de 10 mM en EDTA.

On précise que la fraction non retenue contient 57% en poids des protéines contenues dans l'échantillon de départ, la fraction d'éluat représente 40% en poids des protéines contenues dans l'échantillon de départ et la fraction de régénération représente 3% en poids des protéines contenues dans l'échantillon de départ.

Le pic d'absorption (3) sur la Figure 7 illustre la libération du FIX humain plasmatique à partir des complexes Aptamères nucléiques/FIX recombinant, lors de l'étape d'élution.

Par ailleurs, la Figure 8 illustre l'excellente capacité du support d'affinité sur lequel sont immobilisées des molécules de l'aptamère Mapt-1.2.-CSO à purifier le Facteur IX humain.

Les résultats de la Figure 8 montrent que la fraction d'éluat présente une bonne pureté électrophorétique.

Les résultats de l'Exemple 8-B montrent la capacité de l'aptamère Mapt-1.2.-CSO qui a été immobilisé sur le support d'affinité à purifier le Facteur IX humain à partir d'un milieu complexe de départ contenant de nombreuses impuretés dérivées du plasma.

Ces résultats sont particulièrement inattendus car l'aptamère Mapt-1.2.-CSO comprend seulement une partie de la « core séquence » de l'aptamère Mapt-1.2-CS et comprend une région 5' consistant en la partie 3' d'une région destinée à être reconnue par des amorces consensus. Plus précisément, l'aptamère Mapt-1.2.-CSO comprend la région de 40 nucléotides nt10-nt 49 de l'acide nucléique de séquence 5'-SEQ ID N°1-SEQ ID N°3-SEQ ID N°2-3' de 80 nucléotides de longueur.

### Exemple 9 : Procédé de purification du Facteur VII recombinant humain

On a réalisé des essais de purification de Facteur IX recombinant produit dans le lait de porcs transgéniques pour le Facteur IX humain. Le lait des porcs transgéniques comprend un mélange de (i) Facteur IX humain recombinant transgénique actif possédant un domaine GLA correctement gamma-carboxylé et de (ii) Facteur IX humain recombinant transgénique non actif possédant un domaine GLA incorrectement gamma-carboxylé.

Du FIX humain recombinant transgénique produit chez le porc et pré-purifié par chromatographie sur support MEP HyperCel® a été dialysé contre le tampons utilisé pour l'équillibrage du support de chromatographie afin d'éliminer le citrate de sodium. L'étape de pré-purification sur MEP HyperCel a résulté en une composition contenant du Facteur IX humain à 1,8% de pureté.

Le support d'affinité a été préparé conformément au protocole décrit à l'Exemple 7.

Le support d'affinité Mapt-1WS sans chaîne espaceur utilisé pour réaliser l'exemple 9 possède une densité de ligand théorique de 0,46 mg/ml. On a utilisé un volume de gel de 1 ml. L'aptamère Mapt-1WS comprend l'acide nucléique de séquence SEQ ID N°4.

Le support d'affinité est équilibré avec un tampon de 0,05 M Tris-HCl, 0,01 M CaCl₂ à pH de 7,5.

Une charge de 302 UI (soit 1200 µg) de FIX recombinant humain pré-purifié dérivé de lait de truie transgénique par millilitre de support d'affinité (gel) est utilisée pour l'étape de purification du FIX humain.

La solution de FIX humain plasmatique purifié, *préalablement ajustée à, tris 50 mM* + *10 mM CaCl₂ à pH 7,5,* est injectée sur le gel Aptamère-agarose (support d'affinité) avec une pompe péristaltique à un débit de 0,1 ml/minute soit un temps de contact avec le support d'affinité de 10 minutes (E/S).

Il n'y a pas eu de modification apparente du produit dé départ lors de l'addition de chlorure de calcium.

Après injection, le gel est lavé en tampon *tris 50 mM* + *NaCl 50mM* + *4 mM MgCl₂* + *10 mM CaCl₂ à pH 7,5.*

Un volume de 10 ml de solution non adsorbé est collecté.

Le FIX est élué par un tampon Tris-HCl 20 mM + EDTA 10 mM à pH 7,5. La collecte du pic d'élution est réalisée suivant le profil de DO.

Pour régénérer le support d'affinité, on utilise un tampon de régénération de Tris-HCl 20 mM, NaCl 1M, propylène glycol 50%, à pH 7,5.

La figure 9 illustre un profil de chromatographie du FIX humain plasmatique, avec un suivi en continu des valeurs d'absorbance (D.O.) à 254 nanomètres.

Sur la Figure 9, l'injection (1) du concentré de FIX humain plasmatique est rapidement suivi du pic d'élimination (2) de la fraction non retenue sur le support d'affinité. Le support d'affinité continue de se saturer avec la protéine de la coagulation d'intérêt : des complexes entre (i) les aptamères nucléiques anti-GLA du support d'affinité et (ii) les molécules de FIX humain transgénique initialement contenues dans la composition à purifier ont été formés. Après passage de la composition à purifier, on procède à une étape de lavage de la colonne avec le tampon de lavage spécifié précédemment. Puis on réalise l'étape d'élution, par injection de la solution tampon d'élution comprenant une concentration finale de 10 mM en EDTA.

Le pic d'absorption (3) sur la Figure 9 illustre la libération du FIX humain transgénique à partir des complexes Aptamères nucléiques/FIX recombinant, lors de l'étape d'élution.

On note que les molécules de FIX humain transgénique sont libérées rapidement et donc dans un faible volume. En conséquence, grâce au support d'affinité de décrit ici, on obtient une solution d'élution de forte concentration en protéine FIX humain transgénique.

Après élution, on a réalisé une étape de régénération du support d'affinité, avec un tampon Tris-HCl à 20 mM, EDTA 10 mM à pH 7,5.

On a réalisé un chromatogramme ainsi qu'une électrophorèse en gel SDS-PAGE avec coloration au Bleu de coomassie. Les résultats sont illustrés dans la figure 10.

L'analyse du chromatogramme de la figure 10 montre que l'éluat présente une bonne pureté chromatographique et est caractérisé par un maintien de la fonctionnalité du Facteur IX.

Les résultats de l'Exemple 9 montrent la capacité de l'aptamère Mapt-1WS sans chaîne espaceur qui a été immobilisé sur le support d'affinité en l'absence d'une chaîne espaceur, par exemple en l'absence d'une chaîne espaceur de polyéthylène glycol, à purifier le Facteur IX humain à partir d'un milieu complexe de départ contenant de nombreuses impuretés dérivées du plasma.

Les résultats de l'étape de purification par chromatographie sont également présentés dans le Tableau T1 ci-dessous.

**Tableau T1**

| | Activité Spécifique (UI/mg) | Pureté (%) | Gain de pureté |
|---|---|---|---|
| Produit de départ | 4,0 | 1,8 | 1,0 |
| Fraction non retenue | 3,8 | 1,7 | 0,9 |
| Fraction d'élution | 104,5 | > 46,1 | > 26,0 |
| Fraction de régénération | 1,4 | 0,6 | 0,3 |

Les résultats présentés dans le Tableau T1 montrent que le support d'affinité préparé à l'exemple 9 possède une excellente capacité de purification du Facteur IX humain, et plus précisément du Facteur IX humain recombinant produit dans le lait d'une truie transgénique pour le facteur IX humain. En particulier, les résultats du tableau T1 montrent que l'on obtient un gain de pureté d'au moins 26 fois.

### Exemple 10 : Capture d'acides nucléiques aptamères selon l'invention par du Facteur VII humain immobilisé sur un support.

On a fabriqué un support solide sur lequel ont été immobilisées des molécules de Facteur VII humain recombinant purifié. On a utilisé une préparation purifiée de facteur VII humain recombinant commercialisée sous le nom Novoseven® par la société Laboratoires Français du Fractionnement et des Biotechnologies (LFB).

On réalise une immobilisation du Facteur VII humain sur dextran carboxymethyl activé par NHS-EDC et se liant aux amines libres présentes sur le FIX.

Le Facteur VII recombinant humain est ainsi immobilisé avec un taux d'immobilisation de 2525 RU (1 RU correspond approximativement à *1 pg de produit immobilisé par mm²).*

Des aptamères nucléiques de l'invention (pureté : 99%) ont été dilués dans du tampon de course (Tris 50 mM, CaCl₂ 10 mM, MgCl₂ 4 mM, pH 7,5) de manière à obtenir autant d'échantillons d'aptamères que d'aptamères distincts à tester.

Chaque échantillon a été injecté de façon séquentielle sur une même puce (support solide) contenant le FVII recombinant humain immobilisé. Des contrôles sont obtenus en injectant des blancs ne contenant que du tampon de course. Toutes les injections ont été réalisées avec un débit 30 µl/min pendant 60 sec, après l'injection, du tampon de course a été injecté sur la puce à un débit identique pendant 120 sec.

Du tampon d'élution (EDTA, 5 mM) a ensuite été injecté pendant 60 sec avec un débit de 30 µl/min pour décrocher l'aptamère du FVII humain immobilisé. La puce permet d'étudier en temps réel la formation et la rupture des interactions entre le FVII recombinant humain immobilisé et chacun des aptamères testés, grâce à la résonance plasmonique de surface (RPS). Une liaison sur le FVII recombinant humain immobilisé se traduit par une augmentation du signal en unité de résonance (RU) enregistré par l'appareil (Figure 11). Ces analyses sont effectuées avec l'appareil de RPS Biacore T100 (GE). La modélisation des interactions enregistrées sont effectuées à l'aide du Logiciel Biaevaluation (GE).

Les résultats obtenus montrent que tous les aptamères nucléiques testés se lient avec une affinité significative au Facteur IX recombinant humain plasmatique.

Les résultats de la Figure 11 montrent aussi que les 27 aptamères testés peuvent être classés dans quatre groupes principaux, selon leur niveau d'affinité pour le Facteur VII humain.

On peut noter en particulier la très forte affinité pour le Facteur VII humain des aptamères classés dans le Groupe 4 représenté sur la Figure 11. Parmi les 27 aptamères testés, l'aptamère ayant la plus forte affinité pour le Facteur IX humain, qui est désigné Mapt-2.2, consiste en l'aptamère de séquence SEQ ID N°38 Mapt-2.2-CS.

### Exemple 11 : Procédé de purification du Facteur VII humain plasmatique

### A. Utilisation d'un support d'affinité comprenant l'aptamère Mapt-2CS immobilisé

Les supports d'affinité aptamères ont été testés à partir d'une préparation purifiée en FVII humain plasmatique. On précise que le préparation purifiée de FVII humain plasmatique consiste en un concentré de FVII à 98% de pureté commercialisé sous le nom ACSET® par le Laboratoire Français du Fractionnement et des Biotechnologies (LFB).

Le support d'affinité a été préparé conformément au protocole décrit à l'Exemple 7. Le support d'affinité de l'exemple 11-A comprend l'aptamère Mapt-2-CS comprenant l'acide nucléique de séquence SEQ ID N°37 qui est immobilisé.

Le support d'affinité utilisé pour réaliser l'exemple 11-A possède une densité de ligand théorique de 0,40 mg/ml. On a utilisé un volume de gel de 1 ml.

Le support d'affinité est équilibré avec un tampon de 0,05 M Tris-HCl, 0,01 M CaCl₂, 0,05 mM MgCl₂ à pH de 7,5.

Une charge de FVII humain plasmatique purifié est utilisée pour l'étape de purification du FVII humain.

La solution de FVII humain plasmatique purifié, *préalablement ajustée à 4 mM MgCl₂ et 10 mM CaCl₂ et pH 7,5,* est injectée sur le gel Aptamère-agarose (support d'affinité) avec une pompe péristaltique à un débit de 0,1 ml/minute soit un temps de contact avec le support d'affinité de 10 minutes (E/S).

Après injection, le gel est lavé en tampon *tris 50 mM* + *NaCl 50mM* + *4 mM MgCl₂* + *10 mM CaCl₂ à pH 7,5.*

Un volume de 10 ml de solution non adsorbé est collecté.

Le FVII est élué par un tampon Tris-HCl 20mM + EDTA 10 mM à pH 7,5. La collecte du pic d'élution est réalisée suivant le profil de DO.

Pour régénérer le support d'affinité, on utilise un tampon de régénération de Tris-HCl 20 mM, NaCl 1M, propylène glycol 50%, à pH 7,5.

La figure 12 illustre un profil de chromatographie du FVII humain plasmatique, avec un suivi en continu des valeurs d'absorbance (D.O.) à 254 nanomètres.

Sur la Figure 12, l'injection (1) du concentré de FIX humain plasmatique est rapidement suivi du pic d'élimination (2) de la fraction non retenue sur le support d'affinité. Le support d'affinité continue de se saturer avec la protéine de la coagulation d'intérêt : des complexes entre (i) les aptamères nucléiques anti-GLA du support d'affinité et (ii) les molécules de FVII humain plasmatique initialement contenues dans la composition à purifier ont été formés. Après passage de la composition à purifier, on procède à une étape de lavage de la colonne avec le tampon de lavage spécifié précédemment. Puis on réalise l'étape d'élution, par injection de la solution tampon d'élution comprenant une concentration finale de 10 mM en EDTA.

Le pic d'absorption (3) sur la Figure 12 illustre la libération du FIX humain plasmatique à partir des complexes Aptamères nucléiques/FIX recombinant, lors de l'étape d'élution.

On note que les molécules de FIX humain plasmatique sont libérées rapidement et donc dans un faible volume. En conséquence, grâce au support d'affinité décrit ici, on obtient une solution d'élution de forte concentration en protéine FIX humain plasmatique.

Après élution, on a réalisé une étape de régénération du support d'affinité, avec un tampon Tris à 20 mM.

On a réalisé un chromatogramme ainsi qu'une électrophorèse en gel SDS-PAGE avec coloration au Bleu de Coomassie. Les résultats sont illustrés dans la figure 13.

L'analyse du chromatogramme de la figure 13 montre que l'éluat présente une bonne pureté chromatographique et est caractérisé par un maintien de la fonctionnalité du Facteur VII.

L'analyse du chromatogramme de la figure 13 montre que seules les formes actives du FVII humain plasmatique purifié de départ ont été retenues sur le support d'affinité. Les formes inactives présentent dans la composition purifiée de départ, y compris les formes de FVII mal glycosylées et les formes de FVII Des-Gla, n'ont pas été retenues sur le support d'affinité.

Les résultats de l'Exemple 11-A montrent la capacité de l'aptamère Mapt-2CS qui a été immobilisé sur le support d'affinité en l'absence d'une chaîne espaceur, par exemple en l'absence d'une chaîne espaceur de polyéthylène glycol, à purifier le Facteur VII humain à partir d'un milieu complexe de départ contenant de nombreuses impuretés dérivées du plasma.

### B. Utilisation d'un support d'affinité comprenant l'aptamère Mapt-2.2CS immobilisé

On a utilisé un support d'affinité sur lequel ont été immobilisées des molécules de l'aptamère Mapt-2.2.-CS biotinylé comprenant une chaîne espaceur de PEG(C18).

Avec ce support d'affinité, on a réalisé une purification de facteur VII humain plasmatique à 98% de pureté (ACSET®)..

Le support d'affinité a été préparé conformément au protocole décrit à l'Exemple 7. Le support d'affinité de l'exemple 11-B comprend des aptamères Mapt-2.2.-CS comprenant l'acide nucléique de séquence SEQ ID N°37 qui sont immobilisés.

Le support d'affinité utilisé pour réaliser l'exemple 11-B possède une densité de ligand théorique de 0,50 mg/ml. On a utilisé un volume de gel de 1 ml.

Le support d'affinité est équilibré avec un tampon de 0,05 M Tris-HCl, 0,05 M NaCl, 0,01 M CaCl₂, 0,004 M MgCl₂ à pH de 7,5.

Une charge de FVII humain plasmatique purifié à 98% dans une quantité de 115 µg par millilitre de support d'affinité (gel) est utilisée pour l'étape de purification du FVII humain.

La solution de FVII humain plasmatique purifié, *préalablement ajustée à 4 mM MgCl₂ et 10 mM CaCl₂ et pH 7,5,* est injectée sur le gel Aptamère-agarose (support d'affinité) avec une pompe péristaltique à un débit de 0,05 ml/minute soit un temps de contact avec le support d'affinité de 20 minutes (E/S).

Après injection, le gel est lavé en tampon *tris 50 mM* + *NaCl 50mM* + *4 mM MgCl₂* + *10 mM CaCl₂ à pH 7,5.*

Un volume de 10 ml de solution non adsorbé est collecté.

Le FVII est élué par un tampon Tris-HCl 50mM + EDTA 10 mM à pH 7,5. La collecte du pic d'élution est réalisée suivant le profil de DO.

Pour régénérer le support d'affinité, on utilise un tampon de régénération de NaCl 1M, 50% propylène glycol à pH 7,5.

Le profil chromatographique est illustré dans la Figure 14. Sur la Figure 14, l'injection du concentré de FVII humain plasmatique est suivi du pic d'élimination (1) de la fraction non retenue sur le support d'affinité. Le support d'affinité continue de se saturer avec la protéine de la coagulation d'intérêt : des complexes entre (i) les aptamères nucléiques anti-GLA du support d'affinité et (ii) les molécules de FIX humain plasmatique initialement contenues dans la composition à purifier ont été formés. Après passage de la composition à purifier, on procède à une étape de lavage de la colonne avec le tampon de lavage spécifié précédemment. Puis on réalise l'étape d'élution, par injection de la solution tampon d'élution comprenant une concentration finale de 10 mM en EDTA.

Le pic d'absorption (3) sur la Figure 14 illustre la libération du FVII humain plasmatique à partir des complexes Aptamères nucléiques/FVII recombinant, lors de l'étape d'élution.

On précise que la fraction non retenue représente 9% en poids des protéines contenues dans l'échantillon de départ, la fraction d'élution représente 78% en poids des protéines contenues dans l'échantillon de départ et la fraction de régénération représente 13% en poids des protéines contenues dans l'échantillon de départ.

Par ailleurs, la Figure 15 illustre l'excellente capacité du support d'affinité sur lequel sont immobilisées des molécules de l'aptamère Mapt-2.2.-CS à purifier le Facteur VII humain.

Les résultats de la Figure 15 montrent que la fraction d'éluat présente une bonne pureté électrophorétique.

L'analyse du chromatogramme de la figure 15 montre que seules les formes actives du FVII humain plasmatique purifié de départ ont été retenues sur le support d'affinité. Les formes inactives présentent dans la composition purifiée de départ, y compris les formes de FVII mal glycosylées et les formes de FVII Des-Gla, n'ont pas été retenues sur le support d'affinité.

Les résultats de l'Exemple 11-B montrent la capacité de l'aptamère Mapt-2.2.-CS qui a été immobilisé sur le support d'affinité à purifier le Facteur IX humain à partir d'un milieu complexe de départ contenant de nombreuses impuretés dérivées du plasma.

### Exemple 12 : Optimisation des conditions de capture des aptamères sur le support d'affinité.

On a fabriqué un support solide sur lequel ont été immobilisées des molécules de l'aptamère nucléique de l'invention de séquence SEQ ID N° 39 de 80 nucléotides, aussi désigné ici « Mapt2 ». Préalablement à sa fixation sur le support solide, l'extrémité 5' de l'aptamère Mapt2 a été chimiquement couplée à une chaîne espaceur constituée de 5 molécules de PEG(C18). Puis, l'extrémité libre de la chaîne espaceur, opposée à l'extrémité couplée à l'aptamère, a été couplée à une molécule de biotine.

On dispose d'un support solide contenant des molécules de streptavidine immobilisées (Serie S sensor Chip SA, GE)

Puis, on a mis en contact le support solide ci-dessus avec les composés aptamères ci-dessus afin d'immobiliser les acides nucléiques de séquence SEQ ID N°39, par association non covalente entre les molécules de streptavidine du support et les molécules de biotine des composés aptamères.

L'aptamère Mapt2 est ainsi immobilisé avec un taux d'immobilisation de 4900 RU (*1 RU correspond approximativement à 1 pg de produit immobilisé par mm²*).

Du FVII humain purifié à partir du plasma (FVII HP, pureté : 99%) a été dilué dans divers tampons de course, respectivement :
- Tampon 1 : Tris 50 mM, NaCl 50 mM, CaCl₂ 10 mM, MgCl₂ 4 mM, pH 7,5 ;
- Tampon 2 : Tris 50 mM, NaCl 50 mM, CaCl₂ 10 mM, MgCl₂ 10 mM, pH 7,5 ;
- Tampon 3 : Tris 50 mM, NaCl 50 mM, MgCl₂ 20 mM, pH 7,5 ;
- Tampon 4 : Tris 50 mM, CaCl₂ 10 mM, MgCl₂ 4 mM, pH 7,5 ; et
- Tampon 5 : Tris 50 mM, CaCl₂ 10 mM, pH 7,5 ;

Chaque échantillon a été injecté de façon séquentielle sur une même puce (support solide) contenant l'aptamère Mapt2 immobilisé par une interaction biotine-streptavidine. Des contrôles sont obtenus en injectant des blancs ne contenant que du tampon de course. Toutes les injections ont été réalisées avec un débit 30µl/min pendant 60 sec, après l'injection, du tampon de course a été injecté sur la puce à un débit identique pendant 120 sec.

Du tampon d'élution (EDTA, 5mM) a ensuite été injecté pendant 30 sec avec un débit de 30µl/min pour décrocher le FVII HP de l'aptamère..

La puce permet d'étudier en temps réel la formation et la rupture des interactions entre le FVII HP et l'aptamère immobilisé grâce à la résonance plasmonique de surface (RPS). Une liaison sur l'aptamère immobilisé se traduit par une augmentation du signal en unité de résonance (RU) enregistré par l'appareil. Ces analyses sont effectuées avec l'appareil de RPS Biacore T100 (GE). La modélisation des interactions enregistrées sont effectuées à l'aide du Logiciel Biaevaluation (GE).

Les résultats sont présentés sur la figure 16.

Les résultats de la figure 16 montrent que les conditions de capture optimales sont obtenues avec le Tampon 5, qui ne comprend pas de NaCl ni de MgCl₂.

Les résultats de la figure 16 montrent que la présence de MgCl₂ n'est pas nécessaire pour la capture optimale du FVII par les molécules d'aptamères immobilisées. Les résultats montrent que la présence de MgCl₂ est même défavorable à la fixation optimale du FVII.

De plus, les résultats de la figure 16 montrent que la présence de NaCl dans le tampon de course est défavorable à une fixation optimale du FVII aux molécules d'aptamères immobilisées sur le support d'affinité.

Les figures 17 et 18 illustrent l'analyse cinétique de la fixation du FVII humain plasmatique aux aptamères Mapt-2 immobilisés sur le support d'affinité, respectivement avec le Tampon 1 (figure 17) et avec le tampon 5 (figure 18).

Les résultats de la figure 17 montrent qu'avec le tampon 1, l'aptamère présente une affinité pour le FVII humain de 148 nM (valeur de Kd), une valeur de ka de 3,3 10³ M⁻¹s⁻¹, et *une valeur de kd de 4,8 10⁻⁴s⁻¹*

Les résultats de la figure 18 montrent qu'avec le tampon 5, l'aptamère présente une affinité pour le FVII humain de 13 nM (valeur de Kd), une valeur de ka de 4,7 10⁴ M⁻¹s⁻¹, et *une valeur de kd de 6 10⁻⁴s⁻¹*

### Exemple 13: Optimisation des conditions de lavage des aptamères sur le support d'affinité.

On a utilisé le support d'affinité décrit pour l'exemple X8 ci-dessus.

La figure 19 présente des résultats relatifs aux éventuels effets de différentes conditions de lavage sur le maintien de la fixation du FVII humain sur les aptamères Mapt-2 immobilisés sur le support d'affinité. On a testé des tampons de lavage suivants : (1) tampon Tris 50 mM, NaCl 1 M, CaCl₂ 10 mM, MgCl₂ 4 mM, pH 7,5, (2) tampon Tris 50 mM, NaCl 2 M, CaCl₂ 10 mM, MgCl₂ 4 mM, pH 7,5, (3) tampon Tris 50 mM, NaCl 3 M, CaCl₂ 10 mM, MgCl₂ 4 mM, pH 7,5 et (4) tampon Tris 50 mM, EDTA 10 mM.

Les résultats montrent que l'utilisation de concentrations croissantes en NaCl ne provoque pas d'altération de la liaison du FVII humain aux aptamères Mapt-2.

Les résultats de la figure 19 montrent que le FVII reste fixé à l'aptamère Mapt-2, même lorsqu'on réalise une étape de lavage du support d'affinité avec un tampon de force ionique élevée.

Les résultats de la figure 19 montrent aussi que le FVII est élué avec l'EDTA.

La figure 20 présente des résultats relatifs aux éventuels effets de différentes conditions de lavage sur le maintien de la fixation du FVII humain sur les aptamères Mapt-2 immobilisés sur le support d'affinité. On a testé des tampons de lavage suivants : (1) tampon Tris 50 mM, CaCl₂ 10 mM, MgCl₂ 4 mM, pH 7,5, (2) tampon Tris 50 mM, CaCl₂ 10 mM, MgCl₂ 4 mM, NaCl 1M, pH 7,5, (3) tampon Tris 50 mM, CaCl₂ 10 mM, MgCl₂ 4 mM, NaCl 2M, pH 7,5 (4) tampon Tris 50 mM, CaCl₂ 10 mM, MgCl₂ 4 mM, NaCl 3M, pH 7,5 et (5) tampon Tris 50 mM, EDTA 10 mM.

Les résultats montrent que l'utilisation de concentrations croissantes en NaCl ne provoque pas d'altération de la liaison du FVII humain aux aptamères Mapt-2.

Les résultats de la figure 20 montrent que le FVII reste fixé à l'aptamère Mapt-2, même lorsqu'on réalise une étape de lavage du support d'affinité avec un tampon de force ionique élevée.

Les résultats de la figure 20 montrent aussi que le FVII est élué avec l'EDTA.

La figure 21 présente des résultats relatifs aux éventuels effets de différentes conditions de lavage sur le maintien de la fixation du FVII humain sur les aptamères Mapt-2 immobilisés sur le support d'affinité. On a testé des tampons de lavage suivants : (1) tampon éthanol à 10%, (3) tampon Tris 50 mM, CaCl₂ 10 mM, MgCl₂ 4 mM, NaCl 1M, pH 7,5, (3) tampon Tris 50 mM, CaCl₂ 10 mM, MgCl₂ 4 mM, NaCl 2M, pH 7,5 (4) tampon Tris 50 mM, CaCl₂ 10 mM, MgCl₂ 4 mM, NaCl 3M, pH 7,5 et (5) tampon Tris 50 mM, EDTA 10 mM.

Les résultats de la figure 21 montrent que l'utilisation d'éthanol à l'étape de lavage ne provoque pas d'altération de la liaison du FVII humain aux aptamères Mapt-2.

Les résultats montrent que l'utilisation de concentrations croissantes en NaCl à l'étape de lavage ne provoque pas d'altération de la liaison du FVII humain aux aptamères Mapt-2.

Les résultats de la figure 21 montrent que le FVII reste fixé à l'aptamère Mapt-2, même lorsqu'on réalise une étape de lavage du support d'affinité avec un tampon de force ionique élevée.

Les résultats de la figure 21 montrent aussi que le FVII est élué avec l'EDTA.

### Exemple 14: Optimisation des conditions de lavage des aptamères sur le support d'affinité.

On a fabriqué un support solide sur lequel ont été immobilisées des molécules de l'aptamère nucléique Mapt-1 de l'invention de séquence SEQ ID N° 4, aussi désigné ici « Mapt1 ». Préalablement à sa fixation sur le support solide, l'extrémité 5' de l'aptamère Mapt2 a été chimiquement couplée à une chaîne espaceur constituée de 5 molécules de PEG(C18). Puis, l'extrémité libre de la chaîne espaceur, opposée à l'extrémité couplée à l'aptamère, a été couplée à une molécule de biotine.

On dispose d'un support solide contenant des molécules de streptavidine immobilisées (Serie S sensor Chip SA, GE)

Puis, on a mis en contact le support solide ci-dessus avec les composés aptamères ci-dessus afin d'immobiliser les acides nucléiques de séquence SEQ ID N°4, par association non covalente entre les molécules de streptavidine du support et les molécules de biotine des composés aptamères.

L'aptamère Mapt1 est ainsi immobilisé avec un taux d'immobilisation de 4900 RU (1 RU correspond approximativement à 1 pg de produit immobilisé par mm²).

Chaque échantillon a été injecté de façon séquentielle sur une même puce (support solide) contenant l'aptamère Mapt1 immobilisé par une interaction biotine-streptavidine. Des contrôles sont obtenus en injectant des blancs ne contenant que du tampon de course. Toutes les injections ont été réalisées avec un débit 30µl/min pendant 60 sec, après l'injection, du tampon de course a été injecté sur la puce à un débit identique pendant 120 sec.

Du tampon d'élution (EDTA, 5mM) a ensuite été injecté pendant 30 sec avec un débit de 30µl/min pour décrocher le FVII HP de l'aptamère..

La puce permet d'étudier en temps réel la formation et la rupture des interactions entre le FIX et l'aptamère immobilisé grâce à la résonance plasmonique de surface (RPS). Une liaison sur l'aptamère immobilisé se traduit par une augmentation du signal en unité de résonance (RU) enregistré par l'appareil. Ces analyses sont effectuées avec l'appareil de RPS Biacore T100 (GE). La modélisation des interactions enregistrées sont effectuées à l'aide du Logiciel Biaevaluation (GE).

La figure 22 présente des résultats relatifs aux éventuels effets de différentes conditions de lavage sur le maintien de la fixation du FIX humain sur les aptamères Mapt-1 immobilisés sur le support d'affinité. On a testé des tampons de lavage suivants : (1) tampon Tris 50 mM, CaCl₂ 10 mM, MgCl₂ 4 mM, NaCl 1M, pH 7,5, (2) tampon Tris 50 mM, CaCl₂ 10 mM, MgCl₂ 4 mM, NaCl 2M, pH 7,5 (3) tampon Tris 50 mM, CaCl₂ 10 mM, MgCl₂ 4 mM, NaCl 3M, pH 7,5 et (5) tampon Tris 50 mM, EDTA 10 mM.

Les résultats montrent que l'utilisation de concentrations croissantes en NaCl à l'étape de lavage ne provoque pas d'altération de la liaison du FIX humain aux aptamères Mapt-1.

Les résultats de la figure 22 montrent que le FIX reste fixé à l'aptamère Mapt-1, même lorsqu'on réalise une étape de lavage du support d'affinité avec un tampon de force ionique élevée.

Les résultats de la figure 22 montrent aussi que le FIX est élué avec l'EDTA.

La figure 23 présente des résultats relatifs aux éventuels effets du propylène glycol (à 50%) sur le maintien de la fixation du FIX humain sur les aptamères Mapt-1 immobilisés sur le support d'affinité. On a testé un tampon de lavage Tris 50 mM, CaCl₂ 10 mM, propylène glycol à 50%, à pH 7,5 .

Les résultats de la figure 23 montrent que l'utilisation de propylène glycol à l'étape de lavage ne provoque pas d'altération de la liaison du FIX humain aux aptamères Mapt-1.

## Revendications

1. Utilisation d'un aptamère d'ADN pour purifier simultanément les facteur VII, facteur IX et facteur X dans du plasma sanguin, par :
a) mise en contact dudit plasma sanguin avec un support d'affinité sur lequel est immobilisé ledit aptamère désoxyribonucléique se liant spécifiquement auxdits facteur VII, facteur IX et facteur X, afin de former des complexes entre (i) ledit aptamère désoxyribonucléique et (ii) les facteur VII, facteur IX et facteur X,
b) libération desdits facteur VII, facteur IX et facteur X à partir des complexes formés à l'étape a), et
c) récupération desdits facteur VII, facteur IX et facteur X sous une forme purifiée,
**caractérisée en ce que** ledit aptamère désoxyribonucléique consiste en un aptamère de séquence choisie parmi les séquences SEQ ID NO : 3, 4 et 6 à 36.

2. Utilisation selon la revendication 1, **caractérisée en ce que** ledit aptamère désoxyribonucléique est inclus dans la structure d'un composé de formule (I) suivante :
[IMM]ₓ-[SPAC]_{y}-[APT] (I),
dans laquelle :
- [IMM] signifie un composé d'immobilisation sur un support,
- [SPAC] signifie une chaîne espaceur,
- [APT] signifie un acide désoxyribonucléique se liant spécifiquement auxdits facteur VII, facteur IX et facteur X,
- x est un entier égal à 0 ou 1, et
- y est un entier égal à 0 ou 1.

3. Utilisation selon l'une des revendications 1 à 2, **caractérisée en ce que** ledit aptamère désoxyribonucléique consiste en un aptamère de séquence SEQ ID NO : 4.

4. Utilisation selon l'une quelconque des revendications précédentes, une étape de lavage étant incluse à la fin de l'étape a) et préalable à l'étape b), avec une solution ayant une concentration en NaCl d'au moins 0.5 M.

5. Utilisation selon l'une quelconque des revendications précédentes, dans laquelle l'élution à l'étape b) est réalisée avec un tampon d'élution contenant un agent chélateur des ions divalents.

## Patentansprüche

1. Verwendung eines DNA-Aptamers zur gleichzeitigen Reinigung von Faktor VII, Faktor IX und Faktor X im Blutplasma durch:
a) Inkontaktbringen besagten Blutplasmas mit einem Affinitätsträger, auf dem besagtes Desoxyribonukleinsäure-Aptamer immobilisiert ist, das spezifisch an besagten Faktor VII, Faktor IX und Faktor X bindet, um Komplexe zwischen (i) besagtem Desoxyribonukleinsäure-Aptamer und (ii) Faktor VII, Faktor IX und Faktor X zu bilden,
b) Freisetzen besagten Faktors VII, Faktors IX und Faktors X aus den in Schritt a) gebildeten Komplexen, und
c) Wiedergewinnen besagten Faktors VII, Faktors IX und Faktors X in gereinigter Form,
**dadurch gekennzeichnet, dass** besagtes Desoxyribonukleinsäure-Aptamer aus einem Aptamer der Sequenz, ausgewählt aus den Sequenzen SEQ ID NO: 3, 4 und 6 bis 36, besteht.

2. Verwendung gemäß Anspruch 1, **dadurch gekennzeichnet, dass** besagtes Desoxyribonukleinsäure-Aptamer in der Struktur einer Verbindung der folgenden Formel (I) enthalten ist:
[IMM]ₓ-[SPAC]_{y}-[APT] (I),
in der:
- [IMM] eine Verbindung zur Immobilisierung auf einem Träger bedeutet,
- [SPAC] eine Spacer-Kette bedeutet,
- [APT] eine Desoxyribonukleinsäure bedeutet, die spezifisch an besagten Faktor VII, Faktor IX und Faktor X bindet,
- x eine ganze Zahl gleich 0 oder 1 ist, und
- y eine ganze Zahl gleich 0 oder 1 ist.

3. Verwendung gemäß einem der Ansprüche 1 bis 2, **dadurch gekennzeichnet, dass** besagtes Desoxyribonukleinsäure-Aptamer aus einem Aptamer der Sequenz SEQ ID NO: 4 besteht.

4. Verwendung gemäß einem der vorhergehenden Ansprüche, wobei ein Waschschritt am Ende von Schritt a) und vor Schritt b) mit einer Lösung, die eine NaCl-Konzentration von wenigstens 0,5 M besitzt, eingeschlossen ist.

5. Verwendung gemäß einem der vorhergehenden Ansprüche, wobei die Elution in Schritt b) mit einem Elutionspuffer durchgeführt wird, der einen Chelatbildner für zweiwertige Ionen enthält.

## Claims

1. Use of a DNA aptamer for simultaneously purifying factor VII, factor IX and factor X from blood plasma, by :
a) contacting said blood plasma with an affinity support on which said deoxyribonucleic aptamer that specifically binds said factor VII, factor IX and factor X is immobilized, so as to form complexes between (i) said deoxyribonucleic aptamer and (ii) factor VII, factor IX and factor X,
b) releasing said factor VII, factor IX and factor X from the complexes formed in step a), and
c) recovering said factor VII, factor IX and factor X in a purified form,
wherein said deoxyribonucleic aptamer consists in an aptamer having a sequence selected from SEQ ID NO : 3,4 and 6 to 36.

2. Use according to claim 1, wherein said deoxyribonucleic aptamer is included in the following structure of a compound of formula (I) :
[IMM]ₓ-[SPAC]_{y}-[APT] (I),
in which:
- [IMM] means a compound for immobilization on a support,
- [SPAC] means a spacer chain,
- [APT] means a deoxyribonucleic acid which binds specifically to said factor VII, factor IX and factor X,
- x is an integer equal to 0 or 1, and
- y is an integer equal to 0 or 1.

3. Use according to any of claims 1 to 2, wherein said deoxyribonucleic aptamer consists in an aptamer of SEQ ID NO : 4.

4. Use according to any one of preceding claims, wherein a washing step is included at the end of step a) and before step b), with a solution having a concentration in NaCl of at least 0.5 M.

5. Use according to any one of preceding claims, wherein the elution in step b) is performed with an elution buffer containing a divalent-ion chelating agent.


### SEQUENCE LISTING

<110> LFB-BIOTECHNOLOGIES
<120> Procédé pour la purification de protéines de la coagulation a domaine GLA
<130> W730PCT
<150> FR0955406
   <151> 2009-07-31
<160> 39
<170> PatentIn version 3.3
<210> 1
   <211> 18
   <212> DNA
   <213> artificial sequence
<220>
   <223> partie 5' d'aptamère
<400> 1
   gggagatagc cacgacct 18
<210> 2
   <211> 18
   <212> DNA
   <213> artificial sequence
<220>
   <223> partie 3' d'aptamère
<400> 2
   tccaggctgt gcgaaagc 18
<210> 3
   <211> 44
   <212> DNA
   <213> artificial sequence
<220>
   <223> partie centrale d'aptamère
<400> 3
   cgcacatgac ttgaagttaa acgcgaatta caaacccagc cccc 44
<210> 4
   <211> 80
   <212> DNA
   <213> artificial sequence
<220>
   <223> aptamère
<400> 4
   gggagatagc cacgacctcg cacatgactt gaagttaaac gcgaattaca aacccagccc 60
   cctccaggct gtgcgaaagc 80
<210> 5
   <211> 79
   <212> DNA
   <213> artificial sequence
<220>
   <223> aptamere
<400> 5
   gctttcgcac agcctggaca cttcccatta gggggcattc agctaaatac gcagcggctt 60
   gaggtcgtgg ctatctccc 79
<210> 6
   <211> 43
   <212> DNA
   <213> artificial sequence
<220>
   <223> aptamere
<400> 6
   cgcacacgac ttgaagttaa acgcgaatta cagaccatgc cca 43
<210> 7
   <211> 43
   <212> DNA
   <213> artificial sequence
<220>
   <223> aptamere
<400> 7
   cgcacacgac ttgaagttaa acgcgaatta cggaccaatc cca 43
<210> 8
   <211> 43
   <212> DNA
   <213> artificial sequence
<220>
   <223> aptamere
<400> 8
   cgcacacgac ttgaagttaa acgcgaacta cagaccaagc cca 43
<210> 9
   <211> 43
   <212> DNA
   <213> artificial sequence
<220>
   <223> aptamere
<400> 9
   cgcacatgac ttgaagttaa acgcgaacta cataccaagc cca 43
<210> 10
   <211> 44
   <212> DNA
   <213> artificial sequence
<220>
   <223> aptamere
<400> 10
   cgcacatgac ttgaagttaa acgcgaatta caaacccagc cccc 44
<210> 11
   <211> 44
   <212> DNA
   <213> artificial sequence
<220>
   <223> aptamere
<400> 11
   cgcacaagac ttgaagttaa acgcgaatta caaacccagc cccc 44
<210> 12
   <211> 45
   <212> DNA
   <213> artificial sequence
<220>
   <223> aptamere
<400> 12
   tcgcacatga catgaagtta aacgcgaatt acaaacccag ccccc 45
<210> 13
   <211> 43
   <212> DNA
   <213> artificial sequence
<220>
   <223> aptamere
<400> 13
   cgcacatgac tcgaaataaa cgcgaattac aaacccagcc ccc 43
<210> 14
   <211> 43
   <212> DNA
   <213> artificial sequence
<220>
   <223> aptamere
<400> 14
   cgcacatgac tcgaagttaa acgcgaatta caaaccaagc cca 43
<210> 15
   <211> 43
   <212> DNA
   <213> artificial sequence
<220>
   <223> aptamere
<400> 15
   cgcacatgac ttgaagttaa acgcgaatta caaacctagc cca 43
<210> 16
   <211> 42
   <212> DNA
   <213> artificial sequence
<220>
   <223> aptamere
<400> 16
   cgcacacgac ttgaagttaa cgcgaattac atcccagacc cg 42
<210> 17
   <211> 42
   <212> DNA
   <213> artificial sequence
<220>
   <223> aptamere
<400> 17
   cgcacatgac ttgaagttaa cgcgaattac aacccagacc cg 42
<210> 18
   <211> 41
   <212> DNA
   <213> artificial sequence
<220>
   <223> aptamere
<400> 18
   cgcacatgac ttgaagttaa cgcgaattac aacccagacc c 41
<210> 19
   <211> 43
   <212> DNA
   <213> artificial sequence
<220>
   <223> aptamere
<400> 19
   cgcacacgac ttgaagttaa acgcgaatta caaaccagac ccc 43
<210> 20
   <211> 43
   <212> DNA
   <213> artificial sequence
<220>
   <223> aptamere
<400> 20
   cgcacatgac ttgaagttaa acgcgaatta caaaccagac ccc 43
<210> 21
   <211> 43
   <212> DNA
   <213> artificial sequence
<220>
   <223> aptaere
<400> 21
   cgcacatgac ttgaagttaa acgcgaatta cgaaccagac ccc 43
<210> 22
   <211> 42
   <212> DNA
   <213> artificial sequence
<220>
   <223> aptamere
<400> 22
   cgcacatgac ttgaagttaa cgcgaattac gaaccagacc ca 42
<210> 23
   <211> 43
   <212> DNA
   <213> artificial sequence
<220>
   <223> aptamere
<400> 23
   cgcacatgac ttgaagttaa acgcgaatta caaaccagac cca 43
<210> 24
   <211> 43
   <212> DNA
   <213> artificial sequence
<220>
   <223> aptamere
<400> 24
   cgcacatgac ttgaagttaa acgcgaatta caaaccaaac cca 43
<210> 25
   <211> 43
   <212> DNA
   <213> artificial sequence
<220>
   <223> aptamere
<400> 25
   cgcacatgac ttgaagttaa acgcgaatta caaaccaaac ccg 43
<210> 26
   <211> 42
   <212> DNA
   <213> artificial sequence
<220>
   <223> aptamere
<400> 26
   cgcacatgac ttgaagttaa cgcgaattac aaaccaaccc cc 42
<210> 27
   <211> 43
   <212> DNA
   <213> artificial sequence
<220>
   <223> aptamere
<400> 27
   cgcacatgac ttgaagttaa cgcgaataac aacccatccc ccc 43
<210> 28
   <211> 44
   <212> DNA
   <213> artificial sequence
<220>
   <223> aptamere
<400> 28
   cgcacaatga cttgaagtga aacgcgaata acaaaccagg cccc 44
<210> 29
   <211> 43
   <212> DNA
   <213> artificial sequence
<220>
   <223> aptamere
<400> 29
   cgcacatgac ttgaagttaa acgcgaatta cagaccaaac cca 43
<210> 30
   <211> 43
   <212> DNA
   <213> artificial sequence
<220>
   <223> aptamere
<400> 30
   cgcacatgac ttgaagttaa acgcgaatta cagaccaaac ccc 43
<210> 31
   <211> 43
   <212> DNA
   <213> artificial sequence
<220>
   <223> aptamere
<400> 31
   cgcacatgac ttgaagtaaa acgcgaatta cagaccaaac ccg 43
<210> 32
   <211> 43
   <212> DNA
   <213> artificial sequence
<220>
   <223> aptamere
<400> 32
   cgcacacgac ttgaagttaa ccgcgaatta caaaccaaac cca 43
<210> 33
   <211> 42
   <212> DNA
   <213> artificial sequence
<220>
   <223> aptamere
<400> 33
   cgcacacgac gtgaagttaa cgcgaatcac aaaccaaacc cg 42
<210> 34
   <211> 43
   <212> DNA
   <213> artificial sequence
<220>
   <223> aptamere
<400> 34
   cgcacacagc tcgaagttaa acgcgaatta caaaccaggc ccc 43
<210> 35
   <211> 43
   <212> DNA
   <213> artificial sequence
<220>
   <223> aptamere
<400> 35
   cgcacatgac ttgaagtaaa acgcgaatta cagaccaaac ccg 43
<210> 36
   <211> 40
   <212> DNA
   <213> artificial sequence
<220>
   <223> aptamere
<400> 36
   ccacgacctc gcacatgact tgaagtaaaa cgcgaattac 40
<210> 37
   <211> 44
   <212> DNA
   <213> artificial sequence
<220>
   <223> aptamere
<400> 37
   ccgcacacca cgcgcatgac cccgcgcaca cgacttgaag tagc 44
<210> 38
   <211> 44
   <212> DNA
   <213> artificial sequence
<220>
   <223> aptamere
<400> 38
   ccgcacgcta cgcgcatgaa cccgcgcaca cgacttgaag tagc 44
<210> 39
   <211> 80
   <212> DNA
   <213> artificial sequence
<220>
   <223> aptamere
<400> 39
   gggagatagc cacgacctcc gcacaccacg cgcatgaccc cgcgcacacg acttgaagta 60
gctccaggct gtgcgaaagc 80